(19) Europäisches Patentamt
European Patent Office
Office européen des brevets

(11) Publication number: **0 397 345 A1**

(12) # EUROPEAN PATENT APPLICATION

(21) Application number: **90304388.3**

(22) Date of filing: **24.04.90**

(51) Int. Cl.$^5$: **A01N 47/10, A01N 57/02, A01N 47/06, A01N 37/02, A01N 37/06, A01N 41/04, A01N 41/00, C07C 69/00, C07C 269/00, C07C 305/00, C07C 307/00**

The title of the invention has been amended (Guidelines for Examination in the EPO, A-III, 7.3).

(30) Priority: **25.04.89 US 342876**
**24.08.89 US 398266**

(43) Date of publication of application:
**14.11.90 Bulletin 90/46**

(84) Designated Contracting States:
**GR**

(71) Applicant: **E.I. DU PONT DE NEMOURS AND COMPANY**
**1007 Market Street**
**Wilmington Delaware 19898(US)**

(72) Inventor: **Hartzell, Stephen Lee**
**2017 Oak Lane Road**
**Wilmington, Delaware 19803(US)**

(74) Representative: **Hildyard, Edward Martin et al**
**Frank B. Dehn & Co. European Patent Attorneys Imperial House 15-19 Kingsway**
**London WC2B 6UZ(GB)**

(54) **Fungicidal oxime carbamates.**

(57) A method of controlling plant diseases, especially fungi, using oxime carbamates and analogs thereof and novel compounds within the class.
Reason: Rule 8.1.i PCT.

EP 0 397 345 A1

# FUNGICIDAL OXIME CARBAMATES

## CROSS-REFERENCE TO RELATED APPLICATIONS

This application is a continuation-in-part of my copending application, U.S. Serial No. 07/398,266, filed August 24, 1989 which in turn is a continuation-in-part of my application U.S. Serial No. 07/342,876, filed April 25, 1989.

## BACKGROUND OF THE INVENTION

Bellina U.S. 3,819,700 claims compounds of Formula i as fungicides for crop protection.

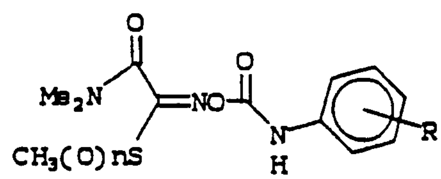

i

Hubele CH 66-16,259 claims compounds of Formula ii as fungicides for crop protection.

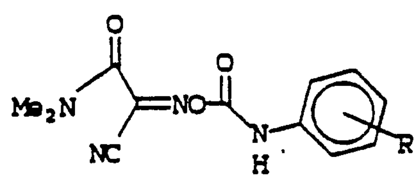

ii

Compounds of Formula iii are disclosed in the patent literature as antidotes for herbicides in U.S. 4,416,686, U.S. 4,426,221, U.S. 4,453,969, U.S. 4,453,974, and U.S. 4,475,945.

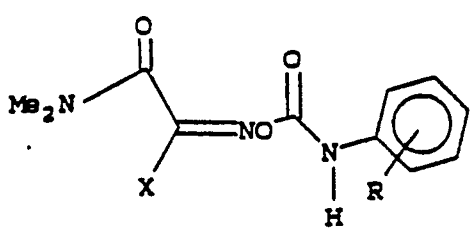

X = halogen

iii

Compounds of formula iv are disclosed in EP 293 667 A as fungicides for crop protection.

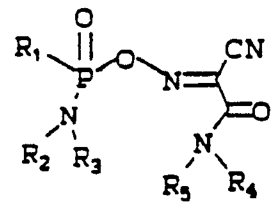

iv

Compounds of formula v are disclosed in U.S. 3,954,992 as fungicides for crop protection.

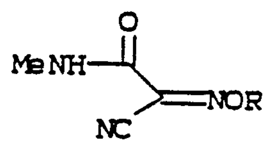

v

SUMMARY OF THE INVENTION

A method of controlling plant disease, especially fungus, comprising the application of an effective amount of a compound of Formula I to the locus to be protected,

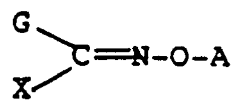

I

wherein:
A is C(=O)R, C(=O)OR$^1$, C(=O)SR$^1$, P(=O)QR$^2$Q$^1$R$^3$; C(=O)NHR, SO$_2$R$^5$, SO$_2$NR$^6$R$^7$;
Q and Q$^1$ are independently oxygen, NR$^8$ or a direct bond;
X is Cl or Br; provided that when X is Br, A is C(=O)R;
G is C(=L)R$^9$, C(=L)NR$^{10}$R$^{11}$, C(=O)OR$^{12}$, CN, SO$_2$NR$^{10}$R$^{11}$, or SO$_m$R$^{13}$;
L is O or S;
m is 0, 1 or 2;
R is $C_1$-$C_{20}$ alkyl, $C_2$-$C_{20}$ alkenyl, $C_2$-$C_{20}$ alkynyl; $C_1$-$C_8$ alkyl, $C_2$-$C_8$ alkenyl, $C_2$-$C_8$ alkynyl, or $C_3$-$C_7$ cycloalkyl, each optionally substituted with halogen, $C_1$-$C_6$ alkoxy, $C_2$-$C_6$ alkoxyalkyl, $C_1$-$C_6$ alkylthio, $C_3$-$C_6$ cycloalkyl, CN, or with a phenyl group substituted with 0-3 halogen and 0-2 groups selected from $CH_3$, $CF_3$, $CH_3O$, CN, $CH_3S$, methylsulfonyl, dimethylamino, amino, phenyl or phenoxy, provided that when A is C-(=O)NHR, R is not $C_2$ alkenyl or $C_2$ alkynyl;
R is a phenyl ring or a $C_{10}$-$C_{14}$ fused carbocyclic aromatic ring systems, wherein said rings are substituted with 0-4 halogen, 0-1 groups selected from -$CH_2(CH_2)_pCH_2$-, -$O(CH_2)_pCH_2$-, -$S(CH_2)_pCH_2$-, -$O(CH_2)_pO$, -O-$(CH_2)_p$S-, R$^4$N$(CH_2)_pCH_2$-, -$O(CH_2)_p$NR$^4$-, and 0-2 groups selected from NH(C=O)OR$^{16}$, SCN, $C_1$-$C_4$ alkyl, $C_1$-$C_4$ haloalkyl, $C_2$-$C_4$ alkoxyalkyl, $C_2$-$C_4$ alkenyl, $C_2$-$C_4$ haloalkenyl, $C_2$-$C_4$ alkynyl, $C_3$-$C_6$ cycloalkoxy, $C_3$-$C_6$ cycloalkyl, $NO_2$, C(=O)R$^{14}$, CN, OR$^{14}$, C(=O)OR$^{14}$, C(=O)NR$^{14}$R$^{15}$, NR$^{14}$R$^{15}$, SR$^{14}$, SOR$^{14}$, $SO_2F$, $SO_2Cl$ or SO$_2$NR$^{14}$R$^{15}$, 2-, 3-, or 4-pyridyl, or phenyl substituted with 0-3 halogen and 0-2 groups selected from $CH_3$, $CF_3$, $CH_3O$, CN, $CH_3S$, methylsulfonyl, dimethylamino or amino;
R can additionally be a heterocyclic ring system of 3-8 atoms, ring-carbon-linked to the carbonyl group

when A is C(=O)R or to the N when A is C(=O)NHR, containing 1 or 2 nitrogen atoms, or 1 nitrogen atom and 1 oxygen atom, or 1 nitrogen atom and 1 sulfur atom; or one or, if the ring size is greater than 3, 2 oxygen atoms, provided that the oxygen atoms are not bonded to each other; or 1 or 2 sulfur atoms;
R can additionally be a heteroaromatic or fused heteroaromatic ring system, ring-carbon-linked to the carbonyl group when A is C(=O)R or to the N when A is C(=O)NHR, containing 5-10 atoms, wherein the heteroatoms comprise 1-3 nitrogen atoms, or 1-2 nitrogen atoms and one oxygen or sulfur atom, or 1-2 oxygen or sulfur atoms, these rings being substituted with 0-1 ($-CH_2(CH_2)_pCH_2-$) or with 0-2 groups selected from $CH_3$, $OCH_3$, $OCF_3$, $OCH_2CF_3$, F, Cl, Br, $OCH_2CH_3$, $NO_2$, $C(=O)CH_3$, $N(CH_3)_2$, $CO_2CH_3$, $CON(CH_3)_2$, $SO_2N(CH_3)_2$, $SCH_3$, CN or $CF_3$;
$R^1$ is $C_1$-$C_{20}$ alkyl, $C_3$-$C_{20}$ alkenyl, $C_3$-$C_{20}$ alkynyl; or $C_1$-$C_8$ alkyl, $C_3$-$C_8$ alkenyl or $C_3$-$C_8$ alkynyl, each optionally substituted with halogen, $C_1$-$C_6$ alkoxy, $C_2$-$C_6$ alkoxyalkyl, $C_1$-$C_6$ alkylthio, $C_3$-$C_6$ cycloalkyl, CN or with a phenyl group substituted with 0-3 halogen and 0-2 groups selected from $CH_3$, $CF_3$, $CH_3O$, CN, $CH_3S$, methylsulfonyl, dimethylamino, amino, phenyl or phenoxy; provided that, if $R^1$ is alkenyl or alkynyl, the unsaturated carbons are not bonded directly to the oxygen atom of

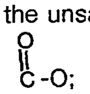

; $R^2$ and $R^3$ are independently selected from $C_1$-$C_4$ alkyl, $C_1$-$C_4$ haloalkyl, benzyl or phenyl;
$R^4$ is H or $C_1$-$C_4$ alkyl;
$R^5$ is $C_1$-$C_4$ alkyl substituted with 0-3 halogen, or $R^5$ is a phenyl group substituted with 0-3 halogen and 0-2 groups selected from $CH_3$, $CF_3$, $CH_3O$, CN, $CH_3S$, methylsulfonyl, dimethylamino, amino, nitro, phenyl or phenoxy;
$R^6$ is H, $C_1$-$C_4$ alkyl substituted with 0-3 halogen, or R6 is a phenyl group substituted with 0-3 halogen and 0-2 groups selected from $CH_3$, $CF_3$, $CH_3O$, CN, $CH_3S$, methylsulfonyl, dimethylamino, amino, phenyl or phenoxy;
$R^7$ is H or $C_1$-$C_4$ alkyl substituted with 0-3 halogen, or $R^6$ and $R^7$ taken together with the nitrogen atom to which they are attached can be piperidine, pyrrolidine or morpholine, each substituted with 0-2 methyl groups;
$R^8$ is H or $C_1$-$C_4$ alkyl;
$R^9$ is $C_1$-$C_8$ alkyl substituted with 0-3 halogen, or $R^9$ is phenyl substituted with 0-2 halogen, $CH_3$, $CF_3$, $CH_3O$ or CN;
$R^{10}$ and $R^{11}$ are independently H, $C_1$-$C_4$ alkyl, $C_1$-$C_4$ haloalkyl, $C_2$-$C_4$ alkoxyalkyl, or benzyl or phenyl, said benzyl or phenyl rings being substituted with 0-2 halogen, $CH_3$, $CF_3$, $CH_3O$ or CN; or $R^{10}$ and $R^{11}$, taken together with the nitrogen atom to which they are attached, can be azetidine, piperidine, homopippiridine, pyrrolidine, or morpholine, each substituted with 0-2 methyl groups;
$R^{12}$ is $C_1$-$C_{12}$ alkyl or haloalkyl, or benzyl substituted with 0-2 halogen, $CH_3$, $CF_3$, $CH_3O$ or CN;
$R^{13}$ is $C_1$-$C_4$ alkyl, haloalkyl or $C_2$-$C_4$ alkoxyalkyl, $C_3$-$C_4$ alkenyl, haloalkenyl, alkynyl or haloalkynyl, or benzyl or phenyl, said benzyl or phenyl rings being substituted with 0-2 halogen, $CH_3$, $CF_3$, $CH_3O$ or CN;
$R^{14}$ is H, $C_1$-$C_4$ alkyl, $C_1$-$C_4$ haloalkyl, or $C_2$-$C_4$ alkoxyalkyl; $C_3$-$C_4$ alkenyl, haloalkenyl, alkynyl or haloalkynyl; or benzyl or phenyl, said benzyl or phenyl rings being substituted with 0-2 halogen, $CH_3$, $CF_3$, $CH_3O$, $CH_3S$ or CN;
$R^{15}$ is H or $C_1$-$C_4$ alkyl;
$R^{16}$ is $C_1$-$C_6$ alkyl, $C_3$-$C_4$ alkenyl, or benzyl substituted with 0-2 halogen, methyl, trifluoromethyl, nitro or methoxy; and
p is 1 or 2.

Also contemplated are compounds of Formula I,

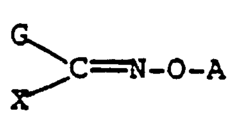

wherein
A is $C(=O)R$, $C(=O)OR^1$, $C(=O)SR^1$, $P(=O)QR^2Q^1R^3$; $C(=O)NHR$, $SO_2R^5$, $SO_2NR^6R^7$;
Q and $Q^1$ are independently oxygen, $NR^8$ or a direct bond;
X is Cl or Br; provided that when X is Br, A is $C(=O)R$;
G is $C(=L)R^9$, $C(=L)NR^{10}R^{11}$, $C(=O)OR^{12}$, CN, $SO_2NR^{10}R^{11}$, or $SO_mR^{13}$;
L is O or S;
m is 0, 1 or 2;

R is $C_1$-$C_{20}$ alkyl, $C_2$-$C_{20}$ alkenyl, $C_2$-$C_{20}$ alkynyl; $C_1$-$C_8$ alkyl, $C_2$-$C_8$ alkenyl, $C_2$-$C_8$ alkynyl, or $C_3$-$C_7$ cycloalkyl, each optionally substituted with halogen, $C_1$-$C_6$ alkoxy, $C_2$-$C_6$ alkoxyalkyl, $C_1$-$C_6$ alkylthio, $C_3$-$C_6$ cycloalkyl, CN, or with a phenyl group substituted with 0-3 halogen and 0-2 groups selected from $CH_3$, $CF_3$, $CH_3O$, CN, $CH_3S$, methylsulfonyl, dimethylamino, amino, phenyl or phenoxy, provided that when A is C(=O)NHR, R is not $C_2$ alkenyl or $C_2$ alkynyl;

R is a phenyl ring or a $C_{10}$-$C_{14}$ fused carbocyclic aromatic ring systems, wherein said rings are substituted with 0-4 halogen, and 0-1 groups selected from $-CH_2(CH_2)_pCH_2-$, $-O(CH_2)_pCH_2-$, $-S(CH_2)_pCH_2-$, $-O(CH_2)_pO$, $-O(CH_2)_pS-$, $-R^4N(CH_2)_pCH_2-$, $-O(CH_2)_pNR^4-$, and 0-2 groups selected from $NH(C=O)OR^{16}$, SCN, $C_1$-$C_4$ alkyl, $C_1$-$C_4$ haloalkyl, $C_2$-$C_4$ alkoxyalkyl, $C_2$-$C_4$ alkenyl, $C_2$-$C_4$ haloalkenyl, $C_2$-$C_4$ alkynyl, $C_3$-$C_6$ cycloalkoxy, $C_3$-$C_6$ cycloalkyl, $NO_2$, $C(=O)R^{14}$, CN, $OR^{14}$, $C(=O)OR^{14}$, $C(=O)NR^{14}R^{15}$, $NR^{14}R^{15}$, $SR^{14}$, $SOR^{14}$, $SO_2F$, $SO_2Cl$ or $SO_2NR^{14}R^{15}$, 2-, 3-, or 4-pyridyl, or phenyl substituted with 0-3 halogen and 0-2 groups selected from $CH_3$, $CF_3$, $CH_3O$, CN, $CH_3S$, methylsulfonyl, dimethylamino or amino;

R can additionally be a heterocyclic ring system of 3-8 atoms, ring-carbon-linked to the carbonyl group when A is C(=O)R or to the N when A is C(=O)NHR, containing 1 or 2 nitrogen atoms, or 1 nitrogen atom and 1 oxygen atom, or 1 nitrogen atom and 1 sulfur atom; or one or, if the ring size is greater than 3, 2 oxygen atoms, provided that the oxygen atoms are not bonded to each other; or 1 or 2 sulfur atoms;

R can additionally be a heteroaromatic or fused heteroaromatic ring system, ring-carbon-linked to the carbonyl group when A is C(=O)R or to the N when A is C(=O)NHR, containing 5-10 atoms, wherein the heteroatoms comprise 1-3 nitrogen atoms, or 1-2 nitrogen atoms and one oxygen or sulfur atom, or 1-2 oxygen or sulfur atoms, these rings being substituted with 0-1 $(-CH_2(CH_2)_pCH_2-)$ or with 0-2 groups selected from $CH_3$, $OCH_3$, $OCF_3$, $OCH_2CF_3$, F, Cl, Br, $OCH_2CH_3$, $NO_2$, $C(=O)CH_3$, $N(CH_3)_2$, $CO_2CH_3$, $CON(CH_3)_2$, $SO_2N(CH_3)_2$, $SCH_3$, CN or $CF_3$;

$R^1$ is $C_1$-$C_{20}$ alkyl, $C_3$-$C_{20}$ alkenyl, $C_3$-$C_{20}$ alkynyl; or $C_1$-$C_8$ alkyl, $C_3$-$C_8$ alkenyl or $C_3$-$C_8$ alkynyl, each optionally substituted with halogen, $C_1$-$C_6$ alkoxy, $C_2$-$C_6$ alkoxyalkyl, $C_1$-$C_6$ alkylthio, $C_3$-$C_6$ cycloalkyl, CN or with a phenyl group substituted with 0-3 halogen and 0-2 groups selected from $CH_3$, $CF_3$, $CH_3O$, CN, $CH_3S$, methylsulfonyl, dimethylamino, amino, phenyl or phenoxy; provided that, if $R^1$ is alkenyl or alkynyl, the unsaturated carbons are not bonded directly to the oxygen atom of

$\overset{O}{\overset{\parallel}{C}}$ -O;

$R^2$ and $R^3$ are independently selected from $C_1$-$C_4$ alkyl, $C_1$-$C_4$ haloalkyl, benzyl or phenyl;

$R^4$ is H or $C_1$-$C_4$ alkyl;

$R^5$ is $C_1$-$C_4$ alkyl substituted with 0-3 halogen, or $R^5$ is a phenyl group substituted with 0-3 halogen and 0-2 groups selected from $CH_3$, $CF_3$, $CH_3O$, CN, $CH_3S$, methylsulfonyl, dimethylamino, amino, nitro, phenyl or phenoxy;

$R^6$ is H, $C_1$-$C_4$ alkyl substituted with 0-3 halogen, or R6 is a phenyl group substituted with 0-3 halogen and 0-2 groups selected from $CH_3$, $CF_3$, $CH_3O$, CN, $CH_3S$, methylsulfonyl, dimethylamino, amino, phenyl or phenoxy;

$R^7$ is H or $C_1$-$C_4$ alkyl substituted with 0-3 halogen, or $R^6$ and $R^7$ taken together with the nitrogen atom to which they are attached can be piperidine, pyrrolidine or morpholine, each substituted with 0-2 methyl groups;

$R^8$ is H or $C_1$-$C_4$ alkyl;

$R^9$ is $C_1$-$C_8$ alkyl substituted with 0-3 halogen, or $R^9$ is phenyl substituted with 0-2 halogen, $CH_3$, $CF_3$, $CH_3O$ or CN;

$R^{10}$ and $R^{11}$ are independently H, $C_1$-$C_4$ alkyl, $C_1$-$C_4$ haloalkyl, $C_2$-$C_4$ alkoxyalkyl, or benzyl or phenyl, said benzyl or phenyl rings being substituted with 0-2 halogen, $CH_3$, $CF_3$, $CH_3O$ or CN; or $R^{10}$ and $R^{11}$, taken together with the nitrogen atom to which they are attached, can be azetidine, piperidine, homopippridine, pyrrolidine, or morpholine, each substituted with 0-2 methyl groups;

$R^{12}$ is $C_1$-$C_{12}$ alkyl or haloalkyl, or benzyl substituted with 0-2 halogen, $CH_3$, $CF_3$, $CH_3O$ or CN;

$R^{13}$ is $C_1$-$C_4$ alkyl, haloalkyl or $C_2$-$C_4$ alkoxyalkyl, $C_3$-$C_4$ alkenyl, haloalkenyl, alkynyl or haloalkynyl, or benzyl or phenyl, said benzyl or phenyl rings being substituted with 0-2 halogen, $CH_3$, $CF_3$, $CH_3O$ or CN;

$R^{14}$ is H, $C_1$-$C_4$ alkyl, $C_1$-$C_4$ haloalkyl, or $C_2$-$C_4$ alkoxyalkyl; $C_3$-$C_4$ alkenyl, haloalkenyl, alkynyl or haloalkynyl; or benzyl or phenyl, said benzyl or phenyl rings being substituted with 0-2 halogen, $CH_3$, $CF_3$, $CH_3O$, $CH_3S$ or CN;

$R^{15}$ is H or $C_1$-$C_4$ alkyl;

$R^{16}$ is $C_1$-$C_6$ alkyl, $C_3$-$C_4$ alkenyl, or benzyl substituted with 0-2 halogen, methyl, trifluoromethyl, nitro or methoxy; and

p is 1 or 2;

provided that

1. when A is C(-O)SR[1] or C(=O)OR[1], then G is not C(=L)NR[10]R[11] or C(=O)OR[12]; and

2. when A is C(=O)NHR, and G is C(=L)NR[10]R[11] or C(=O)OR[12], then R is not unsubstituted phenyl or $C_1$-$C_3$ alkyl.

1. Preferred for ease of synthesis and/or fungicidal activity are compounds of Formula I wherein:
A is C(=O)R, C(=O)OR[1],C(=O)NHR, or P(=O)QR[2]Q[1]R[3];
G is C(=O)NR[10]R[11] or C(=O)OR[12].

2. More preferred for ease of synthesis and/or fungicidal activity are compounds of Formula I wherein:
A is C(=O)R, or C(=O)NHR;
G is C(=O)NR[10]R[11] or C(=O)OR[12];
X is Cl.

3. Even more preferred for ease of synthesis and/or fungicidal activity are compounds of Formula I wherein:
A is C(=O)R, or C(=O)NHR;
G is C(=O)NR[10]R[11] or C(O)OR[12];
R is $C_1$-$C_2$ alkyl substituted with a phenyl group, said phenyl group being substituted with 0-3 halogen and 0-2 groups selected from $CH_3$, $CF_3$, $CH_3O$, CN, $CH_3S$, methylsulfonyl, dimethylamino, amino, phenyl or phenoxy; or
R is a phenyl or naphthyl ring, substituted with 0-3 halogen and 0-2 groups selected from $CH_3$, $CF_3$, $CH_3O$, CN, $CH_3S$, methylsulfonyl, dimethylamino, amino, phenyl or phenoxy;
R[10] and R[11] are independently H, $C_1$-$C_4$ alkyl, haloalkyl or benzyl substituted with 0-2 halogen, $CH_3$, $CF_3$, $CH_3O$ or CN; or R[10] and R[11], taken together with the nitrogen atom to which they are attached, can be piperidine, pyrrolidine or morpholine, each substituted with 0-2 methyl groups; and
X is Cl.

4. Particularly preferred for ease of synthesis and/or fungicidal activity are compounds of Preferred 3 wherein:
A is C(=O)R;
G is C(=O)NR[10]R[11];
R is $C_1$-$C_2$ alkyl substituted with a phenyl group, said phenyl group being substituted with 0-3 halogen and 0-2 groups selected from $CH_3$, $CF_3$, $CH_3O$, CN, $CH_3S$, methylsulfonyl, dimethylamino, amino, phenyl or phenoxy; or
R is a substituted phenyl or naphthyl ring, wherein the substituent is selected from: 0-3 halogen and 0-2 $CH_3$, $CF_3$, $CH_3O$, CN, $CH_3S$, methylsulfonyl, dimethylamino, amino, phenyl or phenoxy; and
X is Cl.

Also particularly preferred for ease of synthesis and/or fungicidal activity are compounds of Preferred 3, wherein:
A is C(=O)NHR;
G is C(=O)NR[10]R[11];
R is $C_1$-$C_2$ alkyl substituted with a phenyl group, said phenyl group being substituted with 0-3 halogen and 0-2 groups selected from $CH_3$, $CF_3$, $CH_3O$, CN, $CH_3S$, methylsulfonyl, dimethylamino, amino, phenyl or phenoxy; or
R is a substituted phenyl or naphthyl ring, wherein the substituent is selected from: 0-3 halogen and 0-2 $CH_3$, $CF_3$, $CH_3O$, CN, $CH_3S$, methylsulfonyl, dimethylamino, amino, phenyl or phenoxy; and
X is Cl.

Specifically preferred are the following compounds:
N-[[2-naphthylcarbonyl]oxy]-2-(dimethylamino)-2-oxoethanimidoyl chloride;
N-[[[(3,5-dichlorophenyl)amino]carbonyl]oxy]-2-(dimethylamino)-2-oxoethanimidoyl chloride;
N-[[[(2,6-dichlorophenyl)amino]carbonyl]oxy]-2-(dimethylamino)-2-oxoethanimidoyl chloride;
N-[[[[3,5-Bis(trifluoromethyl)phenyl]amino]carbonyl]oxy]2-(dimethylamino)-2-oxoethanimidoyl chloride;
N-[[[3,4-(dichlorophenyl)amino]carbonyl]oxy]-2-(dimethylamino)-2-oxoethanimidoyl chloride;
N-[[[3,4-(dichlorophenyl)amino]carbonyl]oxy]-2-oxo-2-piperidino ethanimidoyl chloride;
N-[[[(3,5-dichlorophenyl)amino]carbonyl]oxy]-α-oxo-1-piperidineethanimidoyl chloride;
N-[[bis(2,2,2-trichloroethoxy)phosphinyl]oxy]-2-(dimethylamino)-2-oxoethanimidoyl chloride;
N-[[[[(2-chlorophenyl)methyl]amino]carbonyl]oxy]-2-(diethylamino)-2-oxoethanimidoyl chloride;
N-[[([1,1-biphenyl]-4-yl)carbonyl]oxy]-2-(dimethylamino)-2-oxoethanimidoyl chloride;
N-[[[(3,5-difluorophenyl)amino]carbonyl]oxy]-α-oxo-1-piperidineethanimidoyl chloride;
N-[[(2-naphthalenyl)carbonyl]oxy]-α-oxo-1-piperidineethanimidoyl chloride;
2-(dimethylamino)-2-oxo-N-[[[(2,4,5-trichlorophenyl)amino]carbonyl]oxy]ethanimidoyl chloride;

2-(dimethylamino)-2-oxo-N-[(1-oxooctadecyl)oxy]ethanimidoyl chloride; and
2-(dimethylamino)-2-oxo-N-[[(phenylmethoxy)carbonyl]oxy]ethanimidoyl chloride.

Also preferred as intermediates useful in preparing the above compounds are:
N-[(chlorocarbonyl)oxy]-2-dimethylamino-2-oxo-ethaneimidoyl chloride; and
N-[(chlorocarbonyl)oxy]-α-oxo-1-piperidineethanimidoyl chloride.

Also contemplated are agriculturally suitable compositions of the above-described compounds, comprising a fungicidally active amount of said compound and at least one of the following: surfactant, solid diluent or liquid diluent.

## DETAILED DESCRIPTION OF THE INVENTION

### Synthesis

The compounds of Formula I can be prepared from compounds of Formula III and an appropriate electrophilic reagent of Formula II in an inert solvent with or without a base used either as a catalyst or acid scavenger. Suitable solvents include polar aprotic solvents such as acetonitrile, dimethylformamide, or dimethylsulfoxide; ethers such as tetrahydrofuran, dimethoxyethane, or diethyl ether; ketones such as acetone or 2-butanone; hydrocarbons such as toluene or benzene; or halocarbons such as dichloromethane or chloroform. Appropriate bases include alkali metal alkoxides such as sodium methoxide or potassium tert-butoxide, inorganic bases such as sodium hydride or potassium carbonate, or tertiary amines such as triethylamine, pyridine, 1,8-diazabicyclo[5.4.0] undec-7-ene (DBU), or triethylenediamine (DABCO). The reaction temperature can vary between 0° C and 150° for periods of 1 to 72 hours depending on the choice of base, solvent, temperature, and substrates.

Compounds of Formulas Ia-f can be prepared from combining an appropriate electrophilic reagent of Formula IIa-f with an oxime of Formula III by several processes which are summarized in the following equations.

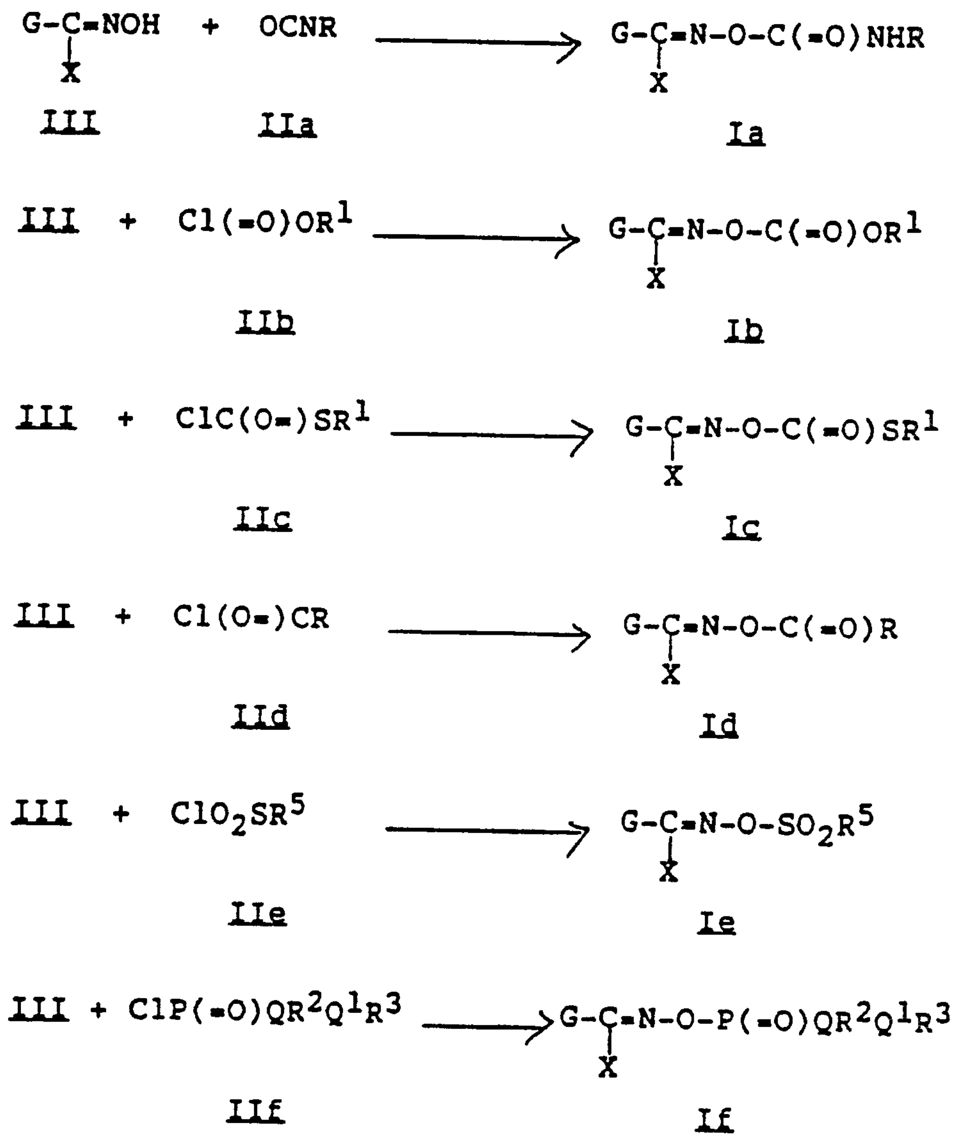

III IIa Ia

IIb Ib

IIc Ic

IId Id

IIe Ie

IIf If

A novel method of preparation of compounds of Formula Ia-c comprises sequential preparation of the novel chloroformate Ig by reaction of compounds of Formula III with phosgene in the presence of organic bases such as N,N-diethylaniline and pyridine, followed by the appropriate $RNH_2$, $R^1OH$ or $R^1SH$ compound in the presence of a suitable acid scavenger such as N,N-diethylaniline, pyridine or triethylamine.

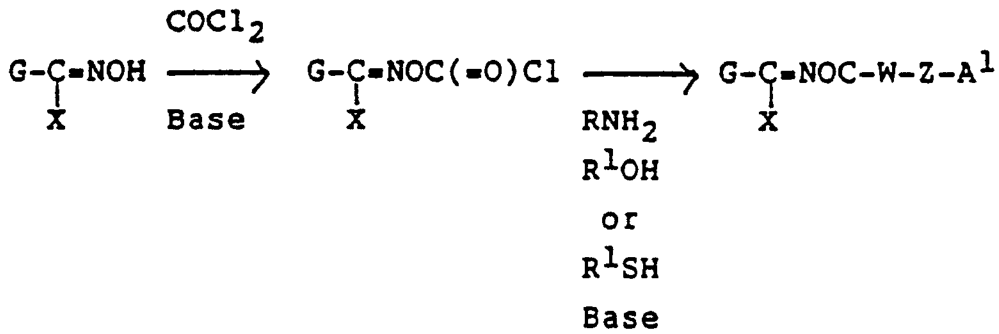


III Ig

I W-Z-A$^1$ = C(=O)NHR
C(=O)OR$^1$
C(=O)SR$^1$

Compounds of Formula Id can be prepared from compounds of Formula III and a carboxylic acid IIg in the presence of a coupling auxiliary reagent such as, but not limited to, N,N-dicyclohexyl carbodiimide (DCC) or 2,2′-dipyridyldisulfide (DPDS).

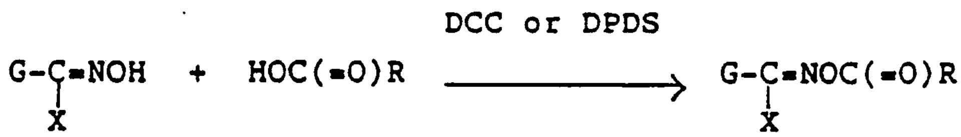


III IIg Id

Alternatively, the hydroximoyl chlorides or bromides of Formulas Ia, b, d, e, f can be prepared from the corresponding aldoximes of Formula IV with halogens (see H. Metzger, Herstellung von Oximen, Houben-Weyl, Methoden der organische Chemie, E. Miller, Editor, Vol. 10(4), 4th ed., p. 98, Thieme, Stuttgart, 1968), N-chlorosuccinimide (see K. C. Liu, B. R. Shelton, and R. K. Howe, J. Org. Chem., 1980, 45,3916), or N-bromosuccinimide (see C. Grundmann and R. Richter, J. Org. Chem., 1968, 33,476).

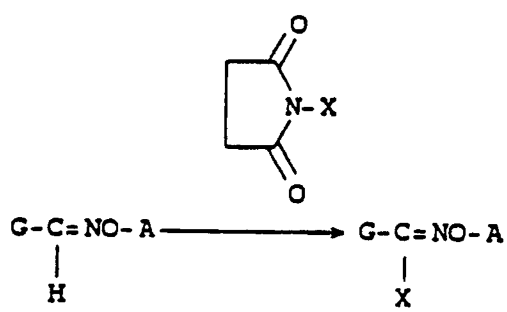

X=Cl, Br

IV I

Hydroximoyl chlorides of Formula Ib and Id can be prepared by the reaction of nitro compounds of Formula Ih with two equivalents of an organic base such as triethylamine and two equivalents of a chloroformate of Formula IIb or an acid chloride of Formula IId (see T. Shinizer, Y. Hayashi, H. Shibafuchi, and K. Teramura, Bull. Chem. Soc. Japan, 1986, 59, 2827).

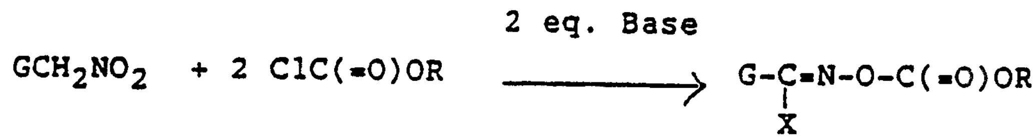


Ih IIb Ib X = Cl

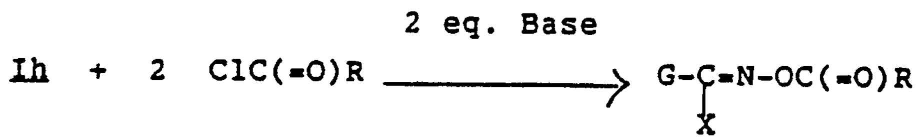


IId Id X = Cl

The thiocarboxamide and thioketone compounds of Formula I can be prepared from the carboxamide or ketone compounds, respectively, of Formula I by methods described elsewhere (see S. Scheibye, E. S. Pedersen and S. O. Lawesson, Bull. Soc. Chim. Belg., 1978, 87, 229 and G. Lajore, F. Lepine, L. Maziak and B. Belleau, Tet. Letters, 1983, 24, 3815).

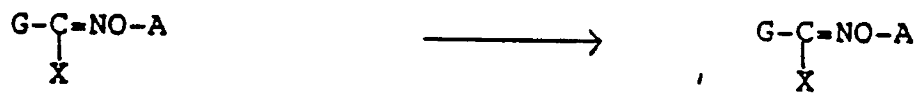

I, G = $C(=O)NR^{10}R^{11}$, $C(=O)R^9$ I, G = $C(=S)NR^{10}R^{11}$, $C(=S)R^9$

Compounds of Formula I can also be prepared by conversion of other compounds of Formula I by standard methods of organic reactions, recognizable by those skilled in the art, such as, but not limited to, reduction and oxidation.

The α-chloroaldoximes of Formula III can be prepared by treating amines of Formula V with sodium nitrite and hydrochloric acid (see G. S. Skinner, J. Am. Chem. Soc., 1924, 46,731).

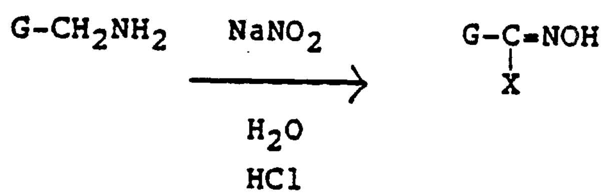


V III

The α-chloroaldoximes of Formula III can also be prepared from aldoximes of Formula VI by treatment with N-chlorosuccinimide (see K. E. Larsen and K. B. G. Torsell, Tetrahedron, 1984, 40, 2985) or t-butylhypochlorite (see C. J. Peake and J. H. Strickland, Synth. Comm., 1986, 16, 763).

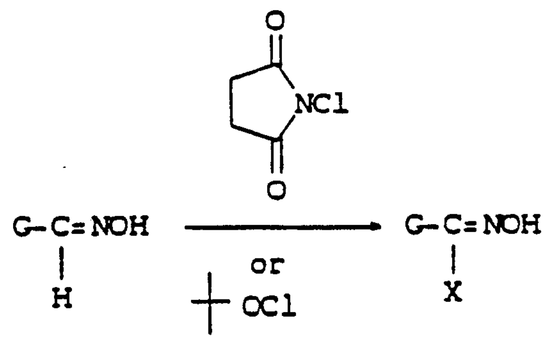


VI III X = Cl

Some α-haloaldoximes of Formula III may be prepared from amide oximes VII by treatment with sodium nitrite in hydrohalic acid solution (see M. Kocevar, S. Polanc, M. Sollner, M. Tisler and B. Vercek, Synth. Comm., 1988, 18, 1427).

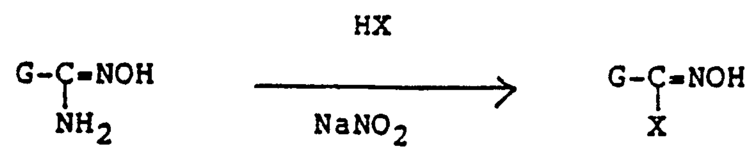


VII III

The α-haloaldoximes of Formula III can be prepared from trihalomethyl compounds of Formula VIII by basic hydrolysis in the presence of hydroxylamine (see A. P. Kozikowski and M. Adamczyk, J. Org. Chem., 1983, 48, 366).

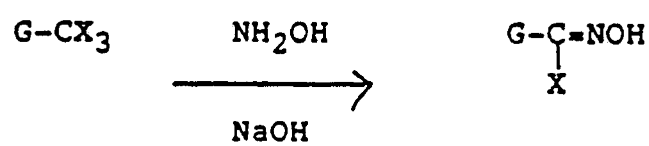


VII III

The α-haloaldoximes of Formula III can be prepared by the reaction of nitrile N-oxides of Formula IX with hydrohalic acids (see C. Grundmann, V. Mini, J. M. Dean, and H. -D. Frommeld, Justis Liebigs Ann. Chem., 1965, 687,191).

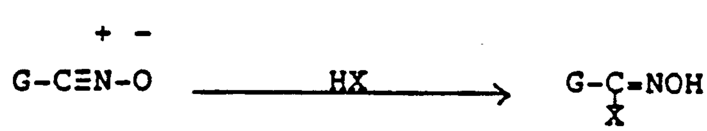


IX III

The nitrile N-oxides of Formula IX can be prepared by several methods well known in the chemical art (for a summary of methods, see T. Shimizu, Y. Hayashi, and K. Taramura, Bull. Soc. Chem. Jpn., 1984, 57,2531). The most familiar method involves treating an α-chloroaldoxime of Formula X with an inorganic base such as sodium hydroxide or sodium carbonate or an organic base such as triethylamine followed by trapping the nitrile N-oxide of Formula VII with a hydrohalic acid. A new α-haloaldoxime of Formula III is

produced.

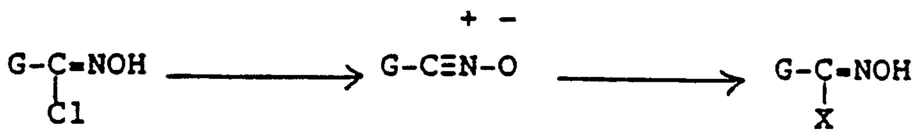

X IX III

The carboxamide and carboxylic acid ester compounds of Formula III can be prepared by the procedures taught in the U.S. Patents 3,557,089, 3,557,190, and 3,560,555.

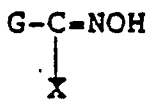

$G=C(=O)NR^{10}R^{11}$, $CO_2R^{12}$

X=Cl

III

The thiocarboxamide compounds of Formula III can be prepared from the trihalothioacetamides of Formula XI which are obained from the haloimmonium chlorides of Formula XII. See W. Walter and K. -D. Bode, Angew. Chem. Internat. Edit., 1966, 5, 447 for a review of the syntheses of thiocarboxamides.

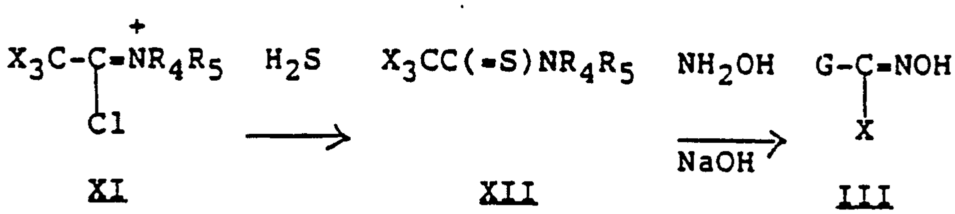


XI XII III

X = halogen

$C = C(=S)NR^{10}R^{11}$

The cyano compound of Formula III can be prepared by methods already reported (see A. P. Kozikowski and M. Adamczyk, J. Org. Chem., 1983, 48, 366).

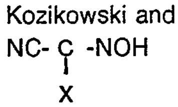
III X = Cl

The ketone compounds of Formula III can be prepared from halomethyl ketones of Formula XIII by treatment with an alkyl nitrite and hydrochloric acid (see N. Levin and W. H. Hartung, Org. Synthesis, 1944, 24, 25).

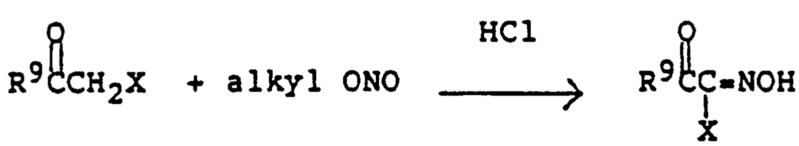


XIII X = Cl

III X = Cl

The ketone compounds of Formula III can also be prepared from halomethyl ketones of Formula XIII by treatment with dimethyl sulfide to afford compounds of Formula XIV, which are then treated with sodium nitrite and hydrochloric acid (see Y. Ofsuji, Y. Tsujii, A. Yoshida and E. Imoto, Bull Chem. Soc. Japan, 1971, 44, 223).

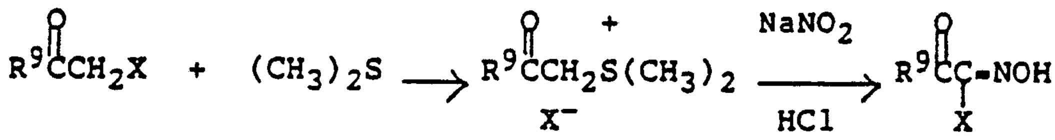


XIII XIV III X = Cl

The ketone compounds of Formula III can be prepared from β-ketosulfoxides of Formula XV by treatment with sodium nitrite in hydrohalic acid solution (see Y. Otsuji, Y. Tsujii, A. Yoshida and E. Imoto, Bull. Chem. Soc. Japan, 1971, 44, 219).

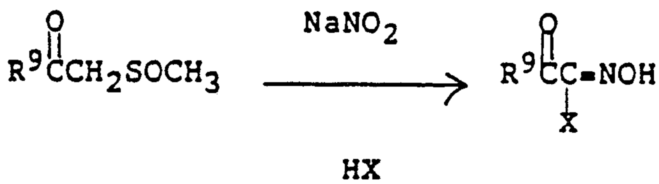


XV III

The ketone compounds of Formula III can also be prepared from compounds of Formula VI by treatment with chlorine gas (see G. Casnati and A. Ricca, Tet. Letters, 1967, 327 and Y. H. Chiang, J. Org. Chem., 1971, 36, 2146).

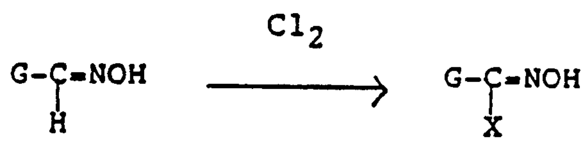


VI G=$R^9$CO III X = Cl

The thioalkyl and thioaryl compounds of Formula III can be prepared from dihaloformaldoxime XVI (see D. Chiarino, M. Napoletano and A. Sala, Synth. Comm., 1988, 18, 1171 and D. M. Vyas, Y. Chiang and T. W. Doyle, Tet. Letters, 1984, 25, 487) by reaction with thiols and one equivalent of an organic base such as triethylamine (see M. H. Benn, Can. J. Chem., 1964, 42, 2393).

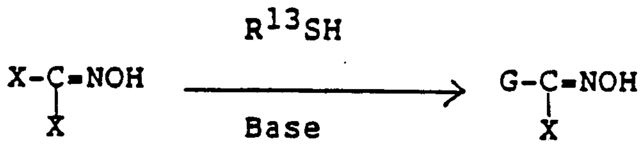


XVI X = Br,Cl III G = $R^{13}$S

The sulfoxide and sulfone compounds of Formula III can be prepared from compounds of Formula III (G = $R^{13}$S) by oxidation using one or two equivalents, respectively, of oxidants such as hydrogen peroxide or organic peracids, such as peracetic acid.

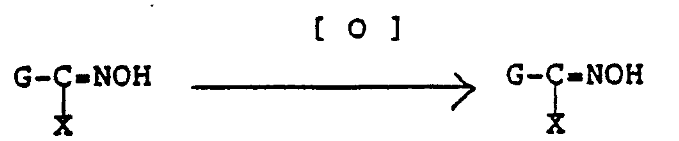


III G= $R^{13}S$ III G = $R^{13}SO$, $R^{13}SO_2$

The sulfone compounds of Formula III can also be prepared from compounds of Formula Ih (G = $R^{13}SO_2$) according to methods already reported (see P. A. Wade and H. R. Hinney, J. Am. Chem. Soc., 1979, 101, 1319).

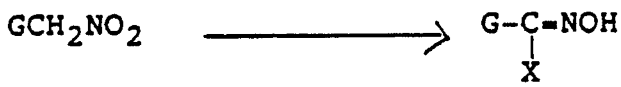

Ih G = $R^{13}SO_2$ III X = Br

Alternatively, the sulfonylcarbohydroximoyl chlorides of Formula III can be prepared from α-diazosulfones of Formula XVII and nitrosyl chloride (see J. C. Jagt, I. van Buuren, J. Strating and A. M. van Leusen, Synth. Commun., 1974, 4,311).

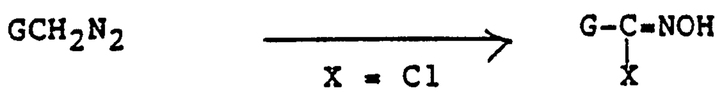


XVII III

$G=R_4SO_2$ X=Cl

Those skilled in the art will recognize compounds of Formula I are O-substituted oximes which can be of either the syn or anti form. The scope of the specification referring to compounds of Formula I includes both stereoisomeric oxime forms either as a specific steroisomer, a mixture of stereoisomers, or as any reciprocal mixture ratio of the two stereoisomeric forms.

The examples which follow are representative of the production of the novel oximes of Formula I.

## EXAMPLE 1

### N-[[[2,6-(dichlorophenyl)amino]carbonyl]oxy]-2-(dimethylamino)-2-oxo-ethanimidoyl chloride

DBU (3 drops) is added to a solution of 2-chloro-2-hydroxyimino-N,N-dimethylacetamide (3.0g) and 2,6-dichlorophenyl isocyanate in 100ml of THF at room temperature. After stirring the reaction overnight, the solvent is removed in vacuo leaving a white solid. The solid is triturated with chloroform and collected by vacuum filtration giving 4.0g (59%) of the title compound, m.p. - 182-185 °C.

## EXAMPLE 2

N-[[[[3,5-bis(trifluoromethyl)phenyl]amino)]carbonyl]oxy]-2-(dimethylamino)]-2-oxoethanimidoyl chloride

DBU (3 drops) is added to a solution of 2-chloro-2-hydroxyimino-N,N-dimethylacetamide (3.0g) and 3,5-bis(trifluromethyl)phenyl isocyanate (5.1g) in 100 mL of THF at room temperature. After stirring the reaction overnight, the solvent is removed in vacuo leaving a white solid. Trituration with carbon tetrachloride gives product as a white solid; yield 7.45g (92%), m. p. 184-187° C.

EXAMPLE 3

N-[[[3,5-(dichlorophenyl)amino]carbonyl]oxy]-2-(dimethylamino)-2-oxoethanimidoyl chloride

DBU (10 drops) is added to a solution of 2-chloro-2-hydroxyimino-N,N-dimethylacetamide (15.0g) and 3,5-dichlorophenyl isocyanate (18.8g) in 200 ml of THF at room temperature. After stirring the reaction for two hours, the solvent is removed in vacuo. The solid is stirred overnight in 200 mL of ethyl acetate. The white solid is collected by vacuum filtration giving 29.4g (87%) of the title compound, m. p. = 180-182° C.

EXAMPLE 4

N-[[[[(3-trifluoromethyl)phenyl]amino]carbonyl]oxy)]-2-(dimethylamino)-2-oxoethanimidoyl chloride

DBU (3 drops) is added to a solution of 2-chloro-2-hydroxyimino-N,N-dimethylacetamide (3.0g) and 3-trifluoromethylphenyl isocyanate (3.7g) in 100 mL of THF at room temperature. After stirring the reaction for two hours, it is left undisturbed for two days. The solvent is removed in vacuo leaving an oil which slowly crystallized. After five hours, the solid is triturated with n-chlorobutane giving product as a white solid; yield 4.6g (69%), m. p. 128-132° C.

EXAMPLE 5

N-[[[3,4-(dichlorophenyl)amino]carbonyl]oxy]-2-(dimethylamino)-2-oxoethanimidoyl chloride

DBU (3 drops) is added to a solution of 2-chloro-2-hydroxyimino-N,N-dimethylacetamide (3.0g) and 3,4-dichlorophenyl isocyanate (3.75g) in 100 mL of THF at room temperature. After stirring the reaction overnight, the solvent is removed in vacuo. The solid is triturated with ethyl acetate giving product as a white solid; yield 2.4g (39%), m. p. = 144-146° C.

EXAMPLE 6

Ethyl-[[[[3,5-(dichlorophenyl)amino]carbonyl]oxy]imino]-2-chloroacetate

DBU (3 drops) is added to a solution of ethyl chlorooximinoacetate (3.0g) and 3,5-dichlorophenyl isocyanate (3.8g) in 100mL of THF at room temperature. After stirring the reaction overnight, the solvent is removed in vacuo. The solid is triturated with n-chlorobutane giving product as a white solid; yield 5.45g (80%), m. p. = 152-154° C.

## EXAMPLE 7

### N-[3,5-(dichlorobenzoyloxy)]-2-(dimethylamino)-2-oxoethanimidoyl chloride

A solution of 3,5-dichlorobenzoyl chloride (4.2g) in 20mL of THF is added to a solution of 2-chloro-2-hydroxyimino-N,N-dimethylacetamide (3.0g) in 80mL of THF at room temperature. Triethylamine (2.0g, 2.8mL) is added dropwise to the stirred solution maintaining the reaction temperature between 25-28° C with external cooling. Triethylamine hydrochloride precipitates, and the reaction is further stirred three hours. The solid is removed by vacuum filtration, and the filtrate removed in vacuo leaving product as a white solid; yield 6.2g (95%), m. p. = 92-95° C.

## EXAMPLE 8

### N-[[[4,6-(dichloro-2-pyrimidinyl)amino]carbonyl]oxy]-2-(dimethylamino)-2-oxoethanimidoyl chloride

DBU (3 drops) is added to a solution of 2-chloro-2-hydroxyimino-N,N-dimethylacetamide (3.0g) and 4,6-dichloro-2-pyrimidinyl isocyanate (3.8g) in 100mL of THF at room temperature. After stirring the reaction overnight, the solvent is removed in vacuo. The pale green solid is triturated with warm n-chlorobutane and collected by vacuum filtration from the warm suspension giving product as a white solid; yield 4.7g (69%), m. p. = 167-169° C.

## EXAMPLE 9

### N-[[[3,5-(dichlorophenyl)amino]carbonyl]oxy]-2-amino-2-oxoethanimidoyl chloride

DBU (3 drops) is added to a mixture of 2-chloro-2-hydroxyimino-acetamide (2.45g) and 3,5-dichlorophenyl isocyanate (3.8g) in 100ml of THF at room temperature. After 40 minutes, a fine white precipitate develops. After stirring the reaction overnight, the precipitate is collected by vacuum filtration giving product as a white solid; yield 2.9g (47%), m. p. = 224-226° C (dec).

## EXAMPLE 10

### N-[[4-(bromophenyl)sulfonyl]oxy]-2-(dimethylamino)-2-oxoethanimidoyl chloride

DBU (1.7mL) in 10ml of THF is added to a solution of 2-chloro-2-hydroxyimino-N,N-dimethylacetamide (1.5g) and 4-bromobenzenesulfonyl chloride (2.6 g) in 50mL of THF at room temperature. The amine salt precipitates, and the reaction is stirred an additional 24 hours. DBU (0.8mL) in 10ml of THF is added, and

the suspension is stirred another three hours. The precipitate is removed by vacuum filtration, and the filtrate reduced in vacuo leaving product as a white crystalline solid; yield 2.3g (63%), m. p. = 134-136° C.

## EXAMPLE 11

2-(dimethylamino)-N-[bis(2,2,2-trichloroethoxy)phosphinyloxy]-2-oxoethanimidoyl chloride

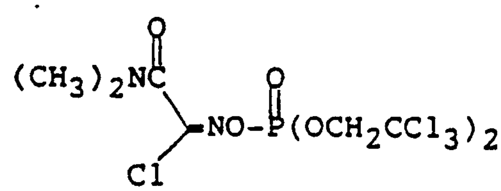

DBU (1.7mL) in 10 mL of THF is added to a solution of 2-chloro-1-hydroxyimino-N,N-dimethyl acetamide (1.5g) and bis(2,2,2-trichloroethyl) phosphorochloridate (3.8 g) at room temperature. The amine salt precipitates, and the reaction is stirred an additional 18 hours. The precipitate is removed by vacuum filtration. The filtrate is reduced in vacuo and the residue purified by flash chromatography on silica gel (1:2 hexane:ethyl acetate as eluant). The crude material is triturated in dichloromethane and hexane to give the product as a white solid; yield 850mg (17%), m. p. = 88-90° C.

## EXAMPLE 12

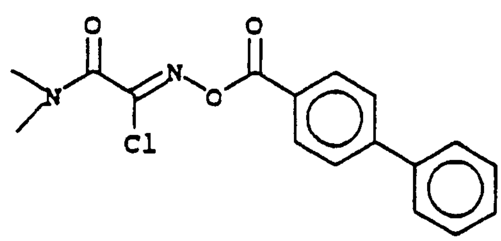

N-[4-Phenylbenzoyloxy]-2-dimethylamino-2-oxoethanimidoyl chloride

A solution of triethylamine (5.6 mL) in 80 mL of THF is added to a solution of 2-chloro-2-hydroximino-N,N-dimethyl-acetamide (6.0 g) and 4-biphenylcarboxylchloride (8.8 g) in 120 mL THF at room temperature. The amine salt precipitates, and the reaction mixture is stirred an additional 2 hours. The precipitate is removed by vacuum filtration, and the filtrate is reduced in vacuo leaving the crude reaction product as an off-white solid. The crude product is recrystallized from 1-chlorobutane to yield 5. 4 g (41%) off-white crystalline solid, m.p. = 126-128° C.

## EXAMPLE 13

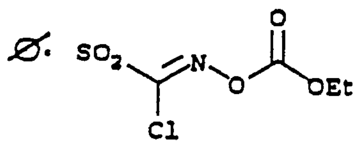

N-[Ethoxycarbonyloxy]-2-phenylsulfonylethanimidoyl chloride

A solution of triethylamine (5.6 mL) in 20 mL of THF is added dropwise to a solution of phenylsulfonylnitromethane (4.0 g) and ethyl chloroformate (3.9 mL) in 100 mL of THF at 0 °C. A precipitate forms and the reaction mixture turns orange. The reaction mixture is stirred an additional 2 hours at room temperature. The precipitate is removed by vacuum filtration and the filtrate is reduced in vacuo leaving the crude reaction product as an orange semi-solid. Trituration in 1-chlorobutane and hexane provides the crude product as a dark amber solid; crude yield 5.0 g, 86% yield. A 2.5 g portion of the crude product was further purified by flash chromatography (2:1 hexane:ethyl acetate as eluant). The material is triturated in 1-chlorobutane and hexane to yield 1.0 g of white solid, m.p. 82-84 °C.

## EXAMPLE 14

Ethanimidoyl chloride, 2-(dimethylamino)-N-(2-naphthalenylcarbonyloxy)-2-oxo-

To a stirred solution of 2-chloro-2-hydroxyimino-N,N-dimethylacetamide (10.52 g) in 150 mL of THF at 10 °C is added 2-naphthoyl chloride (13.34 g), follwed by triethylamine (9.76 mL). After 3.5 hours, the mixture is filtered and the filtrate evaporated in vacuum to a white solid. The solid is dissolved in ethyl acetate and the solution washed with water, 1N HCl, water, saturated sodium bicarbonate solution, and saturated brine, then dried ($MgSO_4$), filtered and evaporated in vacuum to a white solid residue. Recrystallization from 1-chlorobutane provided the title product as 14.43 g (68% of theoretical) of white solid, m.p. 141-143 °C.

## EXAMPLE 15

Ethanimidoyl bromide, 2-(dimethylamino)-N-(2-naphthalenylcarbonyl-oxy)-2-oxo

A solution of 2-chloro-2-hydroxyimino-N,N-dimethylacetamide (1.42 g) in 50 mL of THF was cooled to -15 °C, treated with N,N-diisopropyl-N-ethylamine (1.6 mL), and filtered after 2 minutes (to remove solid) directly into a solution of 2-naphthoyl bromide in 75 mL of THF at -50 °C. The mixture was stirred for 2 hours without cooling, then evaporated to an oily solid. Crystallization from 1-chlorobutane and filtration provided a solid and a filtrate. The filtrate was evaporated to a solid, and the solid recrystallized from hexane, providing the title product as a white solid, m.p. 128-130 °C.

Mass spectral analysis showed m/e 349/351 (m + 1 for title product, with 1 Br).

Analysis Calculated for $C_{15}H_{13}BrN_2O_3$: C, 51.6; H, 3.8; N, 8.0%.

Analysis Found: C, 51.8; H, 4.0; N, 8.0%.

## EXAMPLE 16

Ethanimidoyl chloride, N-(((1,1′-biphenyl))-2-yl)carbonyloxy))-2-(dimethylamino)-2-oxo-

To a solution of 2-chloro-2-hydroxyimino-N,N-dimethylacetamide (2.60 g) in 50 mL of N,N-dimethylformamide was added 2-biphenylcarboxylic acid (3.42 g), followed by N,N′-dicyclohexylcarbodiimide (3.56 g). The next day the mixture was filtered, the filtrate diluted with ice water, and the mixture extracted with ethyl acetate. The ethyl acetate solution was washed with water, 1N HCl, water, saturated sodium bicarbonate solution, and saturated brine, dried ($MgSO_4$), filtered and the filtrate evaporated to a colorless oil. Chromatography of the oil on silica gel with 25% EtOAc in $CHCl_3$ provided a solid, on evaporation of eluent, which was recrystallized from hexane to give the title product, m.p. 68-72° C.

EXAMPLE 17

Ethanimidoyl chloride, N-(((butylthio)carbonyloxy))-2-(dimethylamino)-2-oxo-

2-Chloro-2-hydroxyimino-N,N-dimethylacetamide (2.20 g) was dissolved in 100 ml THF. n-Butyl chlorothiolformate (2.23 g) was added to the solution at room temperature. The solution was cooled to 10° C, then triethylamine was added to the solution. The reaction was allowed to warm to room temperature. After stirring the reaction overnight, it was vacuum filtered and the filtrate was evaporated to an oil. The oil was dissolved in ethyl acetate and washed with water, 1N HCl, $NaHCO_3$ solution and brine. The ethyl acetate solution was dried over $MgSO_4$, vacuum filtered an the filtrate was evaporated to a yellow oil. The title compound was isolated as a colorless oil (0.64 g, 19%) after chromatographing the yellow oil on silica gel with chloroform, then chloroform/ethyl acetate, 9/1.

EXAMPLE 18

Ethanimidoyl chloride, N-(((4-aminophenyl)sulfonyloxy))-2-(dimethylamino)-2-oxo-

Into a stirred suspension of 7.77 g of ethanimidoyl chloride, 2-(dimethylamino)-N-(((4-nitrophenyl)-sulfonyloxy))-2-oxo- in 39.5 mL of acetic acid and 7.9 mL of water was added, portionwise over a 30 minute period, 6.87 g of iron powder. The temperature was kept at 20-24° C by application of an ice bath as needed. Thirty minutes after completion of the iron addition, the mixture was filtered and the solid rinsed with acetic acid.

Extraction of the solid by N,N-dimethylformamide (DMF) followed by dilution of the DMF extract with ice water, provided a precipitate of the title product as 5.3 g (75% of theor.) of white solid, m.p. 148° C, dec. Recrystallization from methylene chloride/hexane provided the analytical sample, m.p. 153-154° C, dec.

Analysis Calculated for $C_{10}H_{12}ClN_3O_4S$: C, 39.3, H, 4.0; N, 13.7%.

Analysis Found: C, 40.1; H, 4.2; N, 13.4%.

EXAMPLE 19

Ethanimidoyl chloride, 2-(dimethylamino)-2-oxo-N-(((phenylmethoxy)carbonyloxy))

Benzyl chloroformate (2.16 mL) was added to a solution of 2-chloro-2-hydroxyimino-N,N-dimethylacetamide (2.28 g) in 100 mL of THF; after the solution was cooled to 10° C, triethylamine (2.11 mL) was added, and the mixture stirred overnight without cooling. The mixture was filtered, the filtrate

evaporated to an oil, and the oil dissolved in ethyl acetate. The ethyl acetate solution was washed with water, 1N HCl, water and saturated sodium bicarbonate solution, dired ($Mg_5O_4$), and evaporated to an oil, which solidified. The solid was recrystallized from 1-chlorobutane/hexane, providing the title compound as a white solid, m.p. 84-86° C.

## EXAMPLE 20

### Preparation of chloroformate of 2-chloro-2-hydroxyimino-N,N-dimethylacetamide

Into a stirred, $N_2$-blanketed suspension of 2-chloro-2-hydroxyimino-N,N-dimethylacetamide (15.06 g, 0.1 mole) in toluene (150 mL) at 20° C was run liquid phosgene (~15 mL, ~0.22 mole). During a 10 minute period a solution of N,N-diethylaniline (17 mL, 0.11 mole) and pyridine (4 drops) in toluene (~10 mL) was added, with the temperature held to 20-24° C during the addition; the reaction was mildly exothermic during the addition and for a few minues thereafter.

After 23 hours the solid (N,N-diethylaniline hydrochloride) was filtered off and the filtrate stripped (38° C bath) to an oil which is the product chloroformate. It can be used directly, or further purified as indicated.

The oil was dissolved in BuCl (~100 mL), diluted to the cloud point with hexane, and restripped to an oil, which solidified to a light-orange solid under a nitrogen stream. The solid was dissolved in 100 mL of hot cyclohexane, and the supernatant decanted from a little insoluble oil; the oil was extracted with 25 mL of hot cyclohexane, and the combined, hot, turbid extracts treated with $MgSO_4$ (6 g) to soak up some of the oil present, and filtered.

The filtrate was stirred as it cooled until some grease, then some solid had formed, then filtered. The filtrate was stirred and cooled to 20° C, substantial white solid precipitating. After dilution with 20 mL of hexane, and cooling to 10° C, the mixture was filtered, and the white solid product dried overnight in a nitrogen-swept vacuum oven (33° C). Yield of white chloroformate: 11.4 g (54% of theor.); m.p. 68-70° C.

Other chloroformates

: 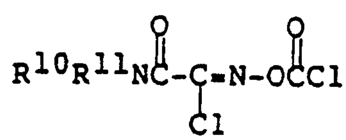

that can be prepared by similar methods include, but are not limited to those shown in the Table below:

| $R^{10}R^{11}N$ | m.p.(°C) |
|---|---|
| 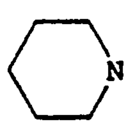 | 68-70 |
| 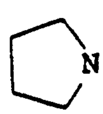 | |
| $Et_2N$ | |
| (Bu,Me)N | |

## EXAMPLE 21

Ethanimidoyl chloride, 2-(dimethylamino)-N-(((2,6-dimethylphenyl)aminocarbonyloxy))-2-oxo-

The chloroformate of 2-chloro-2-hydroxyimino-N,N-dimethylacetamide (2.08 g) was dissolved in 100 mL of THF. The solution was cooled to 0° C, then 2,6-dimethylaniline (1.2 mL) was added to the solution. Triethylamine (1.36 mL) was added to the solution with the temperature at 0° C. The reaction was allowed to warm to room temperature. After stirring the reaction for 2.5 hours, the reaction was vacuum filtered and the filtrate evaporated, leaving an orange oil. The oil was dissolved in ethyl acetate and washed with water, acid, $NaHCO_3$ and brine. The ethyl acetate solution was dried over $MgSO_4$, vacuum filtered and the filtrate was evaporated to a white solid. The solid was recrystallized from butyl chloride/hexane and collected by vacuum filtration giving 1.27 g (44%) of the title compound, m.p. 160-164° C.

## EXAMPLE 22

Preparation of ethanimidoyl chloride, N-(((3,5-dichlorophenyl)amino-carbonyloxy))-2-(dimethylamino)-2-thioxo

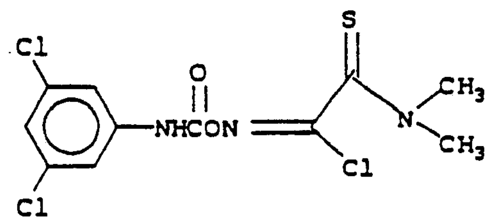

To a solution of 0.53 g ethanimidoyl chloride, N-(((3,5-dichlorophenyl)amino-carbonyloxy))-2-dimethylamino)-2-oxo dissolved in 50 mL of $CH_2Cl_2$ was added 0.35 g of Lawesson's reagent. The yellow solution was stirred for four days at room temperature. After concentration to one-half the original volume, the solution was flash chromatographed, eluting with 25% ethyl acetate/hexanes. The product had a Rf of 0.3 and 0.27 g (52%) of yellow solid were obtained; m.p. 156-157° C.

## EXAMPLE 23

Preparation of 1-piperidinethanimidoyl chloride, N-(((3,5-dichlorophenyl)-amino-carbonyloxy))-α-oxo

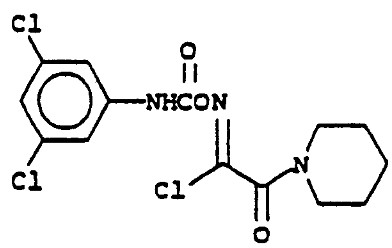

To a solution containing 9.53 g of 1-piperidineethanimidoyl chloride, N-hydroxy-α-oxo, and 9.40 g of 3,5-dichlorophenylisocyanate, dissolved in 300 mL THF, was added 3 drops of DBU. The solution was stirred at room temperature overnight. The solvent was removed in vacuo to yield a white solid. The material was recrystallized by dissolving in hot acetone, cooling, and adding hexanes to produce 12.7 g (67%) of the title compound, following vacuum filtration, m.p. 185-186 °C.

Preparation of 1-piperidineethanimidoyl chloride, N-hydroxy-α-oxo-

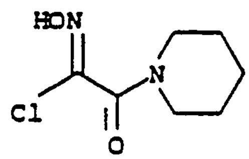

A suspension of 47.6 g of piperidine, 1-((2-(hydroxyimino)-1,3-dioxobutyl))- in a mixture of 100 mL $H_2O$ and 100 acetic acid was heated to 45 °C, then cooled to 30 °C. Chlorine gas (11 mL) was condensed and added dropwise to the suspension over a 1 hour period, while maintaining the temperature less than 35 °C. At the start of the chlorine addition, any remaining starting material dissolved. After half of the required amount of chlorine had been added, a white solid began to form. The mixture was stirred at room temperature for 15 minutes, following completion of the chlorine addition, then cooled to 2 °C. Vacuum filtration and cold water wash yielded a white solid which was recrystallized from boiling $CH_2Cl_2$. Yield -30.6 g (67%), m.p. 129-130 °C.

## EXAMPLE 24

Preparation of piperidine, 1-((2-hydroxyimino)-1,3-dioxobutyl))-

The above was prepared by dissolving 23.7 mL of piperidine in 100 mL $H_2O$. After cooling to 3 °C, diketene was added dropwise over 10 minutes, causing an exotherm to 32 °C. The mixture was stirred while cooling to 5 °C for 10 minutes. 16.6 g of sodium nitrite was added in three portions, keeping the temperature less than 10 °C whereupon the mixture was stirred for 30 minutes. Under a nitrogen blanket, 21 mL of concentrated HCl was added portionwise over 10 minutes, keeping the temperature <35 °C. After all of the acid had been added, the suspension was cooled to 2 °C and stirred at that temperature for 45 minutes. Vacuum filtration, followed by a cold water wash yielded an off-white solid which was partially dried by filtration suction. The material was used without further characterization in the above step.

The following Table lists representative compounds of this invention, which can be prepared by one or more of the methods illustrated in the preceding Examples. The Table is not intended to be all-inclusive.

The formula:

$X^1$-B- C= N-O-W-Z-$A^1$,    1(Ii)
    |
    Y

used at the head of the Table represents exactly the same compounds as the formula:

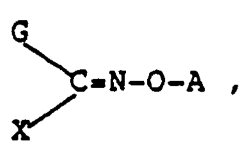

(I)

used earlier. The use of Formula (Ii) with the Table is intended to simplify the understanding of structures of compounds represented in that Table. Inspection of (Ii) and (I) will readily show that:
$X^1$-B of (Ii) is the same as G of (I),
Y of (Ii) is the same as X of (I),
W-Z-$A^1$ of (Ii) is the same as A of (I).

**TABLE 1**

$X^1$-B-C=N-O-W-Z-$A^1$
Y

| CMPD | $X^1$ | B | X | W | Z | $A^1$ | m.p. (°C) |
|---|---|---|---|---|---|---|---|
| 1 | $(Me)_2$-N | CO | Cl | CO | NH | 3,4-Cl-Ph | 144-146 |
| 2 | $(Me)_2$-N | CO | Cl | CO | NH | Ph | oil |
| 3 | $(Me)_2$-N | CO | Cl | CO | NH | 2,4-Cl-Ph | 137-138 |
| 4 | $(Me)_2$-N | CO | Cl | CO | NH | 2,5-Cl-Ph | 155-157 |
| 5 | $(Me)_2$-N | CO | Cl | CO | NH | 3-Cl-Ph | 85-96 |
| 6 | $(Me)_2$-N | CO | Cl | CO | NH | 4-Cl-Ph | 123-129 |
| 7 | $(Me)_2$-N | CO | Cl | CO | NH | 3,5-Me-Ph | 132-146 |
| 8 | $(Me)_2$-N | CO | Cl | CO | NH | 2,6-Cl-Ph | 182-185 |
| 9 | $(Me)_2$-N | CO | Cl | CO | NH | 3-F-Ph | 109-112 |
| 10 | $(Me)_2$-N | CO | Cl | CO | NH | 3-$CF_3$-Ph | 128-132 |
| 11 | $(Me)_2$-N | CO | Cl | CO | NH | 3-$NO_2$-Ph | 159-163 |
| 12 | $(Me)_2$-N | CO | Cl | CO | NH | 3-$OCH_3$-Ph | 70-80 |
| 13 | $(Me)_2$-N | CO | Cl | CO | NH | 1-naphthyl | 126-128 |
| 14 | $(Me)_2$-N | CO | Cl | CO | NH | 2,4,5-Cl-Ph | 184-186 |
| 15 | $(Me)_2$-N | CO | Cl | CO | NH | 3,5-$CF_3$-Ph | 184-187 |
| 16 | $(Me)_2$-N | CO | Cl | CO | NH | 3,4-F-Ph | 133 |
| 17 | $(Me)_2$-N | CO | Cl | CO | NH | 2,5-F-Ph | 111-113 |
| 18 | $(Me)_2$-N | CO | Cl | CO | NH | 3,5-$OCH_3$-Ph | 126-136 |
| 19 | $(Me)_2$-N | CO | Cl | CO | NH | 3,5-$CO_2CH_3$-Ph | 170-178 |
| 20 | $(Me)_2$-N | CO | Cl | CO | NH | 2,3-Cl-Ph | 126-129 |

| CMPD | $X^1$ | B | Y | W | Z | $A^1$ | m.p.(°C) |
|---|---|---|---|---|---|---|---|
| 21 | $(Me)_2$-N | CO | Cl | CO | NH | 4-Br-Ph | 143-145 |
| 22 | $(Me)_2$-N | CO | Cl | $SO_2$ | - | 3,5-Cl-Ph | 75-78 |
| 26 | $(Me)_2$-N | CO | Cl | $SO_2$ | - | 4-Cl-Ph | 153-155 |
| 27 | $(Me)_2$-N | CO | Cl | PO | $CH_2$ | $(O-CH_2-C-Cl_3)_2$ | 88-90 |
| 28 | $(Me)_2$-N | CO | Cl | $SO_2$ | - | 4-Me-Ph | 133-136 |
| 29 | $(Me)_2$-N | CO | Cl | $SO_2$ | - | 4-Br-Ph | 134-136 |
| 30 | OEt | CO | Cl | $SO_2$ | - | 4-Br-Ph | 82-84 |
| 31 | OEt | CO | Cl | $SO_2$ | - | 4-Cl-Ph | 62-65 |
| 32 | $(Me)_2$-N | CO | Cl | CO | NH | 3-CN-Ph | 175-179 |
| 33 | $(Me)_2$-N | CO | Cl | CO | NH | $3-COCH_3$-Ph | 168-169 |
| 34 | OEt | CO | Cl | CO | NH | 3,5-Cl-Ph | 152-154 |
| 35 | $NH_2$ | CO | Cl | CO | NH | 3,5-Cl-Ph | 224-226 |
| 36 | $(Me)_2$-N | CO | Cl | CO | NH | $3-SO_2CH_3$-Ph | 156-161 |
| 37 | $(Me)_2$-N | CO | Cl | CO | NH | $3-SCH_3$-Ph | 116-119 |
| 38 | $(Me)_2$-N | CO | Cl | CO | NH | 2-Cl-Ph | 104-107 |
| 39 | $(Me)_2$-N | CO | Cl | CO | $NHCH_2$ | Ph | 78-93 |
| 40 | $(Me)_2$-N | CO | Cl | CO | - | 3,5-Cl-Ph | 92-95 |
| 41 | $(Me)_2$-N | CO | Cl | CO | NH | 4,6-Cl-2-pyrimidino | 167-169 |
| 42 | $(Me)_2$-N | CO | Cl | CO | NH | 3,5-Cl-Ph | 168-169 |
| 43 | $(Me)_2$-N | CO | Cl | CO | NH | Me | 87-88 |

| CMPD | $X^1$ | B | Y | W | Z | $A^1$ | m.p.(°C) |
|---|---|---|---|---|---|---|---|
| 44 | $(Me)_2$-N | CO | Cl | CO | NH | 2,6-F-Ph | 136-146 |
| 45 | $(Me)_2$-N | CO | Cl | CO | NH | 2-$CF_3$-Ph | 116-118 |
| 46 | $(Me)_2$-N | CO | Cl | CO | NH | 2-$NO_2$-Ph | 130-134 |
| 47 | $(ME)_2$-N | CO | Cl | CO | NH | 2-Et-Ph | 93-98 |
| 48 | $(Me)_2$-N | CO | Cl | CO | NH | 2-$OCH_3$-Ph | 124-127 |
| 49 | $(Me)_2$-N | CO | Cl | CO | NH | 2-Me-Ph | 114-118 |
| 50 | $(Me)_2$-N | CO | Cl | CO | NH | 4-$OCH_3$-Ph | 112-116 |
| 51 | $(Me)_2$-N | CO | Cl | CO | NH | 3,5-Cl-4-SCN-Ph | 210-211 |
| 52 | Et,Me-N | CO | Cl | CO | NH | 3,4-Cl-Ph | 123-126 |
| 53 | Et,Me-N | CO | Cl | CO | NH | 3-Cl-Ph | 95-98 |
| 54 | Et,Me-N | CO | Cl | CO | NH | 3,5-Cl-Ph | 152-155 |
| 55 | Et,Me-N | CO | Cl | CO | NH | 2,6-Cl-Ph | 142-145 |
| 56 | Et,Me-N | CO | Cl | CO | NH | 3-$CF_3$-Ph | 112-114 |
| 57 | Et,Me-N | CO | Cl | CO | NH | 3,5-$CF_3$-Ph | 143-145 |
| 59 | pyrrolidino | CO | Cl | CO | - | 4-Cl-Ph | 114-115 |
| 60 | piperidino | CO | Cl | CO | - | 4-Cl-Ph | 84-86 |
| 61 | $(Me)_2$-N | CO | Cl | CO | NH | 3-Me-Ph | 73-76 |
| 62 | $(Me)_2$-N | CO | Cl | CO | NH | 4-Me-Ph | 101-104 |
| 63 | $(Me)_2$-N | CO | Cl | CO | CH=CH | Ph | oil |
| 64 | $(Me)_2$-N | CO | Cl | CO | - | 2,6-Me-Ph | 88-89 |

| CMPD | $X^1$ | B | X | W | Z | $A^1$ | m.p.(°C) |
|---|---|---|---|---|---|---|---|
| 65 | $(Me)_2$-N | CO | Cl | CO | CHCl | Ph | oil |
| 66 | $(Me)_2$-N | CO | Cl | CO | - | 2-furyl | 79-81 |
| 67 | $(Me)_2$-N | CO | Cl | CO | - | 2-naphthyl | 141-142 |
| 68 | $(Me)_2$-N | CO | Cl | CO | - | 1-naphthyl | 93-95 |
| 69 | $(Me)_2$-N | CO | Cl | CO | $CH_2$ | 1-naphthyl | grease |
| 70 | pyrrolidino | CO | Cl | CO | NH | 3-Cl-Ph | 107-111 |
| 71 | pyrrolidino | CO | Cl | CO | NH | 3,5-Cl-Ph | 150-152 |
| 72 | pyrrolidino | CO | Cl | CO | NH | 2,6-Cl-Ph | 190 |
| 73 | pyrrolidino | CO | Cl | CO | NH | 3,4-Cl-Ph | 137-140 |
| 74 | piperidino | CO | Cl | CO | NH | 3-Cl-Ph | 118-120 dec |
| 75 | piperidino | CO | Cl | CO | NH | 3,5-Cl-Ph | 185-186 |
| 76 | piperidino | CO | Cl | CO | NH | 2,6-Cl-Ph | 160-163 |
| 77 | piperidino | CO | Cl | CO | NH | 3,4-Cl-Ph | 123-125 |
| 78 | $(Me)_2$-N | CO | Cl | CO | $NHCH_2$ | 2-Cl-Ph | 115-119 |
| 79 | $(Me)_2$-N | CO | Cl | CO | $NHCH_2$ | 3,4-Cl-Ph | 83-87 |
| 80 | $(Me)_2$-N | CO | Cl | CO | NH | 3,5-F-Ph | 167-171 |
| 81 | $(Me)_2$-N | CO | Cl | CO | NH | 3,5-CN-Ph | 195-198 |
| 82 | $(Me)_2$-N | CO | Cl | CO | NH | 4-CN-Ph | 171-174 |
| 83 | $(Me)_2$-N | CO | Cl | CO | NH | 4-$COCH_3$-Ph | 149-152 |
| 84 | $(Me)_2$-N | CO | Cl | CO | - | 4-$OCH_3$-Ph | 115-116 |
| 85 | $(Me)_2$-N | CO | Cl | CO | - | 4-Cl-Ph | 97-98 |

| CMPD | $X^1$ | B | Y | W | Z | $A^1$ | m.p.(°C) |
|---|---|---|---|---|---|---|---|
| 86 | $(Me)_2$-N | CO | Cl | CO | - | 3-Cl-Ph | 100-101 |
| 87 | $(Me)_2$-N | CO | Cl | CO | - | 2-Cl-Ph | 99-100 |
| 88 | $(Me)_2$-N | CO | Cl | CO | - | 4-CN-Ph | 90-91 |
| 89 | $(Me)_2$-N | CO | Cl | CO | - | 4-$NO_2$-Ph | 104-105 |
| 90 | $(Me)_2$-N | CO | Cl | CO | - | 3-$NO_2$-Ph | 117-118 |
| 91 | $(Me)_2$-N | CO | Cl | CO | - | 2-$NO_2$-Ph | 88-89 |
| 92 | $(Me)_2$-N | CO | Cl | CO | - | 4-$CF_3$-Ph | 105-106 |
| 93 | $(Me)_2$-N | CO | Cl | CO | - | 3-$CF_3$-Ph | oil |
| 94 | $(Me)_2$-N | CO | Cl | CO | - | 2-$CF_3$-Ph | 86-87 |
| 95 | $(Me)_2$-N | CO | Cl | CO | - | 4-Me-Ph | 98-101 |
| 96 | $(Me)_2$-N | CO | Cl | CO | - | 3-Me-Ph | 73-75 |
| 97 | $(Me)_2$-N | CO | Cl | CO | - | 3-CN-Ph | 132-135 |
| 98 | Me-NH | CO | Cl | CO | NH | 3-Cl-Ph | 191-193 |
| 99 | Me-NH | CO | Cl | CO | NH | 3,4-Cl-Ph | 167-170 |
| 100 | Me-NH | CO | Cl | CO | NH | 3,5-Cl-Ph | 185-190 |
| 101 | Me-NH | CO | Cl | CO | NH | 2,6-Cl-Ph | >200 |
| 102 | Me-NH | CO | Cl | CO | NH | 3-$CF_3$-Ph | 170-173 |
| 103 | Me-NH | CO | CL | CO | NH | 3,5-$CF_3$-Ph | 195-100 |
| 104 | $(Me)_2$-N | CO | Cl | PO | - | $(O-Ph)_2$ | oil |
| 105 | $(Me)_2$-N | CO | Cl | $SO_2$ | - | $CH_3$ | 54-58 |
| 106 | $(Me)_2$-N | CO | Cl | CO | NH | 2-$CO_2CH_3$-Ph | 118-122 |

| CMPD | $X^1$ | B | X | W | Z | $A^1$ | m.p.(° C) |
|---|---|---|---|---|---|---|---|
| 107 | $(Me)_2$-N | CO | Cl | CO | NH | 4-i-Pr-Ph | 102-104 |
| 108 | $(Me)_2$-N | CO | Cl | CO | NH | 2-Me,6-Cl-Ph | 160-166 |
| 109 | $(Me)_2$-N | CO | Cl | CO | NH | 4-$CO_2CH_2CH_3$-Ph | 147-148 |
| 110 | $(Me)_2$-N | CO | Cl | CO | NH | 3-$NO_2$,4-F-Ph | 171-175 |
| 111 | $(Me)_2$-N | CO | Cl | CO | NH | 4-F-Ph | 98-101 |
| 112 | $(Me)_2$-N | CO | Cl | CO | NH | 4-$NO_2$-Ph | 154-157 |
| 113 | $(Me)_2$-N | CO | Cl | CO | NH | 4-$CF_3$-Ph | 123-129 |
| 114 | $(Me)_2$-N | CO | Cl | CO | O | 4-Me-Ph | 93-94 |
| 115 | $(Me)_2$-N | CO | Cl | CO | - | 2-Me-Ph | semi-solid |
| 116 | $(Me)_2$-N | CO | Cl | CO | - | 2-$OCH_3$-Ph | oil |
| 117 | $(Me)_2$-N | CO | Cl | CO | - | 3-$OCH_3$-Ph | oil |
| 118 | $(Me)_2$-N | CO | Cl | CO | NH | 2-i-Pr-Ph | 96-106 |
| 119 | $(Me)_2$-N | CO | Cl | CO | NH | 4-$SCH_3$-Ph | 76-80 |
| 120 | $(Me)_2$-N | CO | Cl | CO | NH | 2-$OCH_3$,4-$NO_2$-Ph | 154-158 |
| 121 | $(Me)_2$-N | CO | Cl | CO | - | 3,5-$CF_3$-Ph | 84-89 |
| 122 | morpholino | CO | Cl | CO | NH | 3,5-Cl-Ph | 140-176 |
| 123 | $(Me)_2$-N | CO | Cl | CO | NH | 4-$SO_2CH_3$-Ph | 35-75 |
| 124 | $(Me)_2$-N | CO | Cl | CO | - | 2,6-Cl-Ph | 113-116 |
| 125 | $(Me)_2$-N | CO | Cl | CO | - | Me | 50-55 |
| 126 | $(Et)_2$-N | CO | Cl | CO | NH | 3-Cl-Ph | 115-116 |

| CMPD | $X^1$ | B | Y | W | Z | $A^1$ | m.p.(°C) |
|---|---|---|---|---|---|---|---|
| 127 | $(Et)_2$-N | CO | Cl | CO | NH | 3,5-Cl-Ph | 160-161 |
| 128 | $(Et)_2$-N | CO | Cl | CO | NH | 3,5-$CF_3$-Ph | 154-160 |
| 129 | $(Et)_2$-N | CO | Cl | CO | NH | 3,4-Cl-Ph | 156-158 |
| 130 | $(Et)_2$-N | CO | Cl | CO | NH | 2,6-Cl-Ph | 116-120 |
| 131 | piperidino | CO | Cl | CO | NH | 3,5-$CF_3$-Ph | 157-161 |
| 132 | piperidino | CO | Cl | CO | NH | 3-$CF_3$-Ph | oil |
| 133 | pyrrolidino | CO | Cl | CO | NH | 3-$CF_3$-Ph | 119 |
| 135 | pyrrolidino | CO | Cl | CO | - | 3-$CF_3$-Ph | oil |
| 136 | Me-NH | CO | Cl | CO | - | 4-$NO_2$-Ph | 156-160 |
| 137 | Me-NH | CO | Cl | CO | - | 4-CN-Ph | 166-169 |
| 138 | Me-NH | CO | Cl | CO | - | 4-$CF_3$-Ph | 126-129 |
| 139 | Me-NH | CO | Cl | CO | - | 2-$CF_3$-Ph | 136-139 |
| 140 | Me-NH | CO | Cl | CO | - | 3-$NO_2$-Ph | 168-170 |
| 141 | Me-NH | CO | Cl | CO | - | 4-$OCH_3$-Ph | 133-135 |
| 142 | Me-NH | CO | Cl | CO | - | 4-Me-Ph | 125-127 |
| 143 | Me-NH | CO | Cl | CO | - | 4-Cl-Ph | 135-137 |
| 144 | Et,Me-N | CO | Cl | CO | - | 4-$NO_2$-Ph | 85-88 |
| 145 | Et,Me-N | CO | Cl | CO | - | 4-CN-Ph | 110-112 |
| 146 | Et,Me-N | CO | Cl | CO | - | 4-$CF_3$-Ph | 53-56 |

| CMPD | $X^1$ | B | Y | W | Z | $A^1$ | m.p.(°C) |
|---|---|---|---|---|---|---|---|
| 147 | Et,Me-N | CO | Cl | CO | - | $2-CF_3-Ph$ | oil |
| 148 | Et,Me-N | CO | Cl | CO | - | $3-NO_2-Ph$ | 55-58 |
| 149 | Et,Me-N | CO | Cl | CO | - | 4-Me-Ph | 50-52 |
| 150 | Et,Me-N | CO | Cl | CO | - | 4-Cl-Ph | 83-85 |
| 151 | $(Me)_2-N$ | CO | Cl | CO | $NHCH_2$ | 4-Cl-Ph | 105-108 |
| 152 | $(Me)_2-N$ | CO | Cl | CO | $CH_2$ | 2,6-Cl-Ph | 118-120 |
| 153 | $(Me)_2-N$ | CO | Cl | CO | - | 5-Me-2-thiophene | 86-87.5 |
| 154 | $(Me)_2-N$ | CO | Cl | CO | - | 5-Br-2-furan | 66-68 |
| 155 | $(Me)_2-N$ | CO | Cl | CO | - | 3-furan | 47-49 |
| 156 | $(Me)_2-N$ | CO | Cl | CO | $CH_2CH_2$ | Ph | oil |
| 157 | $(Me)_2-N$ | CO | Cl | CO | $CH_2$ | Ph | oil |
| 158 | $(Me)_2-N$ | CO | Cl | CO | - | 4-pyridine | gum |
| 159 | $(Me)_2-N$ | CO | Cl | CO | - | 3-pyridine | gum |
| 160 | $(Me)_2-N$ | CO | Cl | CO | - | t-Bu | 61.5-63 |
| 161 | $(Me)_2-N$ | CO | Cl | CO | - | $C(CH_3)_2CH_2Cl$ | oil |
| 162 | $(Me)_2-N$ | CO | Cl | CO | $NHCH_2$ | 2-thiophene | 113-116 |
| 163 | $(Me)_2-N$ | CO | Cl | CO | $NHCH_2$ | 3-Me-Ph | oil |
| 164 | $(Et)_2-N$ | CO | Cl | CO | $NHCH_2$ | 3,4-Cl-Ph | 93-98 |
| 165 | $(Et)_2-N$ | CO | Cl | CO | $NHCH_2$ | 2-Cl-Ph | 86-99 |
| 166 | $(Et)_2-N$ | CO | Cl | CO | $NHCH_2$ | 4-Cl-Ph | 79-82 |

| CMPD | $X^1$ | B | Y | W | Z | $A^1$ | m.p.(°C) |
|---|---|---|---|---|---|---|---|
| 167 | $(Et)_2$-N | CO | Cl | CO | $NHCH_2$ | 2-thiophene | 74-77 |
| 168 | $(Et)_2$-N | CO | Cl | CO | $NHCH_2$ | Ph | 86-91 |
| 169 | $(Me)_2$-N | CO | Cl | CO | - | 2-thiophene | 90-93 |
| 170 | $(Et)_2$-N | CO | Cl | CO | $NHCH_2$ | 3-Cl-Ph | 78-82 |
| 171 | $(Me)_2$-N | CO | Cl | CO | $CH_2$ | 2-thiophene | oil |
| 172 | $(Et)_2$-N | CO | Cl | CO | - | 3-Me-Ph | oil |
| 173 | $(Et)_2$-N | CO | Cl | CO | - | 3-$CF_3$-Ph | oil |
| 174 | $(Et)_2$-N | CO | Cl | CO | - | 3-$NO_2$-Ph | |
| 175 | $(Et)_2$-N | CO | Cl | CO | - | 3-Cl-Ph | oil |
| 176 | $(Et)_2$-N | CO | Cl | CO | - | 3-$OCH_3$-Ph | oil |
| 177 | $(Et)_2$-N | CO | Cl | CO | - | 3-CN-Ph | oil |
| 178 | $(Et)_2$-N | CO | Cl | CO | - | 3,5-$OCH_3$-Ph | oil |
| 179 | piperidino | CO | Cl | CO | - | 2-naphthyl | 52-54 |
| 180 | pyrrolidino | CO | Cl | CO | - | 2-naphthyl | 91 |
| 181 | i-Pr,Me-N | CO | Cl | CO | - | 2-naphthyl | 87-92 |
| 182 | $(i\text{-}Pr)_2$-N | CO | Cl | CO | NH | 3,5-$CF_3$-Ph | 182-189 dec |
| 183 | benzyl, Me-N | CO | Cl | CO | NH | 3,5-$CF_3$-Ph | 126-131 |
| 184 | i-Pr,Me-N | CO | Cl | CO | NH | 3,5-$CF_3$-Ph | 147-148 |
| 185 | pyrrolidino | CO | Cl | CO | NH | 3,5-$CF_3$-Ph | 158-161 |

| CMPD | $X^1$ | B | Y | W | Z | $A^1$ | m.p.(°C) |
|---|---|---|---|---|---|---|---|
| 186 | cyclohexyl,Me-N | CO | Cl | CO | NH | 3,5-Cl-Ph | 170-171 |
| 187 | $(Me)_2$-N | CO | Cl | CO | - | $(CH_2)_5-CH_3$ | oil |
| 188 | $(Me)_2$-N | CO | Cl | CO | - | $(CH_2)_8-CH_3$ | oil |
| 189 | $(Me)_2$-N | CO | Cl | CO | - | $(CH_2)_{12}-CH_3$ | 60-62 |
| 190 | $(Me)_2$-N | CO | Cl | CO | - | $(CH_2)_{16}-CH_3$ | 43-45 |
| 191 | $(Me)_2$-N | CO | Cl | CO | - | p-biphenyl | 124-127 |
| 192 | piperidino | CO | Cl | CO | NH | 3,5-F-Ph | 169-172 |
| 193 | pyrrolidino | CO | Cl | CO | NH | 3,5-F-Ph | 147-151 dec |
| 194 | $(i-Pr)_2$-N | CO | Cl | CO | NH | 3,5-F-Ph | 156-156.5 |
| 195 | $(Me)_2$-N | CO | Cl | CO | - | $CHBrCH_3$ | oil |
| 197 | - | SPh | Cl | CO | O | Et | oil |
| 198 | - | SOPh | Cl | CO | O | Et | oil |
| 199 | - | $SO_2Ph$ | Cl | CO | O | Et | 82-84 |
| 200 | $(Me)_2$-N | CO | Cl | CO | O | Et | 40-44 |
| 201 | OEt | CO | Cl | CO | O | Et | oil |
| 202 | $(Me)_2$-N | $SO_2$ | Cl | CO | O | Et | oil |
| 203 | $(i-Pr)_2$-N | CO | Cl | CO | NH | 2,6-Cl-Ph | 178-188 dec |
| 204 | $(CH_2CH_2OCH_3)_2$-N | CO | Cl | CO | NH | 2,6-Cl-Ph | gum |
| 205 | i-Pr,Me-N | CO | Cl | CO | NH | 3,5-Cl-Ph | 139-146 |
| 206 | $(i-Pr)_2$-N | CO | Cl | CO | - | 2-naphthyl | 158-159 |

| CMPD | $X^1$ | B | X | W | Z | $A^1$ | m.p.(°C) |
|---|---|---|---|---|---|---|---|
| 207 | $(CH_2CH_2OCH_3)_2$-N | CO | Cl | CO | - | 2-naphthyl | 48-51 |
| 208 | $(Me)_2$-N | CO | Br | CO | - | Ph | oil |
| 209 | $(n\text{-}Bu)_2$-N | CO | Cl | CO | NH | 3,5-Cl-Ph | 202-204 |
| 210 | $(n\text{-}Bu)_2$-N | CO | Cl | CO | NH | 3,5-$CF_3$-Ph | 92-96 |
| 211 | $(n\text{-}Bu)_2$-N | CO | Cl | CO | NH | 3-Cl-Ph | 194-196 |
| 212 | $(n\text{-}Bu)_2$-N | CO | Cl | CO | NH | 3,4-Cl-Ph | 185-187 |
| 213 | $(Me)_2$-N | CO | Cl | CO | - | 2,6-Me-Ph | 160-164 |
| 214 | piperidino | CO | Cl | CO | - | p-biphenyl | 111-113 |
| 215 | pyrrolidino | CO | Cl | CO | - | p-biphenyl | 123-126 |
| 216 | n-Bu,Me-N | CO | Cl | CO | NH | 3,5-Cl-Ph | 108-113 |
| 217 | 3-Me-piperidino | CO | Cl | CO | NH | 3,5-Cl-Ph | 172-175 |
| 218 | cyclohexyl,Me-N | CO | Cl | CO | NH | 3,5-Cl-Ph | 159-163 |
| 219 | $(CH_2CH_2OCH_3)_2$-N | CO | Cl | CO | NH | 3,5-Cl-Ph | 110-114 |
| 220 | n-Bu,Me-N | CO | Cl | CO | NH | 2,6-Cl-Ph | 203-205 dec |
| 221 | i-Pr,Me-N | CO | Cl | CO | NH | 2,6-Cl-Ph | 192-197 |
| 222 | cyclohexyl,Me-N | CO | Cl | CO | NH | 2,6-Cl-Ph | 212-216 |
| 223 | pyrrolidino | CO | Cl | CO | NH | 2,4,5-Cl-Ph | 137-141 dec |
| 224 | piperidino | CO | Cl | CO | NH | 2,4,5-Cl-Ph | 121-122 |
| 225 | $(i\text{-}Pr)_2$-N | CO | Cl | CO | NH | 2,4,5-Cl-Ph | 125-126 |
| 226 | benzyl,Me-N | CO | Cl | CO | NH | 2,4,5-Cl-Ph | 128-130 |

| CMPD | $X^1$ | B | Y | W | Z | $A^1$ | m.p.(°C) |
|---|---|---|---|---|---|---|---|
| 227 | cyclohexyl,Me-N | CO | Cl | CO | NH | 2,4,5-Cl-Ph | 111-117 |
| 228 | $(CH_2CH_2OCH_3)_2$-N | CO | Cl | CO | NH | 2,4,5-Cl-Ph | 102-105 |
| 229 | n-Bu,Me-N | CO | Cl | CO | NH | 3,5-$CF_3$-Ph | 119-121 |
| 230 | 3-Me-piperidino | CO | Cl | CO | NH | 3,5-$CF_3$-Ph | 162-163 |
| 231 | cyclohexyl,Me-N | CO | Cl | CO | NH | 3,5-$CF_3$-Ph | 176-177 |
| 232 | pyrrolidino | CO | Cl | CO | NH | 2,3,4,5-Cl-Ph | 152-155 dec |
| 233 | piperidino | CO | Cl | CO | NH | 2,3,4,5-Cl-Ph | 236 |
| 234 | $(Et)_2$-N | CO | Cl | CO | NH | 2,4,5-Cl-Ph | 146-148 |
| 235 | $(Et)_2$-N | CO | Cl | CO | - | 2-naphthyl | 81-82.5 |
| 236 | $(Me)_2$-N | CO | Cl | $SO_2$ | - | 4-$NO_2$-Ph | 114-118 |
| 237 | piperidino | CO | Cl | CO | - | 1-naphthyl | 91-98 |
| 238 | cyclohexyl,Me-N | CO | Cl | CO | - | 2-naphthyl | 127-129 |
| 239 | $(Me)_2$-N | CO | Cl | CO | - | 2-(1-Br-naphthyl) | 96-100 |
| 240 | t-Bu-NH | CO | Cl | CO | - | 2-naphthyl | 132-135 |
| 241 | t-Bu-NH | CO | Cl | CO | NH | 3,5-Cl-Ph | 116-118 |
| 242 | t-Bu-NH | CO | Cl | CO | NH | 3,5-$CF_3$-Ph | 167-168 |
| 243 | t-Bu-NH | CO | Cl | CO | NH | 2,4,5-Cl-Ph | 234-239 dec |
| 244 | 3,5-Me-piperidino | CO | Cl | CO | NH | 3,5-Cl-Ph | 176-178 |
| 246 | $(Me)_2$-N | CO | Br | CO | - | 2-naphthyl | 128-130 |

| CMPD | $X^1$ | B | Y | W | Z | $A^1$ | m.p.(°C) |
|---|---|---|---|---|---|---|---|
| 247 | piperidino | CO | Cl | CO | $NHCH_2$ | 2-Cl-Ph | oil |
| 248 | $(Me)_2$-N | CO | Cl | CO | NH | $CH(Ph)_2$ | oil |
| 249 | $(Me)_2$-N | CO | Cl | CO | NH | t-Bu | 72-74 |
| 250 | $(Me)_2$-N | CO | Cl | CO | NH | cyclohexyl | 76-79 |
| 251 | $(Me)_2$-N | CO | Cl | CO | - | cyclohexyl | 54-57 |
| 252 | $(Me)_2$-N | CO | Cl | CO | O | Bu | oil |
| 253 | $(Et)_2$-N | CO | Cl | CO | - | 2-$OCH_3$-Ph | |
| 254 | n-Bu,Me-N | CO | Cl | CO | O | 2-naphthyl | oil |
| 255 | piperidino | CO | Cl | CO | - | 2-(1-Br-naphthyl) | 116-121 |
| 256 | 3-Me-piperidino | CO | Cl | CO | NH | 2,4,5-Cl-Ph | 106-108 |
| 257 | i-Pr,Me-N | CO | Cl | CO | NH | 2,4,5-Cl-Ph | 210-214 |
| 258 | 3-Me-piperidino | CO | Cl | CO | NH | 2,3,4,5-Cl-Ph | 127-128 |
| 259 | $(Me)_2$-N | CO | Cl | CO | $OCH_2$ | Ph | 84-86 |
| 260 | $(Me)_2$-N | CO | Cl | CO | O | $CH_3$ | 56-69 |
| 261 | $(Me)_2$-N | CO | Cl | CO | NH | 2,6-i-Pr-Ph | 172-174 |
| 262 | $(Me)_2$-N | CO | Cl | CO | O | allyl | oil |
| 263 | $(Me)_2$-N | CO | Cl | CO | NH | 2,6-Et-Ph | 128-130 |
| 264 | $(Me)_2$-N | CO | Cl | CO | NH | 2,4,6-Me-Ph | 99-100 |
| 265 | $(Me)_2$-N | CO | Cl | CO | - | 3-$SO_2Cl$-Ph | 119-120 |
| 266 | O-i-Pr | CO | Cl | CO | - | 2-naphthyl | 85-87 |
| 267 | O-i-Pr | CO | Cl | CO | - | 4-Cl-Ph | 180-182 |

| CMPD | $X^1$ | B | Y | W | Z | $A^1$ | m.p.(°C) |
|---|---|---|---|---|---|---|---|
| 268 | O-i-Pr | CO | Cl | CO | - | 4-Br-Ph | 201-203 |
| 269 | O-i-Pr | CO | Cl | CO | - | 3,5-Cl-Ph | 91-93 |
| 270 | OEt | CO | Cl | CO | - | 2-naphthyl | 96-96.5 |
| 271 | OEt | CO | Cl | CO | - | 4-Cl-Ph | 110-112 |
| 272 | OEt | CO | Cl | CO | - | 4-Br-Ph | 110-112 |
| 273 | OEt | CO | Cl | CO | - | 3,5-Cl-Ph | 87-88 |
| 276 | O-t-Bu | CO | Cl | CO | - | 4-Cl-Ph | 84-86 |
| 277 | O-t-Bu | CO | Cl | CO | - | 4-Br-Ph | 83-85 |
| 278 | O-t-Bu | CO | Cl | CO | - | 3,5-Cl-Ph | 150-152 |
| 279 | Me | CO | Cl | CO | - | 2-naphthyl | 145-147 |
| 280 | Me | CO | Cl | CO | - | 4-Cl-Ph | 112-116 |
| 281 | Me | CO | Cl | CO | - | 4-Br-Ph | 136-138 |
| 282 | Me | CO | Cl | CO | - | 3,5-Cl-Ph | 104-106 |
| 283 | Ph | CO | Cl | CO | - | 4-Cl-Ph | 145-147 |
| 284 | Ph | CO | Cl | CO | - | 4-Br-Ph | 138-140 |
| 285 | Ph | CO | Cl | CO | - | 3,5-Cl-Ph | 99-101 |
| 286 | O-t-Bu | CO | Cl | CO | - | 2-naphthyl | 97-99 |
| 287 | azetidino | CO | Cl | CO | - | 2-naphthyl | 139-141 |

| CMPD | $X^1$ | B | Y | W | Z | $A^1$ | m.p.(°C) |
|---|---|---|---|---|---|---|---|
| 288 | 3-Me-piperidino | CO | Cl | CO | - | 2-naphthyl | oil |
| 289 | 3,5-Me-piperidino | CO | Cl | CO | - | 2-naphthyl | oil |
| 290 | homopiperidino | CO | Cl | CO | - | 2-naphthyl | oil |
| 291 | benzyl,Me-N | CO | Cl | CO | - | 2-naphthyl | gum |
| 292 | $NH_2$ | CO | Cl | CO | - | 2-naphthyl | 178-180 |
| 293 | pyrrolidino | CO | Cl | CO | - | 2-(1-Br-naphthyl) | 119-123 |
| 294 | azetidino | CO | Cl | CO | NH | 3,5-Cl-Ph | 184-185 |
| 295 | homopiperidino | CO | Cl | CO | NH | 3,5-Cl-Ph | 169-171 |
| 296 | i-Pr-NH | CO | Cl | CO | NH | 3,5-Cl-Ph | 221-227 |
| 297 | piperidino | CO | Cl | CO | - | 9-fluorenyl | 175-177 dec |
| 298 | piperidino | CO | Cl | CO | - | 4-$OCH_3$-Ph | 87-93 |
| 299 | pyrrolidino | CO | Cl | CO | - | 4-$OCH_3$-Ph | 74-76 |
| 300 | piperidino | CO | Cl | CO | - | 3-(6-Cl)pyridine) | 134-135 |
| 301 | $(Me)_2$-N | CO | Cl | CO | - | 3-(6-Cl-pyridine) | 111-112 |
| 302 | piperidino | CO | Cl | CO | - | 2-fluoren-9-onyl | 129-132 |
| 303 | $(Me)_2$-N | CO | Cl | CO | - | 2-fluoren-9-onyl | 153-154 |
| 304 | $(Me)_2$-N | CO | Cl | CO | - | 9-anthracenyl | 176-182 |
| 305 | $(Me)_2$-N | CO | Cl | CO | NH | 4-$NH_2$•HCl-Ph | 209 dec |
| 306 | $(Me)_2$-N | CO | Cl | CO | NH | 4-$CO_2H$-Ph | 199-200 |
| 307 | $(Me)_2$-N | CO | Cl | CO | - | Ph | 60-64 |

| CMPD | $X^1$ | B | X | W | Z | $A^1$ | m.p.(°C) |
|---|---|---|---|---|---|---|---|
| 308 | $(Me)_2$-N | CO | Cl | CO | - | 4-$SO_2$Cl-Ph | 91-93 |
| 309 | $(Me)_2$-N | CO | Cl | CO | - | 4-$NH_2$ HBr $H_2$O-Ph | 210 dec |
| 310 | $(Me)_2$-N | CO | Cl | CO | - | 4-$NH_2$-Ph | 153-154 |
| 311 | $(Me)_2$-N | CO | Cl | CO | - | 3-$SO_2NH_2$-Ph | 170-173 |
| 312 | $(Me)_2$-N | CO | Cl | CO | - | 2-$CO_2$-t-Bu-Ph | 86-89 |
| 313 | - | $SCH_2$Ph | Cl | CO | NH | 3,5-Cl-Ph | 98-102 |
| 314 | - | $SCH_2$Ph | Cl | CO | NH | 3,4-Cl-Ph | <50 |
| 315 | - | $SCH_2$Ph | Cl | CO | NH | 3-Cl-Ph | 88-92 |
| 316 | $(Me)_2$-N | CO | Cl | CO | - | S-n-Bu | oil |
| 317 | $(Me)_2$-N | CO | Cl | CO | - | o-biphenyl | 68-72 |
| 318 | $(Me)_2$-N | CO | Cl | CO | - | 4-O-cHexyl-Ph | 90-92 |
| 319 | $(Me)_2$-N | CO | Cl | CO | - | 3-quinolinyl | 111-114 |
| 320 | piperidino | CO | Cl | CO | - | 3-quinolinyl | 101-103 |
| 321 | $(Me)_2$-N | CO | Cl | CO | NH | 3,5-Cl-Ph | 156-157 |
| 322 | t-Bu-NH | CO | Cl | CO | NH | 2,6-Cl-Ph | 234 dec |
| 323 | n-Pr-NH | CO | Cl | CO | - | 2-naphthyl | 138-139 |
| 324 | $(Me)_2$-N | CO | Cl | CO | - | 9-phenanthracenyl | 129 |
| 325 | piperidino | CO | Cl | CO | - | 9-phenanthracenyl | 99-100 |
| 326 | $(Me)_2$-N | Co | Cl | CO | $C(CH_3)_2$ | 4-Cl-Ph | oil |
| 327 | piperidino | CO | Cl | CO | $C(CH_3)_2$ | 4-Cl-Ph | oil |
| 328 | piperidino | CO | Cl | CO | - | 9-anthracenyl | 169-181 |

| $X^1$ | B | Y | W | Z | $A^1$ |
|---|---|---|---|---|---|
| $NH_2$ | CO | Cl | CO | - | p-biphenyl |
| $NH_2$ | CO | Cl | CO | NH | Ph |
| $NH_2$ | CO | Cl | CO | NH | 2-Me-Ph |
| $NH_2$ | CO | Cl | CO | NH | 2-$CF_3$-Ph |
| $NH_2$ | CO | Cl | CO | NH | 2-Cl-Ph |
| $NH_2$ | CO | Cl | CO | NH | 3-Me-Ph |
| $NH_2$ | CO | Cl | CO | NH | 3-$CF_3$-Ph |
| $NH_2$ | CO | Cl | CO | NH | 3-Cl-Ph |
| $NH_2$ | CO | Cl | CO | NH | 2,6-Me-Ph |
| $NH_2$ | CO | Cl | CO | NH | 2-Cl,5-$CF_3$-Ph |
| $NH_2$ | CO | Cl | CO | NH | 2,6-Cl-Ph |
| $NH_2$ | CO | Cl | CO | NH | 3,5-$CF_3$-Ph |
| $NH_2$ | CO | Cl | CO | NH | 3,4-Cl-Ph |
| $NH_2$ | CO | Cl | CO | NH | 2,3,5-Cl-Ph |
| Me-NH | CO | Cl | CO | NH | Ph |
| Me-NH | CO | Cl | CO | NH | 2-Me-Ph |
| Me-NH | CO | Cl | CO | NH | 2-$CF_3$-Ph |
| Me-NH | CO | Cl | CO | NH | 2-Cl-Ph |
| Me-NH | CO | Cl | CO | NH | 3-Me-Ph |

| $X^1$ | B | Y | W | Z | $A^1$ |
|---|---|---|---|---|---|
| Me-NH | CO | Cl | CO | NH | 2,6-Me-Ph |
| Me-NH | CO | Cl | CO | NH | 2-Cl,5-$CF_3$-Ph |
| Me-NH | CO | Cl | CO | NH | 2,3-Cl-Ph |
| $(Me)_2$-N | CO | Cl | CO | NH | 2-Cl-Ph |
| $ClCH_2CH_2$-NH | CO | Cl | CO | NH | 2-Cl-Ph |
| $(Me)_2$-N | CO | Cl | CO | $CH_2$ | 3-Cl-Ph |
| sec-Bu-NH | CO | Cl | CO | NH | 3-Cl-Ph |
| morpholino | CO | CL | CO | NH | 3-Cl-Ph |
| n-Bu,Me-N | CO | Cl | CO | NH | 4-Cl-Ph |
| $(Me)_2$-N | CO | Cl | CO | NH | 3-I-Ph |
| $(Me)_2$-N | CO | Cl | CO | NH | 4-SCN-Ph |
| $(Me)_2$-N | CO | Cl | CO | $CH_2$ | 2-Me-Ph |
| $(Me)_2$-N | CO | Cl | CO | CHMe | 2-Me-Ph |

| $X^1$ | B | X | W | Z | $A^1$ |
|---|---|---|---|---|---|
| $(n-Pr)_2$-N | CO | Cl | CO | NH | 3-Me-Ph |
| $(Me)_2$-N | CO | Cl | CO | NH | 4-n-Pr-Ph |
| $(Me)_2$-N | CO | Cl | CO | NH | 4-n-Bu-Ph |
| $(Me)_2$-N | CO | Cl | CO | NH | 3-$CH_2$Cl-Ph |
| $(Me)_2$-N | CO | Cl | CO | NH | 3-$CH_2$Br-Ph |
| benzyl-NH | CO | Cl | CO | NH | 3-$CF_3$-Ph |
| Ph,Me-N | CO | Cl | CO | NH | 4-$CF_3$-Ph |
| $(Me)_2$-N | CO | Cl | CO | NH | 2-$CH_2$O-Me-Ph |
| $(Me)_2$-N | CO | Cl | CO | NH | 3-$CH_2$O-Me-Ph |
| $(Me)_2$-N | CO | Cl | CO | NH | 4-$CH_2$O-Me-Ph |
| $(Me)_2$-N | CO | Cl | CO | NH | 3-$CH_2OC_2H_5$-Ph |
| 4-Cl-benzyl-NH | CO | Cl | CO | NH | 2-OMe-Ph |
| $(Me)_2$-N | CO | Cl | CO | $NHCH_2$ | 3-OMe-Ph |
| 3,4-Cl-benzyl-NH | CO | Cl | CO | NH | 3-OMe-Ph |
| 2-Me-benzyl-NH | CO | Cl | CO | NH | 4-OMe-Ph |
| $(Me)_2$-N | CO | Cl | CO | NH | 3-OEt-Ph |
| $(Me)_2$-N | CO | Cl | CO | NH | 4-OPh-Ph |
| $(Me)_2$-N | CO | Cl | CO | NH | 3-$OCF_3$-Ph |

| $X^1$ | B | Y | W | Z | $A^1$ |
|---|---|---|---|---|---|
| 3-MeO-benzyl-NH | CO | Cl | CO | NH | $2-NO_2-Ph$ |
| $(Me)_2-N$ | CO | Cl | CO | NH | $3-NO_2-benzyl$ |
| $(Me)_2-N$ | CO | Cl | CO | NH | $4-NO_2-Ph$ |
| $(Me)_2-N$ | CO | Cl | CO | NH | $3-CO_2CH_2CH_2Cl-Ph$ |
| $(Me)_2-N$ | CO | Cl | CO | NH | $3-CONMe_2-Ph$ |
| $(Me)_2-N$ | CO | Cl | CO | NH | 3-CO(4-morpholino)-Ph |
| $(Me)_2-N$ | CO | Cl | CO | NH | $3-NMe_2-Ph$ |
| $(Me)_2-N$ | CO | Cl | CO | NH | 3-SEt-Ph |
| $(Me)_2-N$ | CO | Cl | CO | NH | $2-SCH_2CF_3-Ph$ |
| $(Me)_2-N$ | CO | Cl | CO | NH | $2-SO_2Me-Ph$ |
| $(Me)_2-N$ | CO | Cl | CO | NH | $2-SO_2NMe_2-Ph$ |
| $(Me)_2-N$ | CO | Cl | CO | NH | $3-SO_2NMe_2-Ph$ |
| $(Me)_2-N$ | CO | Cl | CO | NH | $4-SO_2NMe_2-Ph$ |
| 3-NC-Ph-NH | CO | Cl | CO | NH | 2,4-Cl-Ph |
| Et,Me-N | CO | Cl | CO | - | 2,6-Cl-Ph |
| $(Me)_2-N$ | CO | Cl | CO | NH | 2,6-Cl-Ph |

| $X^1$ | B | X | W | Z | $A^1$ | m.p.(°C) |
|---|---|---|---|---|---|---|
| $(Et)_2$-N | CO | Cl | CO | NH | 2,6-Cl-Ph | |
| sec-Bu-NH | CO | Cl | CO | NH | 2,6-Cl-Ph | |
| morpholino | CO | Cl | CO | NH | 2,6-Cl-Ph | |
| $(Me)_2$-N | CO | Cl | CO | - | 3,4-Cl-Ph | |
| $(Me)_2$-N | CO | Cl | CO | NH | 3,4-Cl-Ph | |
| $(Me)_2$-N | CO | Cl | CO | $CH_2$ | 3,4-Cl-Ph | |
| sec-Bu-NH | CO | Cl | CO | NH | 3,4-Cl-Ph | |
| morpholino | CO | Cl | CO | NH | 3,4-Cl-Ph | |
| 4-methylpyrazino | CO | Cl | CO | NH | 3,4-Cl-Ph | |
| 2,6-Me-morpholino | CO | Cl | CO | NH | 3,4-Cl-Ph | |
| $(Me)_2$-N | CO | Cl | CO | $NHCH_2$ | 3,5-Cl-Ph | |
| $(Me)_2$-N | CS | Cl | CO | NH | 3,5-Cl-Ph | 156-157 |
| - | $SO_2Me$ | Cl | CO | NH | 3,5-Cl-Ph | |
| - | $SO_2Ph$ | Cl | CO | NH | 3,5-Cl-Ph | |
| OMe | CO | Cl | CO | NH | 3,5-Cl-Ph | |
| OEt | CO | Cl | CO | $CH_2$ | 3,5-Cl-Ph | |
| - | $SO_2NMe_2$ | Cl | CO | NH | 3,5-Cl-Ph | |
| - | $SO_2NMe_2$ | Cl | CO | - | 3,5-Cl-Ph | |
| - | $SO_2NMe_2$ | Cl | CO | $CH_2$ | 3,5-Cl-Ph | |
| sec-Bu-NH | CO | Cl | CO | NH | 3,5-Cl-Ph | |

| $X^1$ | B | Y | W | Z | $A^1$ |
|---|---|---|---|---|---|
| $(Me)_2$-N | CO | Cl | CO | NH | 3-Cl,5-CN-Ph |
| $(Me)_2$-N | CO | Cl | CO | NH | 3-Cl,5-$COCH_3$-Ph |
| $(Me)_2$-N | CO | Cl | CO | NH | 2-Cl,4-Me-Ph |
| $(Me)_2$-N | CO | Cl | CO | NH | 3-Cl,4-Me-Ph |
| $(Me)_2$-N | CO | Cl | CO | NH | 3-Cl,5-Me-Ph |
| $(Me)_2$-N | CO | Cl | CO | NH | 4-Cl,2-Me-Ph |
| $(Me)_2$-N | CO | Cl | CO | NH | 5-Cl,2-Me-Ph |
| $(Me)_2$-N | CO | Cl | CO | NH | 2-Cl,4-$NO_2$-Ph |
| $(Me)_2$-N | CO | Cl | CO | NH | 2-Cl,5-$NO_2$-Ph |
| $(Me)_2$-N | CO | Cl | CO | NH | 3-Cl,5-$NO_2$-Ph |
| $(Me)_2$-N | CO | Cl | CO | NH | 4-Cl,2-$NO_2$-Ph |
| $(Me)_2$-N | CO | Cl | CO | NH | 4-Cl,3-$NO_2$-Ph |
| $(Me)_2$-N | CO | Cl | CO | NH | 5-Cl,2-$NO_2$-Ph |
| $(Me)_2$-N | CO | Cl | CO | NH | 2-Cl,5-$CF_3$-Ph |
| $(Me)_2$-N | CO | Cl | CO | NH | 3-Cl,5-$CF_3$-Ph |
| $(Me)_2$-N | CO | Cl | CO | NH | 4-Cl,3-$CF_3$-Ph |
| $(Me)_2$-N | CO | Cl | CO | NH | 2,3-Me-Ph |
| $(Me)_2$-N | CO | Cl | CO | NH | 2,4-Me-Ph |

| $X^1$ | B | X | W | Z | $A^1$ |
|---|---|---|---|---|---|
| $(Me)_2$-N | CO | Cl | CO | NH | 2,5-Me-Ph |
| $(Me)_2$-N | CO | Cl | CO | NH | 3,4-Me-Ph |
| $(Me)_2$-N | CO | Cl | CO | NH | 2,4-OMe-Ph |
| $(Me)_2$-N | CO | Cl | CO | NH | 2,5-OMe-Ph |
| $(Me)_2$-N | CO | Cl | CO | NH | 2,4-$NO_2$-Ph |
| $(Me)_2$-N | CO | Cl | CO | NH | 2,6-$NO_2$-Ph |
| $(Me)_2$-N | CO | Cl | CO | NH | 3,5-$NO_2$-Ph |
| $(Me)_2$-N | CO | Cl | CO | NH | 2,4-SCN-Ph |
| $(Me)_2$-N | CO | Cl | CO | NH | 3,5-$CF_3$-Ph |
| $(Me)_2$-N | CS | Cl | CO | NH | 3,5-$CF_3$-Ph |
| - | $SO_2Me$ | Cl | CO | NH | 3,5-$CF_3$-Ph |
| MeO | CO | Cl | CO | NH | 3,5-$CF_3$-Ph |
| EtO | CO | Cl | CO | - | 3,5-$CF_3$-Ph |
| EtO | CO | Cl | CO | $CH_2$ | 3,5-$CF_3$-Ph |
| EtO | CO | Cl | CO | NH | 3,5-$CF_3$-Ph |
| $(Et)_2$-N | CO | Cl | CO | NH | 3,5-$CF_3$-Ph |
| sec-Bu-NH | CO | Cl | CO | NH | 3,5-$CF_3$-Ph |
| pyrrolidino | CO | Cl | CO | NH | 3,5-$CF_3$-Ph |
| morpholino | CO | Cl | CO | NH | 3,5-$CF_3$-Ph |

| $X^1$ | B | Y | W | Z | $A^1$ |
|---|---|---|---|---|---|
| $(Me)_2$-N | CO | Cl | CO | NH | 2-Me,4-MeO-Ph |
| $(Me)_2$-N | CO | Cl | CO | NH | 5-Me,2-OMe-Ph |
| $(Me)_2$-N | CO | Cl | CO | NH | 2-Me,3-$NO_2$-Ph |
| $(Me)_2$-N | CO | Cl | CO | NH | 2-Me,4-$NO_2$-Ph |
| $(Me)_2$-N | CO | Cl | CO | NH | 2-Me,5-$NO_2$-Ph |
| $(Me)_2$-N | CO | Cl | CO | NH | 2-Me,6-$NO_2$-Ph |
| $(Me)_2$-N | CO | Cl | CO | NH | 3-Me,5-$NO_2$-Ph |
| $(Me)_2$-N | CO | Cl | CO | NH | 4-Me,2-$NO_2$-Ph |
| $(Me)_2$-N | CO | Cl | CO | NH | 4-Me,3-$NO_2$-Ph |
| $(Me)_2$-N | CO | Cl | CO | NH | 2-OMe,5-$NO_2$-Ph |
| $(Me)_2$-N | CO | Cl | CO | NH | 4-OMe,2-$NO_2$-Ph |
| $(Me)_2$-N | CO | Cl | CO | NH | 2-$NO_2$,4-$CF_3$-Ph |
| $(Me)_2$-N | CO | Cl | CO | NH | 4-$NO_2$,2-$CF_3$-Ph |
| $(Me)_2$-N | CO | Cl | CO | NH | 4-$NO_2$,3-$CF_3$-Ph |
| $(Me)_2$-N | CO | Cl | CO | NH | 5-$NO_2$,3-$CF_3$-Ph |
| $(Me)_2$-N | CO | Cl | CO | NH | 4-SCN-Ph |
| $(Me)_2$-N | CO | Cl | CO | NH | 2,4,6-Br-Ph |
| $(Me)_2$-N | CO | Cl | CO | NH | 2,3,4-Cl-Ph |
| $(Me)_2$-N | CO | Cl | CO | NH | 2,3,5-Cl-Ph |

| $X^1$ | B | X | W | Z | $A^1$ |
|---|---|---|---|---|---|
| $(Me)_2$-N | CO | Cl | CO | NH | 2,3,6-Cl-Ph |
| $(Me)_2$-N | CO | Cl | CO | NH | 2,4,6-Cl-Ph |
| $(Me)_2$-N | CO | Cl | CO | NH | 3,4,5-Cl-Ph |
| $(Me)_2$-N | CO | Cl | CO | NH | 2,3,5-Me-Ph |
| $(Me)_2$-N | CO | Cl | CO | NH | 2,4,6-Me-Ph |
| $(Me)_2$-N | CO | Cl | CO | NH | 3-SOMe-Ph |
| $(Me)_2$-N | CO | Cl | CO | NH | 4-Ph-Ph |
| $(Me)_2$-N | CO | Cl | CO | NH | 3-$CO_2$Ph-Ph |
| $(Me)_2$-N | CO | Cl | CO | NH | 3-$CO_2$Bzl-Ph |
| $(Me)_2$-N | CO | Cl | CO | NH | 3-$CO_2$allyl-Ph |
| $(Me)_2$-N | CO | Cl | CO | NH | 3-$CO_2CH_2CBr{=}CH$-Ph |
| $(Me)_2$-N | CO | Cl | CO | NH | 3-$CO_2$propargyl-Ph |
| $(Me)_2$-N | CO | Cl | CO | - | Et |
| $(Me)_2$-N | CO | Cl | CO | NH | Et |
| $(Me)_2$-N | CO | Cl | CO | NH | i-Pr |
| $(Me)_2$-N | CO | Cl | CO | NH | sec-Bu |
| $(Me)_2$-N | CO | Cl | CO | NH | $CH_2CH_2OMe$ |
| $(Me)_2$-N | CO | Cl | CO | NH | $CH_2CH_2Cl$ |
| $(Me)_2$-N | CO | Cl | CO | NH | propargyl |

| $X^1$ | B | X | W | Z | $A^1$ |
|---|---|---|---|---|---|
| $(Me)_2$-N | CO | Cl | CO | NH | allyl |
| $(Me)_2$-N | CO | Cl | CO | NH | 1-(2-Cl-naphthyl) |
| $(Me)_2$-N | CO | Cl | CO | NH | 1-(3-Cl-naphthyl) |
| $(Me)_2$-N | CO | Cl | CO | NH | 1-(4-Br-naphthyl) |
| $(Me)_2$-N | CO | Cl | CO | NH | 1-(2-Me-naphthyl) |
| $(Me)_2$-N | CO | Cl | CO | NH | 1-(4-CN-naphthyl) |
| $(Me)_2$-N | CO | Cl | CO | NH | 1-(2-OMe-naphthyl) |
| $(Me)_2$-N | CO | Cl | CO | NH | 1-(4-OMe-naphthyl) |
| $(Me)_2$-N | CO | Cl | CO | NH | 1-(7-OMe-naphthyl) |
| $(Me)_2$-N | CO | Cl | CO | NH | 1-(2,4-Cl-naphthyl) |
| $(Me)_2$-N | CO | Cl | CO | NH | 2-naphthyl) |
| $(Me)_2$-N | CO | Cl | CO | NH | 2-(1-$NO_2$-naphthyl) |
| $(Me)_2$-N | CO | Cl | CO | NH | 2-(3-OMe-naphthyl) |
| $(Me)_2$-N | CO | Cl | CO | NH | 2-(5-OMe-naphthyl) |
| $(Me)_2$-N | CO | Cl | CO | NH | 2-(7-OMe-naphthyl) |
| $(Me)_2$-N | CO | Cl | CO | $CH_2$ | 2-naphthyl |
| $(Me)_2$-N | CO | Cl | CO | NH | 2-furan |
| $(Me)_2$-N | CO | Cl | CO | NH | 2-thiophene |
| $(Me)_2$-N | CO | Cl | CO | NH | 2-(1-Me-pyrrole) |

| $X^1$ | B | Y | W | Z | $A^1$ |
|---|---|---|---|---|---|
| $(Me)_2$-N | CO | Cl | CO | NH | 2-(1-Me-imidazole) |
| $(Me)_2$-N | CO | Cl | CO | NH | 2-oxazole |
| $(Me)_2$-N | CO | Cl | CO | NH | 2-thiazole |
| $(Me)_2$-N | CO | Cl | CO | NH | 5-(1-Me-2-$CO_2$Me-pyrazole) |
| $(Me)_2$-N | CO | Cl | CO | NH | 4-(1,3-Me-5-Cl-pyrazole) |
| $(Me)_2$-N | CO | Cl | CO | NH | 4-(1,3-Me-5-CN-pyrazole) |
| $(Me)_2$-N | CO | Cl | CO | NH | 5-(1-Me,4-$CONMe_2$-pyrazole) |
| $(Me)_2$-N | CO | Cl | CO | NH | 5-(1-Me,4-$SO_2NMe_2$-pyrazole) |
| $(Me)_2$-N | CO | Cl | CO | NH | 5-(1-Me,4-$NO_2$-pyrazole) |
| $(Me)_2$-N | CO | Cl | CO | NH | 4-(1-Me,5-Cl-pyrazole) |
| $(Me)_2$-N | CO | Cl | CO | NH | 2-(4,6-OMe-pyrimidine) |
| $(Me)_2$-N | CO | Cl | CO | NH | 2-(4-$OCF_3$,6-Me-s-triazine) |
| $(Me)_2$-N | CO | Cl | CO | NH | 2-(4-$NMe_2$,6-OEt-s-triazine) |
| $(Me)_2$-N | CO | Cl | CO | NH | 2-(4,6-Cl-s-triazine) |
| $(Me)_2$-N | CO | Cl | CO | NH | 3-pyridine |
| $(Me)_2$-N | CO | Cl | CO | NH | 2-(4,6-Cl-pyridine) |
| $(Me)_2$-N | CO | Cl | CO | NH | 2-(3,5,6-Cl-1,4-pyrazine) |
| $(Me)_2$-N | CO | Cl | CO | NH | 3-(6-OMe-pyridine) |
| $(Me)_2$-N | CO | Cl | $Me_2$-PO | - | - |

| $X^1$ | B | X | W | Z | $A^1$ | m.p.(°C) |
|---|---|---|---|---|---|---|
| $(Me)_2$-N | CO | Cl | Et,$ClCH_2CH_2$-PO | - | - | |
| $(Me)_2$-N | CO | Cl | Ph-PO | - | OMe | |
| $(Me)_2$-N | CO | Cl | PO | - | $(OBu)_2$ | |
| $(Me)_2$-N | CO | Cl | PO | - | $(OEt)_2$ | |
| $(Me)_2$-N | CO | Cl | PO | - | OEt,OPr | |
| $(Me)_2$-N | CO | Cl | Ph-PO | - | OEt | |
| $(Me)_2$-N | CO | Br | CO | - | 3,5-Cl-Ph | |
| $(Me)_2$-N | CO | Br | CO | $CH_2$ | 3,5-Cl-Ph | |
| $Me_2$-N | CO | Cl | CO | - | 3-quinoline | 111-114 |
| pyrrolidine | CO | Cl | CO | - | 6-quinoline | |
| piperidine | CO | Cl | CO | - | 2-quinoline | |
| Bu,Me-N | CO | Cl | CO | - | 2-quinoxaline | |
| Ph | $SO_2$ | Cl | CO | - | 5-benzimidazole | |
| $Me_2$-N | CS | Cl | CO | - | 2-benzofuran | |
| PrO | CO | Cl | CO | - | 5-benzotriazole | |
| NC- | - | Cl | CO | - | 2-benzoxazole | |
| $Me_2$-N | CO | Br | CO | - | 5-(2,1,3-benzothiadiazole) | |
| butyl | CO | Cl | CO | - | 2-benzothiazole | |
| $Et_2$-N | CO | Cl | CO | - | 2-thianophthene | |

| $X^1$ | B | X | W | Z | $A^1$ |
|---|---|---|---|---|---|
| $Me_2$-N | CO | Cl | CO | - | 5-quinoline |
| pyrrolidine | CO | Cl | CO | - | 2-(3-Cl-thianaphthene) |
| piperidine | CO | Cl | CO | - | 2-(7-Cl-benzofuran) |
| $Me_2$-N | CO | Cl | CO | - | 2-(7-OMe-benzofuran) |
| $Et_2$-N | CO | Cl | CO | - | 2-(5-nitrobenzofuran) |
| Bu,Me-N | CO | Cl | CO | - | 2-(5-Cl-benzofuran) |
| pyrrolidine | CO | Cl | CO | - | 2-(6,7$(OMe)_2$benzofuran) |
| piperidine | CO | Cl | CO | - | 2-(3-Me-benzofuran) |
| $Me_2$-N | CO | Cl | CO | - | 2-(5-OMe-indole) |
| $Me_2$-N | CO | Cl | CO | - | 2-azetidine |
| pyrrolidine | CO | Cl | CO | - | 2-piperidine |
| piperidine | CO | Cl | CO | - | 3-piperidine |
| EtO | CO | Cl | CO | - | 4-piperidine |
| $Me_2$-N | CS | Cl | CO | - | 2-(2-methylpiperidine) |
| phenyl | CO | Cl | CO | - | 2-pyrrolidine |
| butyl | CO | Cl | CO | - | 2-(2-methylpyrrolidine) |
| -CN | - | Cl | CO | - | 2-homopiperazine |
| phenyl | $SO_2$ | Cl | CO | - | 3-morpholine |
| $Me_2$-N | $SO_2$ | Cl | CO | - | 2-tetrahydrothiophene |

| $X^1$ | B | Y | W | Z | $A^1$ |
|---|---|---|---|---|---|
| Bu,Me-N | CO | Cl | CO | - | 2-(1,3-dithiane) |
| $Me_2$-N | CO | Br | CO | - | 2-(1,3-dithiolane) |
| pyrrolidine | CO | Cl | CO | - | 4-thiazolidine |
| $Me_2$-N | CO | Cl | $SO_2$ | - | $CF_3$ |
| pyrrolidine | CO | Cl | $SO_2$ | - | butyl |
| piperidine | CO | Cl | $SO_2$ | - | 2,5-$Cl_2$-Ph |
| $Me_2$-N | CS | Cl | $SO_2$ | - | 4-$CF_3$-Ph |
| Ph | CO | Cl | $SO_2$ | - | 2,4,5-$Cl_3$-Ph |
| BuO | CO | Cl | $SO_2$ | - | 3-$NH_2$,4-Cl-Ph |
| NC | - | Cl | $SO_2$ | - | 3-$OCH_3$-Ph |
| phenyl | $SO_2$ | Cl | $SO_2$ | - | 4-Ph-Ph |
| $Me_2$-N | $SO_2$ | Cl | $SO_2$ | - | 2,4$(NO_2)_2$-Ph |
| $Me_2$-N | CO | Br | $SO_2$ | - | 2-naphthalene |
| $Me_2$-N | CO | Cl | CO | - | 5-piperonene |
| piperdine | CO | Cl | CO | - | 2-(1,2,3,4-tetrahydronaphthalene) |
| pyrrolidine | CO | Cl | CO | - | 2-(1,4-benzodioxane) |
| butyl | CO | Cl | CO | - | 6-(1,4-benzodioxane) |
| Ph | $SO_2$ | Cl | CO | - | 6-chromane |
| i-Pr-O | CO | Cl | CO | - | 8-chromane |
| $Et_2N$ | CO | Cl | CO | - | 5-(1,2,3,4-tetrahydronaphthalene) |

58

| $X^1$ | B | Y | W | Z | $A^1$ |
|---|---|---|---|---|---|
| $(Me)_2$-N | CO | Cl | CO | - | $C_{20}H_{41}$ |
| $(Me)_2$-N | CO | Cl | CO | NH | $C_{20}H_{41}$ |
| $(Me)_2$-N | CO | Cl | CO | - | $CH=CH_2$ |
| $(Me)_2$-N | CO | Cl | CO | NH | $CH_2CH=CH_2$ |
| $(Me)_2$-N | CO | Cl | CO | - | $CH=CH(CH_2)_{17}CH_3$ |
| $(Me)_2$-N | CO | Cl | CO | NH | $CH_2CH=CH(CH_2)_{16}CH_3$ |
| $(Me)_2$-N | CO | Cl | CO | - | $C\equiv CH$ |
| $(Me)_2$-N | CO | Cl | CO | NH | $CH_2C\equiv CH$ |
| $(Me)_2$-N | CO | Cl | CO | - | $C\equiv C(CH_2)_{17}CH_3$ |
| $(Me)_2$-N | CO | Cl | CO | NH | $CH_2C\equiv C(CH_2)_{16}CH_3$ |
| $(Me)_2$-N | CO | Cl | CO | - | cyclopropyl |
| $(Me)_2$-N | CO | Cl | CO | NH | cyclopropyl |
| $(Me)_2$-N | CO | Cl | CO | - | cycloheptyl |
| $(Me)_2$-N | CO | Cl | CO | NH | cycloheptyl |
| $(Me)_2$-N | CO | Cl | CO | - | $CH_2OMe$ |
| $(Me)_2$-N | CO | Cl | CO | NH | $CH_2CH_2OMe$ |
| $(Me)_2$-N | CO | Cl | CO | - | $CH_2O(CH_2)_5CH_3$ |
| $(Me)_2$-N | CO | Cl | CO | - | $CHCl_2$ |
| $(Me)_2$-N | CO | Cl | CO | - | $CH_2CH_2SMe$ |

| $X^1$ | B | X | W | Z | $A^1$ |
|---|---|---|---|---|---|
| $(Me)_2$-N | CO | Cl | CO | NH | 4-OEt-Ph |
| $(Me)_2$-N | CO | Cl | CO | NH | 4-$CH_2CH=CH_2$-Ph |
| $(Me)_2$-N | CO | Cl | CO | NH | 4-$CH_2CH=CHCH_2Cl$-Ph |
| $(Me)_2$-N | CO | Cl | CO | NH | 4-$CH_2C\equiv CH$-Ph |
| $(Me)_2$-N | CO | Cl | CO | NH | 4-o-cyclohexyl-Ph |
| $(Me)_2$-N | CO | Cl | CO | NH | 4-cyclohexyl-Ph |
| $(Me)_2$-N | CO | Cl | CO | NH | 4-$SO_2F$-Ph |
| $(Me)_2$-N | CO | Cl | CO | NH | 4-$SO_3CH_2CF_3$-Ph |
| $(Me)_2$-N | CO | Cl | CO | NH | 4-(4-pyridyl)-Ph |
| $(Me)_2$-N | CO | Cl | CO | NH | 4-OPh-Ph |
| $(Me)_2$-N | CO | Cl | CO | NH | 4-(4-Cl-Ph)-Ph |
| $(Me)_2$-N | CO | Cl | CO | NH | 4-OBu-Ph |
| $(Me)_2$-N | CO | Cl | CO | - | 4-OBu-Ph |
| $(Me)_2$-N | CO | Cl | CO | - | 4-$CH_2CH_2Cl$-Ph |
| $(Me)_2$-N | CO | Cl | CO | - | 4-OEt-Ph |
| $(Me)_2$-N | CO | Cl | CO | - | 4-$CH_2CH=CH_2$-Ph |
| $(Me)_2$-N | CO | Cl | CO | - | 4-$CH_2CH=CH_2CH_2Cl$-Ph |
| $(Me)_2$-N | CO | Cl | CO | - | 4-$CH_2C\equiv CH$-Ph |
| $(Me)_2$-N | CO | Cl | CO | - | 4-cyclohexyl-Ph |

| $X^1$ | B | X | W | Z | $A^1$ |
|---|---|---|---|---|---|
| $(Me)_2$-N | CO | Cl | CO | - | $CH_2S(CH_2)_5CH_3$ |
| $(Me)_2$-N | CO | Cl | CO | $CH_2$ | cyclopropyl |
| $(Me)_2$-N | CO | Cl | CO | $CH_2$ | cyclohexyl |
| $(Me)_2$-N | CO | Cl | CO | $CH_2$ | CN |
| $(Me)_2$-N | CO | Cl | CO | $CH_2$ | 4-Cl-Ph |
| $(Me)_2$-N | CO | Cl | CO | $CH_2$ | 4-F-Ph |
| $(Me)_2$-N | CO | Cl | CO | $CH_2$ | 4-Br-Ph |
| $(Me)_2$-N | CO | Cl | CO | $CH_2$ | 2,4,6-Cl-Ph |
| $(Me)_2$-N | CO | Cl | CO | $CH_2$ | 4-Me-Ph |
| $(Me)_2$-N | CO | Cl | CO | $CH_2$ | 3-$CF_3$-Ph |
| $(Me)_2$-N | CO | Cl | CO | $CH_2$ | 4-OMe-Ph |
| $(Me)_2$-N | CO | Cl | CO | $CH_2$ | 4-CN-Ph |
| $(Me)_2$-N | CO | Cl | CO | $CH_2$ | 4-SMe-Ph |
| $(Me)_2$-N | CO | Cl | CO | $CH_2$ | 4-$SO_2Me$-Ph |
| $(Me)_2$-N | CO | Cl | CO | $CH_2$ | 4-$NMe_2$Ph |
| $(Me)_2$-N | CO | Cl | CO | $CH_2$ | 4-$NH_2$-Ph |
| $(Me)_2$-N | CO | Cl | CO | $CH_2$ | 4-Ph-Ph |
| $(Me)_2$-N | CO | Cl | CO | $CH_2$ | 4-OPh-Ph |
| $(Me)_2$-N | CO | Cl | CO | NH | 4-$CH_2CH_2Cl$-Ph |

| $X^1$ | B | Y | W | Z | $A^1$ |
|---|---|---|---|---|---|
| $(Me)_2$-N | CO | Cl | CO | - | 4-$SO_2F$-Ph |
| $(Me)_2$-N | CO | Cl | CO | - | 4-$SO_3CH_2CF_3$-Ph |
| $(Me)_2$-N | CO | Cl | CO | - | 4-(4-pyridyl)-Ph |
| $(Me)_2$-N | CO | Cl | CO | - | 4-OPh-Ph |
| $(Me)_2$-N | CO | Cl | CO | - | 4-(4-Cl-Ph)-Ph |
| $(Me)_2$-N | CO | Cl | CO | - | 2-(6-Me-naphthyl) |
| $(Me)_2$-N | CO | Cl | CO | - | 4-$CO_2CH_2CH_2Cl$-Ph |
| $(Me)_2$-N | CO | Cl | CO | - | 4-$CO_2CH_2OMe$-Ph |
| $(Me)_2$-N | CO | Cl | CO | - | 4-$CO_2CH_2C{\equiv}CCH_2Cl$-Ph |
| $(Me)_2$-N | CO | Cl | CO | - | 4-$CO_2CH_2$Ph-Ph |
| $(Me)_2$-N | CO | Cl | CO | - | 4-$OCH_2$Ph-Ph |
| $(Me)_2$-N | CO | Cl | CO | - | 3-CONHMe-Ph |
| $(Me)_2$-N | CO | Cl | CO | O | $(CH_2)_{19}-CH_3$ |
| $(Me)_2$-N | CO | Cl | CO | O | $CH_2CH=CH(CH_2)_{16}CH_3$ |
| $(Me)_2$-N | CO | Cl | CO | O | $CH_2C{\equiv}CH$ |
| $(Me)_2$-N | CO | Cl | CO | O | $CH_2C{\equiv}C(CH_2)_{16}CH_3$ |
| $(Me)_2$-N | CO | Cl | CO | O | Me |
| $(Me)_2$-N | CO | Cl | CO | O | 4-cyclohexyl-Ph |

| $X^1$ | B | Y | W | Z | $A^1$ |
|---|---|---|---|---|---|
| $(Me)_2$-N | CO | Cl | CO | O | $(CH_2)_2OMe$ |
| $(Me)_2$-N | CO | Cl | CO | O | $(CH_2)_2Cl$ |
| $(Me)_2$-N | CO | Cl | CO | O | $(CH_2)_2SMePh$ |
| $(Me)_2$-N | CO | Cl | CO | O | $CH_2$-cyclopropyl |
| $(Me)_2$-N | CO | Cl | CO | O | $CH_2$-cyclohexyl |
| $(Me)_2$-N | CO | Cl | CO | O | $CH_2CH_2CN$ |
| $(Me)_2$-N | CO | Cl | CO | O | $(CH_2)_8Cl$ |
| $(Me)_2$-N | CO | Cl | CO | $O(CH_2)_2$ | Ph |
| $(Me)_2$-N | CO | Cl | CO | $OCH_2$ | 4-Me-Ph |
| $(Me)_2$-N | CO | Cl | CO | $OCH_2$ | 4-Cl-Ph |
| $(Me)_2$-N | CO | Cl | CO | $OCH_2$ | 4-F-Ph |
| $(Me)_2$-N | CO | Cl | CO | $OCH_2$ | 4-$NO_2$-Ph |
| $(Me)_2$-N | CO | Cl | CO | $OCH_2$ | 3-$CF_3$-Ph |
| $(Me)_2$-N | CO | Cl | CO | $OCH_2$ | 3-CN-Ph |
| $(Me)_2$-N | CO | Cl | CO | $OCH_2$ | 4-OMe-Ph |
| $(Me)_2$-N | CO | Cl | CO | $OCH_2$ | 4-SMe-Ph |
| $(Me)_2$-N | CO | Cl | CO | $OCH_2$ | 4-$SO_2$Me-Ph |
| $(Me)_2$-N | CO | Cl | CO | $OCH_2$ | 4-$NMe_2$-Ph |
| $(Me)_2$-N | CO | Cl | CO | $OCH_2$ | 4-$NH_2$-Ph |

| $X^1$ | B | X | W | Z | $A^1$ | m.p.(°C) |
|---|---|---|---|---|---|---|
| $(Me)_2$-N | CO | Cl | CO | $OCH_2$ | 4-OPh-Ph | |
| $(Me)_2$-N | CO | Cl | CO | S | Me | |
| $(Me)_2$-N | CO | Cl | CO | S | $(CH_2)_{19}CH_3$ | |
| $(Me)_2$-N | CO | Cl | CO | $SCH_2$ | Ph | |
| $(Me)_2$-N | CO | Cl | CO | $SCH_2$ | 4-Cl-Ph | |
| $(Me)_2$-N | CO | Cl | CO | $OCH_2$ | 4-Ph-Ph | 142-143 |
| $(Me)_2$-N | CO | Cl | CO | $OCH_2$ | 4-OPh-Ph | |
| $(Pr)_2$-N | CO | Cl | CO | NH | 3,5-Cl-Ph | |
| $(Pr)_2$-N | CO | Cl | CO | NH | 3,5-F-Ph | |
| $(Pr)_2$-N | CO | Cl | CO | - | 2-naphthyl | |
| $(Pr)_2$-N | CO | Cl | CO | - | 4-CN-Ph | |
| $(Pr)_2$-N | CO | Cl | CO | - | p-biphenyl | |
| Pr,Me-N | CO | Cl | CO | NH | 3,5-Cl-Ph | |
| $(Pr)_2$-N | CO | Cl | CO | NH | 3,5-Cl-Ph | |
| $(Pr)_2$-N | CO | Cl | CO | NH | 3,5-F-Ph | |
| $(Pr)_2$-N | CO | Cl | CO | - | 2-naphthyl | |
| $(Pr)_2$-N | CO | Cl | CO | - | 4-CN-Ph | |
| $(Pr)_2$-N | CS | Cl | CO | - | p-biphenyl | |
| Pr,Me-N | CO | Cl | CO | NH | 3,5-Cl-Ph | |
| Pr,Me-N | CO | Cl | CO | NH | 3,5-F-Ph | |
| Pr,Me-N | CO | Cl | CO | - | 2-naphthyl | |
| Pr,Me-N | CO | Cl | CO | - | 4-CN-Ph | |

| $X^1$ | B | Y | W | Z | $A^1$ |
|---|---|---|---|---|---|
| Pr,Me-N | CO | Cl | CO | - | p-biphenyl |
| -N-Me,$CH_2CH_2CH_2Cl$ | CO | Cl | CO | NH | 3,5-Cl-Ph |
| -N-Me,$CH_2CH_2CH_2Cl$ | CO | Cl | CO | NH | 3,5-F-Ph |
| -N-Me,$CH_2CH_2CH_2Cl$ | CO | Cl | CO | - | 2-naphthyl |
| -N-Me,$CH_2CH_2CH_2Cl$ | CO | Cl | CO | - | 4-CN-Ph |
| -N-Me,$CH_2CH_2CH_2Cl$ | CO | Cl | CO | - | p-biphenyl |
| azetidino | CO | Cl | CO | NH | 3,5-Cl-Ph |
| azetidino | CO | Cl | CO | NH | 3,5-F-Ph |
| azetidino | CO | Cl | CO | - | 2-naphthyl |
| azetidino | CO | Cl | CO | - | 4-CN-Ph |
| azetidino | CO | Cl | CO | - | p-biphenyl |
| 3,5-Me-morpholine | CO | Cl | CO | NH | 3,5-Cl-Ph |
| 3,5-Me-morpholine | CS | Cl | CO | NH | 3,5-Cl-Ph |
| 3,5-Me-morpholine | CO | Cl | CO | NH | 3,5-F-Ph |
| 3,5-Me-morpholine | CS | Cl | CO | NH | 3,5-F-Ph |
| 3,5-Me-morpholine | CO | Cl | CO | - | 2-naphthyl |
| 3,5-Me-morpholine | CO | Cl | CO | - | p-biphenyl |
| 3,5-Me-morpholine | CO | Cl | CO | - | 4-CN-Ph |
| 3,5-Me-morpholine | CS | Cl | CO | - | 2-naphthyl |
| 3,5-Me-morpholine | CS | Cl | CO | - | 4-CN-Ph |
| 3,5-Me-morpholine | CS | Cl | CO | - | p-biphenyl |

| $X^1$ | B | X | W | Z | $A^1$ | m.p.(°C) |
|---|---|---|---|---|---|---|
| piperidino | CS | Cl | CO | NH | 3,5-Cl-Ph | |
| piperidino | CS | Cl | CO | NH | 3,5-F-Ph | |
| pyrrolidino | CS | Cl | CO | NH | 3,5-Cl-Ph | |
| pyrrolidino | CS | Cl | CO | NH | 3,5-F-Ph | |
| pyrrolidino | CS | Cl | CO | - | 2-naphthyl | |
| pyrrolidino | CS | Cl | CO | - | 4-CN-Ph | |
| pyrrolidino | CS | Cl | CO | - | p-biphenyl | |
| $(CH_3)_2$-N | CS | Cl | CO | NH | 3,5-F-Ph | |
| $(CH_3)_2$-N | CS | Cl | CO | - | 4-CN-Ph | |
| $(CH_3)_2$-N | CS | Cl | CO | - | p-biphenyl | |
| $(CH_3)_2$-N | CS | Cl | CO | - | 2-naphthyl | |
| azetidino | CS | Cl | CO | NH | 3,5-Cl-Ph | |
| azetidino | CS | Cl | CO | NH | 3,5-F-Ph | |
| homopiperidine | CS | Cl | CO | NH | 3,5-F-Ph | |
| homopiperidine | CS | Cl | CO | NH | 3,5-Cl-Ph | |
| homopiperidine | CS | Cl | CO | - | 2-naphthyl | |
| piperidino | CS | Cl | CO | - | 2-naphthyl | 132-135 |
| piperidino | CS | Cl | CO | - | p-biphenyl | |

39

| $X^1$ | B | Y | W | Z | $A^1$ |
|---|---|---|---|---|---|
| 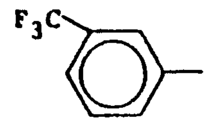 | CO | Cl | CO | NH | 3,5-Cl-Ph |
| 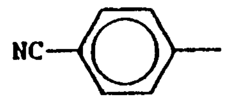 | CO | Cl | CO | NH | 3,5-Cl-Ph |
| $CH_3$ | CO | Cl | CO | NH | 3,5-F-Ph |
| $-C(CH_3)_3$ | CO | Cl | CO | NH | 3,5-F-Ph |
| 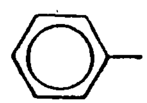 | CO | Cl | CO | NH | 3,5-F-Ph |
| 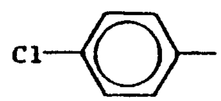 | CO | Cl | CO | - | 3,5-F-Ph |
| $CH_3$ | CO | Cl | CO | - | 2-naphthyl |
| $-CH_2CH_3$ | CO | Cl | CO | - | 2-naphthyl |
| $-C(CH_3)_3$ | CO | Cl | CO | - | 2-naphthyl |

| $X^1$ | B | Y | W | Z | $A^1$ |
|---|---|---|---|---|---|
| 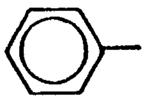 | CO | Cl | CO | - | 2-naphthyl |
| 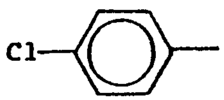 | CO | Cl | CO | - | 2-naphthyl |
| 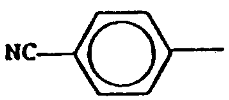 | CO | Cl | CO | - | 2-naphthyl |
| 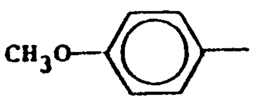 | CO | Cl | CO | - | 2-naphthyl |
| $CH_3$ | CO | Cl | CO | - | p-biphenyl |
| 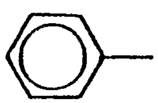 | CO | Cl | CO | - | p-biphenyl |

| $X^1$ | B | X | W | Z | $A^1$ |
|---|---|---|---|---|---|
| $-CH_3$ | CO | Cl | CO | NH | 3,5-Cl-Ph |
| $-CH_2-Cl$ | CO | Cl | CO | NH | 3,5-Cl-Ph |
| $-CF_3$ | CO | Cl | CO | NH | 3,5-Cl-Ph |
| $-CH_2CH_3$ | CO | Cl | CO | NH | 3,5-Cl-Ph |
| $-CH_2-CF_3$ | CO | Cl | CO | NH | 3,5-Cl-Ph |
| $-CH_2CH_2CH_3$ | CO | Cl | CO | NH | 3,5-Cl-Ph |
| $-CH_2CH_2CH_2CH_3$ | CO | Cl | CO | NH | 3,5-Cl-Ph |
| $-(CH_2)_4CH_3$ | CO | Cl | CO | NH | 3,5-Cl-Ph |
| $-(CH_2)_5CH_3$ | CO | Cl | CO | NH | 3,5-Cl-Ph |
| $-(CH_2)_6CH_3$ | CO | Cl | CO | NH | 3,5-Cl-Ph |
| $-(CH_2)_7CH_3$ | CO | Cl | CO | NH | 3,5-Cl-Ph |
| 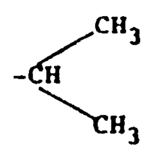 | CO | Cl | CO | NH | 3,5-Cl-Ph |
| 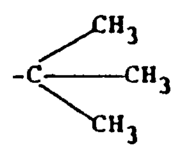 | CO | Cl | CO | NH | 3,5-Cl-Ph |

| $X^1$ | B | X | W | Z | $A^1$ |
|---|---|---|---|---|---|
| 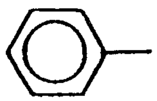 | CO | Cl | CO | NH | 3,5-Cl-Ph |
| 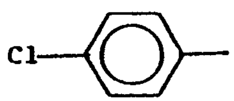 | CO | Cl | CO | NH | 3,5-Cl-Ph |
| 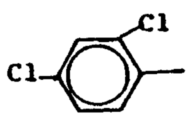 | CO | Cl | CO | NH | 3,5-Cl-Ph |
| 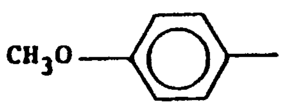 | CO | Cl | CO | NH | 3,5-Cl-Ph |
| $-CH_3$ | CS | Cl | CO | NH | 3,5-Cl-Ph |
| $-CF_3$ | CS | Cl | CO | NH | 3,5-Cl-Ph |

| $X^1$ | B | Y | W | Z | $A^1$ |
|---|---|---|---|---|---|
| 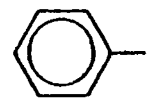 | CS | Cl | CO | NH | 3,5-Cl-Ph |
| Cl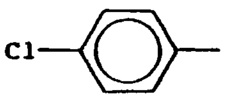 | CS | Cl | CO | NH | 3,5-Cl-Ph |
| $-CH_3$ | CS | Cl | CO | NH | 3,5-F-Ph |
| $-CF_3$ | CS | Cl | CO | NH | 3,5-F-Ph |
| 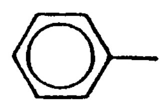 | CS | Cl | CO | NH | 3,5-F-Ph |
| $-CH_3$ | CS | Cl | CO | NH | 2-naphthyl |
| $-CF_3$ | CS | Cl | CO | NH | 2-naphthyl |

| $X^1$ | B | Y | W | Z | $A^1$ |
|---|---|---|---|---|---|
| 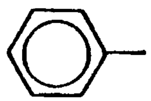 | CS | Cl | CO | NH | 2-naphthyl |
| Cl 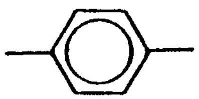 | CS | Cl | CO | NH | 2-naphthyl |
| $-CH_3$ | CS | Cl | CO | - | 3,5-Cl-Ph |
| $-CF_3$ | CS | Cl | CO | - | 3,5-Cl-Ph |
| 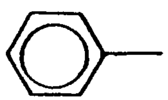 | CS | Cl | CO | - | 3,5-Cl-Ph |
| $CH_3$ | CS | Cl | CO | - | 4-CN-Ph |
| 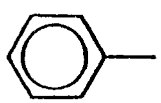 | CS | Cl | CO | - | 4-CN-Ph |

| $X^1$ | B | Y | W | Z | $A^1$ | $X^1$ | B | Y | W | Z | $A^1$ |
|---|---|---|---|---|---|---|---|---|---|---|---|
| $(Me)_2$-N | $SO_2$ | Cl | CO | - | 2-naphthyl | $CH_3$ | CS | Cl | CO | - | p-biphenyl |
| piperidino | $SO_2$ | Cl | CO | NH | 3,5-F-Ph | | | | | | |
| n-Bu,Me-N | $SO_2$ | Cl | CO | - | 2-naphthyl | | | | | | |
| morpholino | $SO_2$ | Cl | CO | NH | 3,5-Cl-Ph | 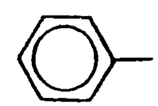 | CS | Cl | CO | - | p-biphenyl |
| 4-Cl-Ph-$CH_2$ | S | Cl | CO | NH | 3,5-F-Ph | | | | | | |
| 4-Cl-Ph | S | Cl | CO | - | 2-naphthyl | | | | | | |
| $-(CH_2)_3CH_3$ | S | Cl | CO | NH | 3,5-Cl-Ph | - | -CN | Cl | CO | NH | 3,5-F-Ph |
| $-(CH_2)_3CH_3$ | S | Cl | CO | NH | 3,5-F-Ph | - | -CN | Cl | CO | NH | 3,5-Cl-Ph |
| $-(CH_2)_3CH_3$ | S | Cl | CO | - | 2-naphthyl | - | -CN | Cl | CO | - | p-biphenyl |
| $-(CH_2)_3CH_3$ | S | Cl | CO | - | p-biphenyl | $-CH_3$ | $SO_2$ | Cl | CO | NH | 3,5-Cl-Ph |
| $-CH_3$ | SO | Cl | CO | - | 2-naphthyl | $-CF_3$ | $SO_2$ | Cl | CO | - | 2-naphthyl |
| $-CH_3$ | SO | Cl | CO | NH | 3,5-Cl-Ph | $-CH_2C{\equiv}C-CF_3$ | $SO_2$ | Cl | CO | NH | 3,5-F-Ph |
| $-(CH_2)_3CH_3$ | SO | Cl | CO | - | 2-naphthyl | $-(CH_2)_2CH_3$ | $SO_2$ | Cl | CO | - | p-biphenyl |
| $-(CH_2)_3CH_3$ | SO | Cl | CO | NH | 3,5-F-Ph | $-(CH_2)_2CH_3$ | $SO_2$ | Cl | CO | - | 2-naphthyl |
| $-CH_2CF_3$ | SO | Cl | CO | NH | 3,5-Cl-Ph | $-(CH_2)_2CH_3$ | $SO_2$ | Cl | CO | - | 4-CN-Ph |
| $-CH_2OCH_3$ | SO | Cl | CO | - | 3,5-$OCH_3$-Ph | $-(CH_2)_2CH_3$ | $SO_2$ | Cl | CO | NH | 3,5-F-Ph |
| $-CH_2OCH_3$ | SO | Cl | CO | $NHCH_2$ | 2-Cl-Ph | $-(CH_2)_2CH_3$ | $SO_2$ | Cl | CO | NH | 3,5-Cl-Ph |
| $-CH_2CH=CH_2$ | SO | Cl | CO | - | 2-naphthyl | 2,4-Cl-Ph | $SO_2$ | Cl | CO | - | 2-naphthyl |
| $-CH_2C{\equiv}CF_3$ | SO | Cl | CO | NH | 3,5-Cl-Ph | $(Me)_2$-N | $SO_2$ | Cl | CO | NH | 3,5-Cl-Ph |

| $X^1$ | B | Y | W | Z | $A^1$ |
|---|---|---|---|---|---|
| $-CH_2Ph$ | SO | Cl | CO | NH | 3,5-F-Ph |
| $-CH_2Ph$ | SO | Cl | CO | - | 2-naphthyl |
| 2,4-Cl-Ph-$CH_2$- | SO | Cl | CO | NH | 3,5-Cl-Ph |
| 2,4-Cl-Ph-$CH_2$- | SO | Cl | CO | NH | 2-naphthyl |
| 4-$CF_3$-Ph | SO | Cl | CO | NH | 3,5-Cl-Ph |
| $CH_3$ | CO | Cl | CO | NH | 3,5-Cl-Ph |
| $CH_3$ | CO | Cl | CO | NH | 3,5-F-Ph |
| $CH_3$ | CO | Cl | CO | - | 2-naphthyl |
| $CH_3$ | CO | Cl | CO | - | 4-CN-Ph |
| $CH_3$ | CO | Cl | CO | - | p-biphenyl |
| $-CH_2CH_2CH_2-Cl$ | CO | Cl | CO | NH | 3,5-Cl-Ph |
| $-CH_2CH_2CH_2-Cl$ | CO | Cl | CO | - | 2-naphthyl |
| $-(CH_2)_4CH_3$ | SO | Cl | CO | NH | 3,5-Cl-Ph |
| $-(CH_2)_4CH_3$ | SO | Cl | CO | - | 2-naphthyl |
| $-(CH_2)_5CH_3$ | SO | Cl | CO | NH | 3,5-Cl-Ph |
| $-(CH_2)_5CH_3$ | SO | Cl | CO | - | 2-naphthyl |
| $-(CH_2)_6CH_3$ | SO | Cl | CO | NH | 3,5-F-Ph |
| $-(CH_2)_6CH_3$ | SO | Cl | CO | - | 4-CN-Ph |
| $-(CH_2)_7CH_3$ | SO | Cl | CO | NH | 3,5-Cl-Ph |
| $-(CH_2)_7CH_3$ | SO | Cl | CO | - | 2-naphthyl |
| $-(CH_2)_8CH_3$ | SO | Cl | CO | NH | 3,5-F-Ph |

| $X^1$ | B | Y | W | Z | $A^1$ |
|---|---|---|---|---|---|
| $-(CH_2)_8CH_3$ | SO | Cl | CO | NH | 3,5-Cl-Ph |
| $-(CH_2)_8CH_3$ | SO | Cl | CO | - | 2-naphthyl |
| $-CH_2CF_3$ | CO | Cl | CO | NH | 3,5-Cl-Ph |
| $-CH_2CF_3$ | CO | Cl | CO | NH | 3,5-F-Ph |
| $-CH_2CF_3$ | CO | Cl | CO | - | 2-naphthyl |
| $-CH_2CF_3$ | CO | Cl | CO | - | 4-CN-Ph |
| $-CH_2CF_3$ | CO | Cl | CO | - | p-biphenyl |
| $-CH_2Ph$ | CO | Cl | CO | NH | 3,5-Cl-Ph |
| $-CH_2Ph$ | CO | Cl | CO | NH | 3,5-F-Ph |
| $-CH_2Ph$ | CO | Cl | CO | - | 4-CN-Ph |
| $-CH_2Ph$ | CO | Cl | CO | - | 2-naphthyl |
| $-CH_2Ph$ | CO | Cl | CO | - | p-biphenyl |
| 4-Cl-Ph-$CH_2$- | CO | Cl | CO | NH | 3,5-Cl-Ph |
| 4-Cl-Ph-$CH_2$- | CO | Cl | CO | NH | 3,5-F-Ph |
| 4-Cl-Ph-$CH_2$- | CO | Cl | CO | - | 4-Cl-Ph |
| 4-Cl-Ph-$CH_2$- | CO | Cl | CO | - | 4-CN-Ph |
| 4-Cl-Ph-$CH_2$- | CO | Cl | CO | - | p-biphenyl |
| 4-$CF_3$-Ph-$CH_2$- | CO | Cl | CO | NH | 3,5-Cl-Ph |
| 4-$CF_3$-Ph-$CH_2$- | CO | Cl | CO | - | 2-naphthyl |
| 4-$CF_3$-Ph-$CH_2$- | CO | Cl | CO | - | p-biphenyl |

Formulation

The compounds of this invention will generally be used in formulation with a liquid or solid diluent or with an organic solvent. Useful formulations of the compounds of Formula I can be prepared in conventional ways. They include dusts, granules, pellets, solutions, emulsions, wettable powders, emulsifiable concentrates and the like. Many of these may be applied directly. Sprayable formulations can be extended in suitable media and used at spray volumes of from about one to several hundred liters per hectare. High strength compositions are primarily used as intermediates for further formulation. The formulations, broadly, contain about 1% to 99% by weight of active ingredient(s) and at least one of a) about 0.1% to 35% surfactant(s) and b) about 5% to 99% solid or liquid inert diluent(s). More specifically, they will contain these ingredients in the following approximate proportions:

| | Percent by Weight | | |
|---|---|---|---|
| | Active Ingredient | Diluent(s) | Surfactant(s) |
| Wettable Powders and Water Dispersible Granules | 20-90 | 0-74 | 1-10 |
| Oil Suspensions, Emulsion, Solutions, (including Emulsifiable Concentrates) | 5-50 | 40-95 | 0-35 |
| Aqueous Suspensions | 10-50 | 40-84 | 1-20 |
| Dusts | 1-25 | 70-99 | 0-5 |
| Granules and Pellets | 1-95 | 5-99 | 0-15 |
| High Strength Compositions | 90-99 | 0-10 | 0-2 |

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey. The more absorptive diluents are preferred for the wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publ. Co., Inc., New York, 1964, list surfactants and recommended uses. All formulations can contain minor amounts of additives to reduce foam, caking, corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Water dispersible granules may be produced by agglomerating a fine powder composition (see, for example, B. Cross and H. Scher, "Pesticide Formulations", ACS Symposium Series 371, American Chemical Society, Washington, D. C., 1988, pp. 251-259). Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, Dec. 4, 1967, pp. 147ff. and "Perry's Chemical Engineer's Handbook", 4th Edn., McGraw-Hill, N.Y., 1963, pp. 8-59ff.

For further information regarding the art of formulation, see for example:

H. M. Loux, U.S. Patent 3,235,361, Feb. 15, 1966, Col. 6, Line 16 through Col. 7, Line 19 and Examples 10 through 41.

R. W. Luckenbaugh, U.S. Patent 3,309,192, March 14, 1967, Col. 5, Line 43 through Col. 7, Line 62 and Examples 8, 12, 15, 39, 41, 52, 53, 58, 132, 138-140, 162-164, 166, 167, 169-182.

H. Gysin and E. Knusli, U.S. Patent 2,891,855, June 23, 1959, Col. 3, Line 66 through Col. 5, Line 17 and Examples 1-4.

G. C. Klingman, "Weed Control as a Science", John Wiley and Sons, Inc., New York, 1961, pp. 81-96.

J. D. Fryer and S. A. Evans, "Weed Control Handbook", 5th Edn. Blackwell Scientific Publications, Oxford, 1968, pp. 101-103.

Examples of useful formulations of compounds of the present invention are as follows:

| EXAMPLE 371 | |
|---|---|
| Wettable Powder | |
| N-[[[3,5-(dichlorophenyl)amino]carbonyl]oxy]-2-(dimethylamino)-2-oxoethanimidoyl chloride | 80% |
| Sodium Alkylnapthalenesulfonate | 4% |
| Sodium Ligninsulfonate | 2% |
| Synthetic Amorphous Silica | 1% |
| Kaolinite | 13% |

The ingredients are blended, hammermilled, re-blended and packaged.

| EXAMPLE 372 | |
|---|---|
| High Strength Concentrate | |
| N-[[[3,5-(dichlorophenyl)amino]carbonyl]oxy]-2-(dimethylamino)-2-oxoethanimidoyl chloride | 98.5% |
| Silica Aerogel | 0.5% |
| Synthetic Amorphous Silica | 1.0% |

The ingredients are blended and ground in a hammermill to produce a high strength concentrate

essentially all passing a U.S.S. No. 50 Sieve (0.3 mm openings). This material may then be formulated in a variety of ways.

| EXAMPLE 373 | |
|---|---|
| Solution | |
| N-[[[3,5-(dichlorophenyl)amino]carbonyl]oxy]-2-(dimethylamino)-2-oxoethanimidoyl chloride<br>N-methyl-2-pyrrolidone | 30%<br>70% |

The ingredients are combined and stirred to produce a solution, which can be used for low volume applications.

Utility

The compounds of this invention are useful as plant disease control agents. They provide control of diseases caused by a broad spectrum of fungal plant pathogens in the Basidiomycete, Ascomycete and Oomycete classes. They are effective in controlling a broad spectrum of plant diseases, particularly foliar pathogens of ornamental, vegetable, field, cereal and fruit crops. These pathogens include, Plasmopora viticola, Phytophthora infestans, Peronospora tabacina, Pseudoperonospora cubensis, Pythium aphanidermatum, Alternaria brassicae, Septoria nodorum, Cercosporidium personatum, Cercospora arachidicola, Pseudocercosporella herpotrichoides, Cercospora beticola, Botrytis cinerea, Monilinia fructicola, Pyricularia oryzae, Podosphaera leucotricha, Venturia inaequalis, Puccinia recondita, Puccinia gramminis, Hemileia vastatrix, Puccinia strilformis, Puccinia arachidis, and other species closely related to these pathogens.

The compounds of this invention can be mixed with fungicides, bactericides, acaricides, nematicides, insecticides, or other biologically active compounds in order to achieve desired results with a minimum expenditure of time, effort and material. Suitable agents of this type are well-known to those skilled in the art. Some are listed below:

Fungicides
methyl 2-benzimidazolecarbamate (carbendazim)
tetramethylthiuram disulfide (thiuram)
n-dodecylguanidine acetate (dodine)
manganese ethylenebisdithiocarbamate (maneb)
1,4-dichloro-2,5-dimethoxybenzene (chloroneb)methyl 1-(butylcarbamoyl)-2-benzimidazolecarbamate (benomyl)
2-cyano-N-ethylcarbamoyl-2-methoxyiminoacetamide (cymoxanil)
N-trichloromethylthiotetrahydrophthalamide (captan)
N-trichloromethylthiophthalimide (folpet)
dimethyl 4,4′-(o-phenylene)bis(3-thioallophanate)(thiophanate-methyl)
2-(thiazol-4-yl)benzimidazole (thiabendazole)
aluminum tris(O-ethyl phosphonate)(phosethyl aluminum) tetrachloroisophthalonitrile (chlorothalonil)
2,6-dichloro-4-nitroaniline (dichloran)
N-(2,6-dimethylphenyl)-N-(methoxyacetyl)alanine methyl ester (metalaxyl)
cis-N-[1,1,2,2-tetrachloroethyl)thio]cyclohex-4-ene-1,2-dicarbioximide (captafol)
3-(3,5-dichlorophenyl)-N-(1-methylethyl)-2,4-dioxo-1-imidazolidine carboxamide (iprodione)
3-(3,5-dichlorophenyl)-5-ethenyl-5-methyl-2,4-oxazolidinedione (vinclozolin)
kasugamycin
O-ethyl-S,S-diphenylphosphorodithioate(edifenphos)
4-(3-(4-(1,1-dimethyl-ethyl)phenyl)-2-methyl)propyl-2,6-dimethylmorpholine (Fenpropimorph)
4-(3-4(1,1-dimethyl-ethyl)phenyl)-2-methyl)propylpiperidine (Fenpropidine)
Bayleton® 1-(4-chlorophenoxy)-3,3-dimethyl-1-1H-1,2,4-triazol-1-yl)butanone
Systhane® 2-(4-chlorophenyl)-2-(1H-1,2,4-triazol-1-ylmethyl)hexanenitrile
Folicur® (tebuconazol)
Score® 3-chloro-4-[4-methyl-2-(1H-1,2,4-triazol)-1-ylmethyl)-1,3-dioxolan-2-yl]phenyl-4-chlorophenyl ether
Topaz® 1-[2-(2,4-dichlorophenyl)pentyl]1H-1,2,4-triazole
Impact® (±α-(2-fluorophenyl-α-(4-fluorophenyl)-1H-1,2,4-triazole-1-ethano

Nustar® 1-[[bis(4-fluorophenyl)methylsilyl)methyl]-1H-1,2,4-triazole
Sportak® 1-N-propyl-N-[2(2,4,6-trichlorophenoxy)ethyl]carbamoylimidazole
Tilt® 1-[[2-(2,4-dichlorophenyl)-4-propyl-1,3-dioxolan-2-yl]methyl]-1H-1,2,4-triazole
Rubigan® α-(2-chlorophenyl)-α-(4-chlorophenyl)-5-pyridine-methanol
copper oxychloride
furalaxyl methyl N-(2,6-dimethylphenyl)-N-(2-furanylcarbonyl)-DL-alaninate
Bactericides
tribasic copper sulfate
streptomycin sulfate
oxytetracycline
Acaricides
senecioic acid, ester with 2-sec-butyl-4,6-dinitrophenol (binapacryl)
6-methyl-1,3-dithiolo[2,3-B]quinonolin-2-one (oxythioquinox)
2,2,2-trichloro-1,1-bis(4-chlorophenyl)ethanol(dicofol)
bis(pentachloro-2,4-cyclopentadien-1-yl)(dienochlor)
tricyclohexyltin hydroxide (cyhexatin)
hexakis(2-methyl-2-phenylpropyl)distannoxane (fenbutin oxide)
Nematicides
2-[diethoxyphosphinylimino]-1,3-diethietane (fosthietan)
S-methyl-1-(dimethylcarbamoyl)-N-(methylcarbamoyloxy)thioformimidate(oxamyl)
S-methyl-1-carbamoyl-N-(methylcarbamoyloxy)thioformimidate
N-isopropylphosphoramidic acid, O-ethyl-O′-[4-(methylthio)-m-tolyl]diester (fenamiphos)
Insecticides
3-hydroxy-N-methylcrotonamide(dimethylphosphate)ester (monocrotophos)
methylcarbamic acid, ester with 2,3-dihydro-2,2-dimethyl-7-benzofuranol (carbofuran)
O-[2,4,5-trichloro-a-(chloromethyl)benzyl]phosphoric acid, O′,O′-dimethyl ester (tetrachlorvinphos)
2-mercaptosuccinic acid, diethyl ester, S-ester with thionophosphoric acid, dimethyl ester (malathion)
phosphorothioic acid, O,O-dimethyl, O-p-nitrophenyl ester (methyl parathion)
methylcarbamic acid, ester with a-naphthol (carbaryl)
methyl N-[[(methylamino)carbonyl]oxy]ethanimidothioate (methomyl)
N′-(4-chloro-o-tolyl)-N,N-dimethylformamidine (chlordimeform)
O,O-diethyl-O-(2-isopropyl-4-methyl-6-pyrimidyl)phosphorothioate (diazinon)
octachlorocamphene (toxaphene)
O-ethyl O-p-nitrophenyl phenylphosphonothioate (EPN)
cyano(3-phenoxyphenyl)-methyl 4-chloro-a-(1-methylethyl)benzeneacetate (fenvalerate)
(3-phenoxyphenyl)methyl (+)-cis,trans-3-(2,2-dichloroethenyl)-2,2-dimethylcyclopropanecarboxylate (permethrin)
dimethyl N,N′-[thiobis(N-methylimmo)carbonyloxy]]bis[ethanimidothioate] (thiodicarb)
phosphorothiolothionic acid, O-ethyl-O-[4-(methylthio)phenyl]-S-n-propyl ester (sulprofos)
a-cyano-3-phenoxybenzyl 3-(2,2-dichlorovinyl)-2,2-dimethylcyclopropane carboxylate (cypermethrin)
cyano(3-phenoxyphenyl)methyl 4-(difluoromethoxy)-a-(methylethyl)benzeneacetate (flucythrinate)
O,O-diethyl-O-(3,5,6-trichloro-2-pyridyl)phosphorothioate (chlorpyrifos)
O,O-dimethyl-S-[(4-oxo-1,2,3-benzotriazin-3-(4H)-yl)methyl]phosphorodithioate (azinphos-methyl)
5,6-dimethyl-2-dimethylamino-4-pyrimidinyl dimethyl carbamate (pirimicarb)
S-(N-formyl-N-methylcarbamoylmethyl)-O,O-dimethyl phosphorodithioate (formothion)
S-2-(ethylthioethyl)-O,O-dimethyl phosphiorothioate (demeton-S-methyl)
a-cyano-3-phenoxybenzyl cis-3-(2,2-dibromovinyl)-2,2-dimethylcyclopropane carboxylate (deltamethrin)
cyano(3-phenoxyphenyl)methyl ester of N-(2-chloro-4-trifluoromethylphenyl)alanine (fluvalinate)

## Application

Disease control is ordinarily accomplished by applying an effective amount of the compound, pre-infection, to the portion of the plant to be protected such as the roots, stems, foliage, fruit, seeds, tubers or bulbs. The compound may also be applied to the seed, to protect the seed and seedling.

Rates of application for these compounds can be influenced by many factors of the environment and should be determined under actual use conditions. Foliage can normally be protected when treated at a rate of from less than 10 g/ha to 10,000 g/ha of active ingredient. Seed and seedlings can normally be protected

when seed is treated at a rate of from .1 to 10 g per kilogram of seed.

## Example A

The test compounds were dissolved in acetone in an amount equal to 3% of the final volume and then suspended at a concentration of 200 ppm in purified water containing 250 ppm of the surfactant Trem 014 (polyhydric alcohol esters). This suspension was sprayed to the point of run-off on apple seedlings. The following day the seedlings were inoculated with a spore suspension of Venturia inaequalis (the causal agent of apple scab) and incubated in a saturated atmosphere at 20° C for 24 hr, and then moved to a growth chamber at 22° C for 11 days, after which disease ratings were made.

## Example B

The test compounds were dissolved in acetone in an amount equal to 3% of the final volume and then suspended at a concentration of 200 ppm in purified water containing 250 ppm of the surfactant Trem 014 (polyhydric alcohol esters). This suspension was sprayed to the point of run-off on peanut seedlings. The following day the seedlings were inoculated with a spore suspension of Cercosporidium personatum (the causal agent of peanut late leafspot) and incubated in a saturated atmosphere at 22° C for 24 hr, a high humidity atmosphere at 22 to 30° C for 5 days, and then moved to a growth chamber at 29° C for 6 days, after which disease ratings were made.

## Example C

The test compounds were dissolved in acetone in an amount equal to 3% of the final volume and then suspended at a concentration of 200 ppm in purified water containing 250 ppm of the surfactant Trem 014 (polyhydric alcohol esters). This suspension was sprayed to the point of run-off on wheat seedlings. The following day the seedlings were inoculated with a spore suspension of Puccinia recondita (the causal agent of wheat leaf rust) and incubated in a saturated atmosphere at 20° C for 24 hr, and then moved to a growth chamber at 20° C for 6 days, after which disease ratings were made.

## Example D

The test compounds were dissolved in acetone in an amount equal to 3% of the final volume and then suspended at a concentration of 200 ppm in purified water containing 250 ppm of the surfactant Trem 014 (polyhydric alcohol esters). This suspension was sprayed to the point of run-off on rice seedlings. The following day the seedlings were inoculated with a spore suspension of Pyricularia oryzae (the causal agent of rice blast) and incubated in a saturated atmosphere at 27° C for 24 hr, and then moved to a growth chamber at 30° C for 5 days, after which disease ratings were made.

## Example E

The test compounds were dissolved in acetone in an amount equal to 3% of the final volume and then suspended at a concentration of 200 ppm in purified water containing 250 ppm of the surfactant Trem 014 (polyhydric alcohol esters). This suspension was sprayed to the point of run-off on tomato seedlings. The following day the seedlings were inoculated with a spore suspension of Phytophthora infestans (the causal agent of potato and tomato late blight) and incubated in a saturated atmosphere at 20° C for 24 hr, and then moved to a growth chamber at 20° C for 5 days, after which disease ratings were made.

## Example F

The test compounds were dissolved in acetone in an amount equal to 3% of the final volume and then suspended at a concentration of 200 ppm in purified water containing 250 ppm of the surfactant Trem 014 (polyhydric alcohol esters). This suspension was sprayed to the point of run-off on grape seedlings. The following day the seedlings were inoculated with a spore suspension of Plasmopara viticola (the causal agent of grape downy mildew) and incubated in a saturated atmosphere at 20° C for 24 hr, moved to a growth chamber at 20° C for 6 days, and then incubated in a saturated atmosphere at 20° C for 24 hr, after which disease ratings were made.

## Example G

The test compounds were dissolved in acetone in an amount equal to 3% of the final volume and then suspended at a concentration of 200 ppm in purified water containing 250 ppm of the surfactant Trem 014 (polyhydric alcohol esters). This suspension was sprayed to the point of run-off on cucumber seedlings. The following day the seedlings were inoculated with a spore suspension of Botrytis cinerea (the causal agent of gray mold on many crops) and incubated in a saturated atmosphere at 20° C for 48 hr, and moved to a growth chamber at 20° C for 5 days, after which disease ratings were made.

## Example H

The test compounds were dissolved in acetone in an amount equal to 3% of the final volume and then suspended at a concentration of 200 ppm in purified water containing 250 ppm of the surfactant Trem 014 (polyhydric alcohol esters). This suspension was sprayed to the point of run-off on sugar beet seedlings. The following day the seedlings were inoculated with a spore suspension of Cercospora beticola (the causal agent of sugar beet leafspot) and incubated in a a high humidity atmosphere at 22 to 30° C for 3 days, and then moved to a greenhouse at 20 to 25° C for 7 days, after which disease ratings were made.

## Example I

The test compounds were dissolved in acetone in an amount equal to 3% of the final volume and then suspended at a concentration of 1000 ppm in purified water containing 250 ppm of the surfactant Trem 014 (polyhydric alcohol esters). This suspension was sprayed to the point of run-off on tobacco seedlings. The following day the seedlings were inoculated with a spore suspension of Peronospora tabacina (the causal agent of tobacco blue mold) and incubated in a saturated atmosphere at 20° C for 24 hr, moved to a growth chamber at 22° C for 6 days, and then incubated in a saturated atmosphere at 20° C for 24 hr, after which disease ratings were made.

## Example J

The test compounds were dissolved in acetone in an amount equal to 3% of the final volume and then suspended at a concentration of 200 ppm in purified water containing 250 ppm of the surfactant Trem 014 (polyhydric alcohol esters). This suspension was sprayed to the point of run-off on cucumber seedlings. The following day the seedlings were inoculated with a spore suspension of Pseudoperonospora cubensis (the causal agent of cucumber downy mildew) and incubated in a saturated atmosphere at 20° C for 24 hr, moved to a growth chamber at 20° C for 6 days, and then incubated in a saturated atmosphere at 20° C for 24 hr, after which disease ratings were made.

Results for Examples A to J are given in Table A, B, and/or C. Data in Tables A and C are the average of three replicates. Data in Table B are from a single replicate. In the tables, a rating of 100 indicates 100%

disease control and a rating of 0 indicates no disease control relative to the carrier sprayed controls). NT indicates that no test was performed.

TABLE A

| CMPD NO. | EX A | EX B | EX C | EX D | EX E | EX F | EX G |
|---|---|---|---|---|---|---|---|
| 1 | 100 | 88 | 97 | 93 | 100 | 100 | 99 |
| 2 | 100 | NT | 97 | NT | 100 | 100 | NT |
| 3 | 100 | 88 | 90 | 93 | 100 | 100 | 99 |
| 4 | 100 | 92 | 96 | 94 | 100 | 100 | 99 |
| 5 | 100 | 49 | 50 | 80 | 100 | 100 | NT |
| 6 | 100 | 95 | 97 | 97 | 100 | 100 | NT |
| 7 | 100 | 100 | 82 | 85 | 100 | 100 | 99 |
| 8 | 100 | 100 | 100 | 95 | 100 | 100 | 99 |
| 9 | 100 | 75 | 85 | 93 | 100 | 100 | 14 |
| 10 | 100 | 98 | 97 | 91 | 100 | 100 | 99 |
| 11 | 100 | 100 | 97 | 97 | 100 | 100 | 99 |
| 12 | 100 | 98 | 97 | 89 | 100 | 100 | 90 |
| 13 | 100 | 90 | 99 | 73 | 100 | 100 | 99 |
| 14 | 100 | 82 | 27 | 26 | 100 | 91 | 5 |
| 15 | 100 | 98 | 97 | 94 | 100 | 100 | 97 |
| 16 | 100 | 95 | 99 | 95 | 100 | 100 | 67 |
| 17 | 100 | 40 | 84 | 0 | 73 | 100 | 3 |
| 28 | 100 | 98 | 97 | 79 | 100 | 100 | 99 |
| 19 | 100 | 92 | 96 | 40 | 100 | 100 | 66 |
| 20 | 100 | 97 | 100 | 93 | 100 | 100 | 99 |
| 21 | 95 | 96 | 100 | 97 | 100 | 100 | 99 |
| 22 | 95 | NT | 8 | NT | NT | 100 | NT |
| 26 | 29 | NT | 0 | NT | NT | 55 | NT |
| 27 | 100 | 99 | 84 | 92 | 100 | 100 | 99 |
| 28 | 0 | NT | NT | NT | NT | NT | NT |
| 30 | 96 | NT | 26 | NT | 81 | 100 | NT |
| 31 | 100 | NT | NT | NT | NT | NT | NT |

| CMPD NO. | EX A | EX B | EX C | EX D | EX E | EX F | EX G |
|---|---|---|---|---|---|---|---|
| 32 | 100 | 100 | 97 | 94 | 100 | 100 | 99 |
| 33 | 100 | 89 | 100 | 94 | 100 | 100 | 99 |
| 34 | 100 | 62 | 37 | 0 | 99 | 100 | 94 |
| 35 | 34 | 36 | 96 | 0 | 91 | 99 | 0 |
| 36 | 100 | 69 | 91 | 71 | 100 | 100 | 99 |
| 37 | 100 | 75 | 90 | 27 | 100 | 100 | 99 |
| 38 | 100 | 14 | 69 | 21 | 96 | 100 | 3 |
| 39 | 100 | 97 | 99 | 84 | 99 | 100 | 0 |
| 40 | 96 | 29 | 43 | 21 | 95 | 99 | 0 |
| 41 | 99 | 14 | 0 | 14 | 9 | 100 | 2 |
| 42 | 100 | 99 | 94 | 93 | 100 | 100 | 99 |
| 43 | 100 | NT | 0 | NT | 84 | 100 | NT |
| 44 | 99 | 10 | 43 | 0 | 0 | 86 | 34 |
| 45 | 99 | 0 | 0 | 13 | 43 | 96 | 1 |
| 46 | 100 | 91 | 86 | 91 | 100 | 100 | 90 |
| 47 | 100 | 21 | 56 | 71 | 92 | 100 | 3 |
| 48 | 100 | 69 | 77 | 96 | 100 | 100 | 1 |
| 49 | 99 | 58 | 52 | 34 | 97 | 98 | 0 |
| 50 | 100 | 89 | 91 | 74 | 99 | 100 | 3 |
| 51 | 100 | 96 | 43 | 41 | 100 | 100 | 4 |
| 52 | 100 | 94 | 100 | 60 | 100 | 100 | NT |
| 53 | 100 | NT | 26 | NT | NT | 100 | NT |
| 54 | 100 | NT | 95 | 77 | 100 | 100 | NT |
| 55 | 100 | 77 | 99 | 89 | 100 | 100 | 94 |
| 56 | 100 | 96 | 47 | 84 | 100 | 100 | NT |
| 57 | 100 | 96 | 69 | 67 | 100 | 100 | NT |
| 58 | 6 | NT | NT | 10 | 7 | 39 | NT |
| 59 | 100 | NT | 87 | 38 | 99 | 98 | 99 |
| 60 | 99 | NT | 88 | 41 | 96 | 100 | 94 |
| 61 | 100 | 91 | 88 | 97 | 98 | 100 | 0 |

| CMPD NO. | EX A | EX B | EX C | EX D | EX E | EX F | EX G |
|---|---|---|---|---|---|---|---|
| 62 | 100 | 87 | 88 | 97 | 91 | 100 | 29 |
| 63 | 100 | 79 | 17 | 76 | 98 | 100 | 15 |
| 64 | 100 | 26 | 17 | 13 | 85 | 74 | 15 |
| 65 | 100 | 9 | 0 | 13 | 0 | 2 | 0 |
| 66 | 100 | 13 | 0 | 28 | 84 | 65 | 1 |
| 67 | 100 | 99 | 85 | NT | 100 | 100 | 59 |
| 68 | 100 | 98 | 25 | 60 | 95 | 100 | 1 |
| 69 | 100 | 96 | 94 | 52 | 52 | 100 | 0 |
| 70 | 100 | NT | 80 | 60 | 99 | 100 | 4 |
| 71 | 100 | NT | 90 | 60 | 97 | 100 | 17 |
| 72 | 100 | NT | 97 | 53 | 99 | 100 | 31 |
| 73 | 100 | NT | 97 | 62 | 99 | 100 | 94 |
| 74 | 100 | NT | 92 | 86 | 97 | 100 | 1 |
| 75 | 100 | NT | 93 | 83 | 99 | 100 | 31 |
| 76 | 100 | NT | 94 | 88 | 99 | 98 | 49 |
| 77 | 100 | NT | 75 | 10 | 98 | 100 | 4 |
| 78 | 100 | 88 | 87 | 57 | 100 | 100 | 74 |
| 79 | 100 | 85 | 100 | 97 | 100 | 100 | 49 |
| 80 | 100 | 98 | 99 | 71 | 100 | 100 | 97 |
| 81 | 96 | 93 | 63 | 71 | 100 | 100 | 90 |
| 82 | 100 | 93 | 91 | 66 | 100 | 100 | 97 |
| 83 | 100 | 9 | 53 | 14 | 61 | 100 | 18 |
| 84 | 100 | 100 | 58 | NT | 100 | 99 | NT |
| 85 | 100 | 81 | 26 | 74 | 100 | 100 | 45 |
| 86 | 100 | 83 | 62 | 85 | 100 | 100 | 85 |
| 87 | 100 | 97 | 70 | 62 | 100 | 100 | 85 |
| 88 | 100 | 63 | 87 | 85 | 100 | 100 | 77 |
| 89 | 100 | 77 | 90 | 82 | 100 | 100 | NT |
| 90 | 100 | 97 | 70 | 85 | 100 | 100 | 32 |
| 91 | 100 | 31 | 66 | NT | 98 | 100 | NT |
| 92 | 100 | NT | 53 | 85 | 100 | 100 | 83 |

| CMPD NO. | EX A | EX B | EX C | EX D | EX E | EX F | EX G |
|---|---|---|---|---|---|---|---|
| 93 | 100 | NT | 0 | NT | 9 | 52 | NT |
| 94 | 100 | NT | 0 | NT | 31 | 42 | NT |
| 95 | 100 | 62 | 18 | NT | 99 | 95 | 0 |
| 96 | 100 | NT | 0 | NT | 9 | 70 | NT |
| 97 | 100 | 96 | 99 | 89 | 100 | 100 | 99 |
| 98 | 100 | 26 | 93 | 14 | 95 | 100 | 4 |
| 99 | 96 | 55 | 96 | 14 | 95 | 100 | 4 |
| 100 | 96 | 26 | 100 | 14 | 99 | 100 | 30 |
| 101 | 0 | 26 | 0 | 7 | 0 | 39 | 15 |
| 102 | 81 | 13 | 63 | 0 | 87 | 100 | 1 |
| 103 | 99 | 13 | 91 | 14 | 89 | 100 | 4 |
| 104 | 100 | 0 | 70 | 0 | 87 | 99 | 0 |
| 106 | 100 | 93 | 63 | 91 | 100 | 100 | 18 |
| 107 | 100 | 35 | 87 | 32 | 100 | 100 | 3 |
| 108 | 100 | 94 | 100 | 96 | 100 | 100 | 99 |
| 109 | 96 | 64 | 18 | 16 | 100 | 100 | 1 |
| 110 | 100 | 94 | 84 | 94 | 100 | 100 | 99 |
| 111 | 81 | 50 | 0 | 16 | 17 | 100 | 3 |
| 112 | 100 | 88 | 91 | 96 | 100 | 100 | 4 |
| 113 | 100 | 88 | 100 | 38 | 100 | 100 | 31 |
| 114 | 36 | 3 | 0 | 11 | 0 | 15 | 4 |
| 115 | 100 | NT | NT | NT | NT | NT | NT |
| 116 | 100 | NT | NT | NT | 46 | 37 | NT |
| 117 | 100 | NT | NT | NT | 9 | 22 | NT |
| 118 | 100 | NT | NT | NT | 0 | 100 | NT |
| 119 | 100 | NT | 16 | NT | 72 | 100 | NT |
| 120 | 100 | 85 | 89 | NT | 98 | 100 | 99 |
| 121 | 100 | 76 | 0 | 0 | 98 | 100 | 0 |
| 122 | 93 | 70 | 34 | 48 | 99 | 100 | 40 |
| 123 | 20 | 65 | 0 | 12 | 7 | 30 | 0 |

| CMPD NO. | EX A | EX B | EX C | EX D | EX E | EX F | EX G |
|---|---|---|---|---|---|---|---|
| 124 | 100 | 70 | 46 | 0 | 0 | 100 | 0 |
| 125 | 87 | 41 | 10 | 0 | 0 | 8 | 62 |
| 126 | 100 | 86 | 97 | 93 | 100 | 100 | NT |
| 127 | 100 | NT | 93 | 73 | 100 | 100 | NT |
| 128 | 100 | 58 | 62 | NT | 100 | 100 | NT |
| 129 | 100 | 72 | 98 | 95 | 100 | 100 | 4 |
| 130 | 100 | 42 | 91 | 54 | 100 | 100 | NT |
| 131 | 96 | 0 | 0 | 0 | 96 | 94 | 1 |
| 132 | 100 | 18 | 0 | 0 | 82 | 100 | 1 |
| 133 | 100 | 35 | 36 | 26 | 100 | 100 | 29 |
| 134 | 16 | 0 | 0 | 0 | 0 | 59 | 1 |
| 135 | 100 | 0 | 0 | 0 | 73 | 97 | 16 |
| 136 | 81 | 55 | 84 | NT | 97 | 100 | 21 |
| 137 | 100 | 61 | 82 | NT | 100 | 100 | 94 |
| 138 | 100 | 71 | 84 | NT | 99 | 100 | 99 |
| 139 | 100 | NT | 24 | NT | 55 | 98 | NT |
| 140 | 81 | NT | 79 | NT | 99 | 100 | 90 |
| 141 | 100 | 88 | 77 | NT | 98 | 98 | 95 |
| 142 | 100 | 74 | 87 | NT | 99 | 100 | NT |
| 143 | 99 | 90 | 82 | NT | 100 | 100 | 94 |
| 144 | 100 | 83 | 100 | 9 | 100 | 100 | 48 |
| 145 | 100 | 73 | 97 | 39 | 100 | 100 | 17 |
| 146 | 100 | NT | NT | NT | 29 | 86 | NT |
| 147 | 100 | NT | NT | NT | NT | NT | NT |
| 148 | 100 | NT | NT | NT | 96 | 100 | NT |
| 149 | 100 | NT | NT | NT | 85 | 86 | NT |
| 150 | 100 | 74 | 100 | NT | 100 | 100 | 45 |
| 151 | 100 | 82 | 49 | 0 | 94 | 91 | 3 |
| 152 | 100 | 71 | 75 | 0 | 100 | 100 | 31 |
| 153 | 100 | 34 | 96 | 17 | 100 | 100 | 34 |
| 154 | 100 | 0 | 0 | 0 | 39 | 100 | 3 |

| CMPD NO. | EX A | EX B | EX C | EX D | EX E | EX F | EX G |
|---|---|---|---|---|---|---|---|
| 155 | 100 | 0 | 0 | 0 | 0 | 30 | 4 |
| 156 | 100 | 0 | 0 | 0 | 8 | 99 | 3 |
| 157 | 100 | 12 | 0 | 0 | 0 | 70 | 3 |
| 158 | 87 | 0 | 0 | 0 | 0 | 66 | 3 |
| 159 | 53 | 0 | 0 | 0 | 0 | 91 | 1 |
| 160 | 41 | 0 | 0 | 0 | 0 | 14 | 0 |
| 161 | 87 | 0 | 0 | 0 | 0 | 62 | 3 |
| 162 | 100 | 27 | 96 | 90 | 98 | 100 | 4 |
| 163 | 100 | 0 | 87 | 27 | 94 | 100 | 0 |
| 164 | 100 | 84 | 87 | 90 | 99 | 100 | 1 |
| 165 | 100 | 96 | 89 | 87 | 100 | 100 | NT |
| 166 | 100 | 78 | 100 | 84 | 100 | 100 | NT |
| 167 | 100 | 0 | 93 | 85 | 68 | 100 | 1 |
| 168 | 100 | NT | 80 | 9 | 96 | 100 | NT |
| 169 | 100 | 0 | 32 | 0 | 24 | 90 | 0 |
| 170 | 92 | 64 | 85 | 85 | 100 | 100 | 0 |
| 171 | 82 | 0 | 16 | 0 | 0 | 4 | 0 |
| 172 | 100 | NT | NT | NT | NT | NT | NT |
| 175 | 84 | NT | 18 | 4 | NT | NT | NT |
| 176 | 100 | NT | NT | NT | NT | NT | NT |
| 177 | 100 | NT | NT | NT | NT | NT | NT |
| 178 | 100 | 36 | NT | NT | 100 | 100 | NT |
| 179 | 100 | NT | 78 | 0 | 99 | 100 | NT |
| 180 | 100 | 82 | 86 | 8 | 99 | 100 | 18 |
| 181 | 100 | 25 | 18 | NT | 99 | 100 | 20 |
| 182 | NT | NT | NT | NT | NT | NT | NT |
| 183 | 100 | 66 | 89 | NT | 100 | 100 | NT |
| 184 | 100 | 66 | 70 | 51 | 100 | 100 | NT |
| 185 | 100 | 93 | 90 | 8 | 100 | 100 | 7 |
| 186 | 184 | 39 | 0 | NT | 84 | 100 | NT |

| CMPD NO. | EX A | EX B | EX C | EX D | EX E | EX F | EX G |
|---|---|---|---|---|---|---|---|
| 188 | 91 | NT | 36 | 0 | 92 | 100 | NT |
| 190 | 100 | NT | 36 | NT | 100 | 100 | NT |
| 191 | NT | 97 | 96 | 6 | 98 | 100 | NT |
| 192 | 100 | 99 | 93 | 8 | 100 | 100 | 6 |
| 193 | 100 | 95 | 97 | 15 | 100 | 100 | 93 |
| 194 | 100 | 9 | 94 | 8 | 100 | 100 | 6 |
| 195 | NT | NT | NT | NT | 27 | 0 | NT |
| 197 | 63 | NT | NT | NT | NT | NT | NT |
| 198 | 72 | NT | NT | NT | NT | NT | NT |
| 199 | 100 | 17 | 22 | NT | 99 | 97 | NT |
| 200 | 25 | NT | NT | NT | NT | NT | NT |
| 203 | 8 | NT | 24 | 0 | 8 | 42 | NT |
| 204 | 16 | NT | NT | NT | NT | NT | NT |
| 205 | 100 | 35 | 90 | 0 | 100 | 100 | NT |
| 207 | 100 | 38 | 14 | NT | 44 | 100 | NT |
| 208 | NT | NT | NT | 0 | NT | NT | 0 |
| 209 | 99 | NT | 55 | 19 | 97 | 100 | 0 |
| 210 | 91 | NT | 45 | 10 | 98 | 100 | 27 |
| 211 | 66 | NT | 37 | 0 | 88 | 100 | 0 |
| 212 | 99 | NT | 0 | NT | 17 | 99 | NT |
| 213 | NT | 100 | 100 | 99 | 100 | 100 | NT |
| 214 | 93 | 42 | 69 | 0 | 36 | 96 | NT |
| 215 | 87 | 20 | 88 | 0 | 88 | 100 | NT |
| 216 | 100 | 70 | 99 | 67 | 100 | 100 | NT |
| 217 | 67 | 0 | 17 | NT | 98 | 97 | NT |
| 218 | 69 | NT | 17 | NT | 42 | 97 | NT |
| 219 | 100 | NT | 90 | NT | 99 | 100 | NT |
| 220 | 100 | NT | 93 | NT | 99 | 100 | NT |
| 221 | 100 | 61 | 100 | 85 | 100 | 100 | 4 |
| 222 | 100 | 97 | 97 | 26 | 100 | 100 | NT |
| 223 | 100 | 61 | 99 | NT | 100 | 100 | 99 |

| CMPD NO. | EX A | EX B | EX C | EX D | EX E | EX F | EX G |
|---|---|---|---|---|---|---|---|
| 224 | 100 | 98 | 97 | 26 | 100 | 100 | 4 |
| 225 | 93 | NT | 69 | NT | 94 | 100 | NT |
| 226 | 100 | 14 | 61 | NT | 100 | 100 | NT |
| 227 | 100 | 14 | 35 | NT | 100 | 100 | NT |
| 228 | 75 | NT | 88 | NT | 98 | 99 | NT |
| 229 | 100 | 56 | 88 | NT | 100 | 100 | NT |
| 230 | 67 | 0 | 17 | NT | 100 | 100 | NT |
| 231 | 0 | NT | 35 | NT | 84 | 85 | NT |
| 232 | 100 | 14 | 84 | NT | 100 | 100 | NT |
| 233 | 86 | 0 | 17 | NT | 98 | 100 | NT |
| 235 | 100 | 51 | 92 | 21 | 97 | 100 | NT |
| 237 | 99 | 60 | 67 | NT | 31 | 54 | NT |
| 238 | NT | NT | NT | NT | 31 | 35 | NT |
| 239 | NT | 60 | 67 | NT | NT | NT | NT |
| 240 | NT | 14 | 51 | NT | 97 | NT | NT |
| 241 | NT | 14 | 75 | NT | NT | NT | 61 |
| 242 | 100 | NT | 35 | NT | 100 | 100 | NT |
| 243 | 100 | NT | 75 | NT | 100 | 100 | NT |
| 244 | 93 | 0 | 12 | NT | NT | NT | NT |
| 245 | 9 | NT | NT | 0 | NT | NT | NT |

TABLE B

| CMPD NO. | EX A | EX B | EX D | EX E | EX G |
|---|---|---|---|---|---|
| 23 | 33 | 0 | 0 | 0 | 6 |
| 29 | 47 | 14 | 0 | 0 | 0 |
| 105 | 69 | 0 | 0 | 0 | 7 |
| 173 | 69 | 24 | 0 | 0 | 0 |
| 174 | 42 | 0 | 0 | 0 | 0 |
| 187 | 0 | 0 | 0 | 0 | 0 |
| 189 | 4 | 0 | 25 | 0 | 0 |
| 196 | NT | 0 | 0 | 0 | 0 |
| 201 | 12 | 0 | 0 | 0 | 0 |
| 202 | 0 | 27 | 0 | 0 | 0 |
| 206 | 0 | 1 | 0 | 0 | 9 |
| 234 | 100 | 95 | 85 | 99 | 0 |
| 236 | 67 | 34 | 27 | 76 | 11 |
| 246 | 100 | 91 | 67 | 99 | 0 |
| 247 | 100 | 96 | 97 | 99 | 4 |
| 248 | 100 | 96 | 85 | 92 | 0 |
| 249 | 100 | 0 | 27 | 97 | 0 |
| 251 | 100 | 0 | 0 | 97 | 0 |
| 252 | 100 | 0 | 27 | 44 | 0 |
| 253 | 90 | 0 | 27 | 0 | 0 |
| 254 | 100 | 0 | 27 | NT | 0 |
| 255 | 90 | 34 | 0 | 22 | 0 |
| 256 | 100 | 96 | 97 | 99 | 90 |
| 257 | 100 | 82 | 97 | 99 | 11 |
| 258 | 100 | 64 | 0 | 92 | 0 |
| 259 | 100 | 64 | 86 | 99 | 94 |
| 260 | 96 | 0 | 0 | 63 | 0 |
| 261 | 100 | 91 | 99 | 99 | 99 |
| 262 | 100 | 0 | 0 | 25 | 0 |

| CMPD NO. | EX A | EX B | EX D | EX E | EX G |
|---|---|---|---|---|---|
| 263 | 100 | 81 | 99 | 99 | 89 |
| 264 | 100 | 64 | 99 | 99 | 89 |
| 288 | 100 | 29 | 0 | 99 | 0 |
| 289 | 96 | 29 | 0 | 96 | 0 |
| 291 | 96 | 62 | 0 | 96 | 8 |
| 292 | 100 | 96 | 0 | 99 | 82 |
| 293 | 96 | 62 | 27 | 61 | 8 |
| 296 | 0 | 29 | 0 | 19 | 8 |
| 297 | 0 | 29 | 0 | 42 | 0 |
| 298 | 100 | 62 | 0 | 92 | 0 |
| 299 | 100 | 62 | 0 | 75 | 0 |
| 300 | 100 | 91 | 85 | 99 | 98 |
| 301 | 100 | 62 | 67 | 99 | 69 |
| 302 | 99 | 29 | 0 | 61 | 8 |
| 303 | 90 | 62 | 0 | 85 | 0 |
| 304 | 68 | 0 | 0 | 19 | 8 |

TABLE C

| CMPD NO. | EX H | EX I | EX J |
|---|---|---|---|
| 1 | 100 | 100 | 100 |
| 2 | 63 | 100 | 94 |
| 3 | 88 | 98 | 96 |
| 4 | 26 | 70 | 10 |
| 5 | 32 | 96 | 64 |
| 6 | 95 | 96 | 100 |
| 7 | 100 | 96 | 100 |
| 8 | 94 | 96 | 100 |
| 9 | 51 | 100 | 61 |
| 10 | 94 | 98 | 100 |
| 11 | 75 | 96 | 100 |
| 12 | 27 | 100 | 32 |
| 42 | 100 | 80 | 100 |

## Claims

1. A method of controlling plant disease, especially fungus, comprising the application of an effective amount of a compound of Formula I tothe locus to be protected,

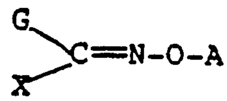

I

wherein:
A is C(=O)R, C(=O)OR$^1$, C(=O)SR$^1$, P(=O)QR$^2$Q$^1$R$^3$; C(=O)NHR, $SO_2R^5$, $SO_2NR^6R^7$;
Q and Q$^1$ are independently oxygen, NR$^8$ or a direct bond;
X is Cl or Br; provided that when X is Br, A is C(=O)R;
G is C(=L)R$^9$, C(=L)NR$^{10}$R$^{11}$, C(=O)OR$^{12}$, CN, $SO_2NR^{10}R^{11}$, or $SO_mR^{13}$;
L is O or S;
m is 0, 1 or 2;
R is $C_1$-$C_{20}$ alkyl, $C_2$-$C_{20}$ alkenyl, $C_2$-$C_{20}$ alkynyl; $C_1$-$C_8$ alkyl, $C_2$-$C_8$ alkenyl, $C_2$-$C_8$ alkynyl, or $C_3$-$C_7$ cycloalkyl, each optionally substituted with halogen, $C_1$-$C_6$ alkoxy, $C_2$-$C_6$ alkoxyalkyl, $C_1$-$C_6$ alkylthio, $C_3$-$C_6$ cycloalkyl, CN, or with a phenyl group substituted with 0-3 halogen and 0-2 groups selected from $CH_3$, $CF_3$, $CH_3O$, CN, $CH_3S$, methylsulfonyl, dimethylamino, amino, phenyl or phenoxy, provided that when A is C(=O)NHR, R is not $C_2$ alkenyl or $C_2$ alkynyl;
R is a phenyl ring or a $C_{10}$-$C_{14}$ fused carbocyclic aromatic ring systems, wherein said rings are substituted with 0-4 halogen, 0-1 groups selected from $-CH_2(CH_2)_pCH_2-$, $-O(CH_2)_pCH_2-$, $-S(CH_2)_pCH_2-$, $-O(CH_2)_pO$, $-O-(CH_2)_pS-$, $-R^4N(CH_2)_pCH_2-$, $-O(CH_2)_pNR^4-$, and 0-2 groups selected from NH(C=O)OR$^{16}$, SCN, $C_1$-$C_4$ alkyl, $C_1$-$C_4$ haloalkyl, $C_2$-$C_4$ alkoxyalkyl, $C_2$-$C_4$ alkenyl, $C_2$-$C_4$ haloalkenyl, $C_2$-$C_4$ alkynyl, $C_3$-$C_6$ cycloalkoxy, $C_3$-$C_6$ cycloalkyl, $NO_2$, C(=O)R$^{14}$, CN, OR$^{14}$, C(=O)OR$^{14}$, C(=O)NR$^{14}$R$^{15}$, NR$^{14}$R$^{15}$, SR$^{14}$, SOR$^{14}$, $SO_2F$, $SO_2Cl$ or $SO_2NR^{14}R^{15}$, 2-, 3-, or 4-pyridyl, or phenyl substituted with 0-3 halogen and 0-2 groups selected from $CH_3$, $CF_3$, $CH_3O$, CN, $CH_3S$, methylsulfonyl, dimethylamino or amino;
R can additionally be a heterocyclic ring system of 3-8 atoms, ring-carbon-linked to the carbonyl group when A is C(=O)R or to the N when A is C(=O)NHR, containing 1 or 2 nitrogen atoms, or 1 nitrogen atom and 1 oxygen atom, or 1 nitrogen atom and 1 sulfur atom; or one or, if the ring size is greater than 3, 2 oxygen atoms, provided that the oxygen atoms are not bonded to each other; or 1 or 2 sulfur atoms;
R can additionally be a heteroaromatic or fused heteroaromatic ring system, ring-carbon- linked to the carbonyl group when A is C(=O)R or to the N when A is C(=O)NHR, containing 5-10 atoms, wherein the heteroatoms comprise 1-3 nitrogen atoms, or 1-2 nitrogen atoms and one oxygen or sulfur atom, or 1-2 oxygen or sulfur atoms, these rings being substituted with 0-1 ($-CH_2(CH_2)_pCH_2-$) or with 0-2 groups selected from $CH_3$, $OCH_3$, $OCF_3$, $OCH_2CF_3$, F, Cl, Br, $OCH_2CH_3$, $NO_2$, C(=O)$CH_3$, $N(CH_3)_2$, $CO_2CH_3$, $CON(CH_3)_2$, $SO_2N(CH_3)_2$, $SCH_3$, CN or $CF_3$;
R$^1$ is $C_1$-$C_{20}$ alkyl, $C_3$-$C_{20}$ alkenyl, $C_3$-$C_{20}$ alkynyl; or $C_1$-$C_8$ alkyl, $C_3$-$C_8$ alkenyl or $C_3$-$C_8$ alkynyl, each optionally substituted with halogen, $C_1$-$C_6$ alkoxy, $C_2$-$C_6$ alkoxyalkyl, $C_1$-$C_6$ alkylthio, $C_3$-$C_6$ cycloalkyl, CN or with a phenyl group substituted with 0-3 halogen and 0-2 groups selected from $CH_3$, $CF_3$, $CH_3O$, CN, $CH_3S$, methylsulfonyl, dimethylamino, amino, phenyl or phenoxy; provided that, if R$^1$ is alkenyl or alkynyl, the unsaturated carbons are not bonded directly to the oxygen atom of

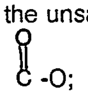

R$^2$ and R$^3$ are independently selected from $C_1$-$C_4$ alkyl, $C_1$-$C_4$ haloalkyl, benzyl or phenyl;
R$^4$ is H or $C_1$-$C_4$ alkyl;
R$^5$ is $C_1$-$C_4$ alkyl substituted with 0-3 halogen, or R$^5$ is a phenyl group substituted with 0-3 halogen and 0-2 groups selected from $CH_3$, $CF_3$, $CH_3O$, CN, $CH_3S$, methylsulfonyl, dimethylamino, amino, nitro, phenyl or phenoxy;
R$^6$ is H, $C_1$-$C_4$ alkyl substituted with 0-3 halogen, or R6 is a phenyl group substituted with 0-3 halogen and 0-2 groups selected from $CH_3$, $CF_3$, $CH_3O$, CN, $CH_3S$, methylsulfonyl, dimethylamino, amino, phenyl or phenoxy;
R$^7$ is H or $C_1$-$C_4$ alkyl substituted with 0-3 halogen, or R$^6$ and R$^7$ taken together with the nitrogen atom to which they are attached can be piperidine, pyrrolidine or morpholine, each substituted with 0-2 methyl groups;
R$^8$ is H or $C_1$-$C_4$ alkyl;
R$^9$ is $C_1$-$C_8$ alkyl substituted with 0-3 halogen, or R$^9$ is phenyl substituted with 0-2 halogen, $CH_3$, $CF_3$, $CH_3O$ or CN;
R$^{10}$ and R$^{11}$ are independently H, $C_1$-$C_4$ alkyl, $C_1$-$C_4$ haloalkyl, $C_2$-$C_4$ alkoxyalkyl, or benzyl or phenyl, said

benzyl or phenyl rings being substituted with 0-2 halogen $CH_3$, $CF_3$, $CH_3O$ or CN; or $R^{10}$ and $R^{11}$, taken together with the nitrogen atom to which they are attached, can be azetidine, piperidine, homopippiridine, pyrrolidine, or morpholine, each substituted with 0-2 methyl groups;

$R^{12}$ is $C_1$-$C_{12}$ alkyl or haloalkyl, or benzyl substituted with 0-2 halogen, $CH_3$, $CF_3$, $CH_3O$ or CN;

$R^{13}$ is $C_1$-$C_4$ alkyl, haloalkyl or $C_2$-$C_4$ alkoxyalkyl, $C_3$-$C_4$ alkenyl haloalkenyl alkynyl or haloalkynyl, or benzyl or phenyl said benzyl or phenyl rings being substituted with 0-2 halogen, $CH_3$, $CF_3$, $CH_3O$ or CN;

$R^{14}$ is H, $C_1$-$C_4$ alkyl, $C_1$-$C_4$ haloalkyl, or $C_2$-$C_4$ alkoxyalkyl; $C_3$-$C_4$ alkenyl, haloalkenyl, alkynyl or haloalkynyl; or benzyl or phenyl, said benzyl or phenyl rings being substituted with 0-2 halogen, $CH_3$, $CF_3$, $CH_3O$, $CH_3S$ or CN;

$R^{15}$ is H or $C_1$-$C_4$ alkyl;

$R^{16}$ is $C_1$-$C_6$ alkyl, $C_3$-$C_4$ alkenyl, or benzyl substituted with 0-2 halogen, methyl, trifluoromethyl, nitro or methoxy; and

p is 1 or 2.

2. The method of Claim 1 wherein:

A is C(=O)R, C(=O)$OR^1$,C(=O)NHR, or P(=O)$QR^2Q^1R^3$;

G is C(=O)$NR^{10}R^{11}$ or C(=O)$OR^{12}$.

3. The method of Claim 1 wherein:

A is C(=O)R, or C(=O)NHR;

G is C(=O)$NR^{10}R^{11}$ or C(=O)$OR^{12}$;

X is Cl.

4. The method of Claim 1 wherein:

A is C(=O)R, or C(=O)NHR;

G is C(=O)$NR^{10}R^{11}$ or C(=O)$OR^{12}$;

R is $C_1$-$C_2$ alkyl substituted with a phenyl group, said phenyl group being substituted with 0-3 halogen and 0-2 groups selected from $CH_3$, $CF_3$, $CH_3O$, CN, $CH_3S$, methylsulfonyl, dimethylamino, amino, phenyl or phenoxy; or

R is a phenyl or naphthyl ring, substituted with 0-3 halogen and 0-2 groups selected from $CH_3$, $CF_3$, $CH_3O$, CN, $CH_3S$, methylsulfonyl, dimethylamino, amino, phenyl or phenoxy;

$R^{10}$ and $R^{11}$ are independently H, $C_1$-$C_4$ alkyl, haloalkyl or benzyl substituted with 0-2 halogen, $CH_3$, $CF_3$, $CH_3O$ or CN; or $R^{10}$ and $R^{11}$, taken together with the nitrogen atom to which they are attached, can be piperidine, pyrrolidine or morpholine, each substituted with 0-2 methyl groups; and X is Cl.

5. The method of Claim 4 wherein:

A is C(=O)R;

G is C(=O)$NR^{10}R^{11}$;

R is $C_1$-$C_2$ alkyl substituted with a phenyl group, said phenyl group being substituted with 0-3 halogen and 0-2 groups selected from $CH_3$, $CF_3$, $CH_3O$, CN, $CH_3S$, methylsulfonyl, dimethylamino, amino, phenyl or phenoxy; or

R is a substituted phenyl or naphthyl ring, wherein the substituent is selected from: 0-3 halogen and 0-2 $CH_3$, $CF_3$, $CH_3O$, CN, $CH_3S$, methylsulfonyl, dimethylamino, amino, phenyl or phenoxy; and

X is Cl.

6. The method of Claim 4 wherein:

A is C(=O)NHR;

G is C(=O)$NR^{10}R^{11}$;

R is $C_1$-$C_2$ alkyl substituted with a phenyl group, said phenyl group being substituted with 0-3 halogen and 0-2 groups selected from $CH_3$, $CF_3$, $CH_3O$, CN, $CH_3S$, methylsulfonyl, dimethylamino, amino, phenyl or phenoxy; or

R is a substituted phenyl or naphthyl ring, wherein the substituent is selected from: 0-3 halogen and 0-2 $CH_3$, $CF_3$, $CH_3O$, CN, $CH_3S$, methylsulfonyl, dimethylamino, amino, phenyl or phenoxy; and

X is Cl.

7. A compound of Formula I,

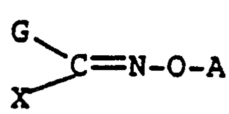

wherein

A is C(=O)R, C(=O)$OR^1$, C(=O)$SR^1$, P(=O)$QR^2Q^1R^3$; C(=O)NHR, $SO_2R^5$, $SO_2NR^6R^7$;

Q and $Q^1$ are independently oxygen, $NR^8$ or a direct bond;
X is Cl or Br; provided that when X is Br, A is C(=O)R;
G is $C(=L)R^9$, $C(=L)NR^{10}R^{11}$, $C(=O)OR^{12}$, CN, $SO_2NR^{10}R^{11}$, or $SO_mR^{13}$;
L is O or S;
m is 0, 1 or 2;
R is $C_1$-$C_{20}$ alkyl, $C_2$-$C_{20}$ alkenyl, $C_2$-$C_{20}$ alkynyl; $C_1$-$C_8$ alkyl, $C_2$-$C_8$ alkenyl, $C_2$-$C_8$ alkynyl, or $C_3$-$C_7$ cycloalkyl, each optionally substituted with halogen, $C_1$-$C_6$ alkoxy, $C_2$-$C_6$ alkoxyalkyl, $C_1$-$C_6$ alkylthio, $C_3$-$C_6$ cycloalkyl, CN, or with a phenyl group substituted with 0-3 halogen and 0-2 groups selected from $CH_3$, $CF_3$, $CH_3O$, CN, $CH_3S$, methylsulfonyl, dimethylamino, amino, phenyl or phenoxy, provided that when A is C(=O)NHR, R is not $C_2$ alkenyl or $C_2$ alkynyl;
R is a phenyl ring or a $C_{10}$-$C_{14}$ fused carbocyclic aromatic ring systems, wherein said rings are substituted with 0-4 halogen, and 0-1 groups selected from $-CH_2(CH_2)_pCH_2-$, $-O(CH_2)_pCH_2-$, $-S(CH_2)_pCH_2-$, $-O(CH_2)_pO$, $-O(CH_2)_pS-$, $-R^4N(CH_2)_pCH_2-$, $-O(CH_2)_pNR^4-$, and 0-2 groups selected from $NH(C=O)OR^{16}$, SCN, $C_1$-$C_4$ alkyl, $C_1$-$C_4$ haloalkyl, $C_2$-$C_4$ alkoxyalkyl, $C_2$-$C_4$ alkenyl, $C_2$-$C_4$ haloalkenyl, $C_2$-$C_4$ alkynyl, $C_3$-$C_6$ cycloalkoxy, $C_3$-$C_6$ cycloalkyl, $NO_2$, $C(=O)R^{14}$, CN, $OR^{14}$, $C(=O)OR^{14}$, $C(=O)NR^{14}R^{15}$, $NR^{14}R^{15}$, $SR^{14}$, $SOR^{14}$, $SO_2F$, $SO_2Cl$ or $SO_2NR^{14}R^{15}$, 2-, 3-, or 4-pyridyl, or phenyl substituted with 0-3 halogen and 0-2 groups selected from $CH_3$, $CF_3$, $CH_3O$, CN, $CH_3S$, methylsulfonyl, dimethylamino or amino;
R can additionally be a heterocyclic ring system of 3-8 atoms, ring-carbon-linked to the carbonyl group when A is C(=O)R or to the N when A is C(=O)NHR, containing 1 or 2 nitrogen atoms, or 1 nitrogen atom and 1 oxygen atom, or 1 nitrogen atom and 1 sulfur atom; or one or, if the ring size is greater than 3, 2 oxygen atoms, provided that the oxygen atoms are not bonded to each other; or 1 or 2 sulfur atoms;
R can additionally be a heteroaromatic or fused heteroaromatic ring system, ring-carbon-linked to the carbonyl group when A is C(=O)R or to the N when A is C(=O)NHR, containing 5-10 atoms, wherein the heteroatoms comprise 1-3 nitrogen atoms, or 1-2 nitrogen atoms and one oxygen or sulfur atom, or 1-2 oxygen or sulfur atoms, these rings being substituted with 0-1 $(-CH_2(CH_2)_pCH_2-)$ or with 0-2 groups selected from $CH_3$, $OCH_3$, $OCF_3$, $OCH_2CF_3$, F, Cl, Br, $OCH_2CH_3$, $NO_2$, $C(=O)CH_3$, $N(CH_3)_2$, $CO_2CH_3$, $CON(CH_3)_2$, $SO_2N(CH_3)_2$, $SCH_3$, CN or $CF_3$;
$R^1$ is $C_1$-$C_{20}$ alkyl, $C_3$-$C_{20}$ alkenyl, $C_3$-$C_{20}$ alkynyl; or $C_1$-$C_8$ alkyl, $C_3$-$C_8$ alkenyl or $C_3$-$C_8$ alkynyl, each optionally substituted with halogen, $C_1$-$C_6$ alkoxy, $C_2$-$C_6$ alkoxyalkyl, $C_1$-$C_6$ alkylthio, $C_3$-$C_6$ cycloalkyl, CN or with a phenyl group substituted with 0-3 halogen and 0-2 groups selected from $CH_3$, $CF_3$, $CH_3O$, CN, $CH_3S$, methylsulfonyl, dimethylamino, amino, phenyl or phenoxy; provided that, if $R^1$ is alkenyl or alkynyl, the unsaturated carbons are not bonded directly to the oxygen atom of

$\overset{O}{\overset{\|}{C}}$ -O;

$R^2$ and $R^3$ are independently selected from $C_1$-$C_4$ alkyl, $C_1$-$C_4$ haloalkyl, benzyl or phenyl;
$R^4$ is H or $C_1$-$C_4$ alkyl;
$R^5$ is $C_1$-$C_4$ alkyl substituted with 0-3 halogen, or $R^5$ is a phenyl group substituted with 0-3 halogen and 0-2 groups selected from $CH_3$, $CF_3$, $CH_3O$, CN, $CH_3S$, methylsulfonyl, dimethylamino, amino, nitro, phenyl or phenoxy;
$R^6$ is H, $C_1$-$C_4$ alkyl substituted with 0-3 halogen, or R6 is a phenyl group substituted with 0-3 halogen and 0-2 groups selected from $CH_3$, $CF_3$, $CH_3O$, CN, $CH_3S$, methylsulfonyl, dimethylamino, amino, phenyl or phenoxy;
$R^7$ is H or $C_1$-$C_4$ alkyl substituted with 0-3 halogen, or $R^6$ and $R^7$ taken together with the nitrogen atom to which they are attached can be piperidine, pyrrolidine or morpholine, each substituted with 0-2 methyl groups;
$R^8$ is H or $C_1$-$C_4$ alkyl;
$R^9$ is $C_1$-$C_8$ alkyl substituted with 0-3 halogen, or $R^9$ is phenyl substituted with 0-2 halogen, $CH_3$, $CF_3$, $CH_3O$ or CN;
$R^{10}$ and $R^{11}$ are independently H, $C_1$-$C_4$ alkyl, $C_1$-$C_4$ haloalkyl, $C_2$-$C_4$ alkoxyalkyl, or benzyl or phenyl, said benzyl or phenyl rings being substituted with 0-2 halogen, $CH_3$, $CF_3$, $CH_3O$ or CN; or $R^{10}$ and $R^{11}$, taken together with the nitrogen atom to which they are attached, can be azetidine, piperidine, homopippridine, pyrrolidine, or morpholine, each substituted with 0-2 methyl groups;
$R^{12}$ is $C_1$-$C_{12}$ alkyl or haloalkyl, or benzyl substituted with 0-2 halogen, $CH_3$, $CF_3$, $CH_3O$ or CN;
$R^{13}$ is $C_1$-$C_4$ alkyl, haloalkyl or $C_2$-$C_4$ alkoxyalkyl, $C_3$-$C_4$ alkenyl, haloalkenyl, alkynyl or haloalkynyl, or benzyl or phenyl, said benzyl or phenyl rings being substituted with 0-2 halogen, $CH_3$, $CF_3$, $CH_3O$ or CN;
$R^{14}$ is H, $C_1$-$C_4$ alkyl, $C_1$-$C_4$ haloalkyl, or $C_2$-$C_4$ alkoxyalkyl; $C_3$-$C_4$ alkenyl, haloalkenyl, alkynyl or haloalkynyl; or benzyl or phenyl, said benzyl or phenyl rings being substituted with 0-2 halogen, $CH_3$, $CF_3$, $CH_3O$, $CH_3S$ or CN;

$R^{15}$ is H or $C_1$-$C_4$ alkyl;

$R^{16}$ is $C_1$-$C_6$ alkyl, $C_3$-$C_4$ alkenyl, or benzyl substituted with 0-2 halogen, methyl, trifluoromethyl, nitro or methoxy; and

p is 1 or 2;

provided that

1. when A is C(-O)$SR^1$ or C(=O)$OR^1$, then G is not C(=L)$NR^{10}R^{11}$ or C(=O)$OR^{12}$; and

2. when A is C(=O)NHR, and G is C(=L)$NR^{10}R^{11}$ or C(=O)$OR^{12}$, then R is not unsubstituted phenyl or $C_1$-$C_3$ alkyl.

8. A compound of Claim 7 wherein:

A is C(=O)R, C(=O)$OR^1$,C(=O)NHR, or P(=O)$QR^2Q^1R^3$;

G is C(=O)$NR^{10}R^{11}$ or C(=O)$OR^{12}$.

9. A compound of Claim 7 wherein:

A is C(=O)R, or C(=O)NHR;

G is C(=O)$NR^{10}R^{11}$ or C(=O)$OR^{12}$;

X is Cl.

10. A compound of Claim 7 wherein:

A is C(=O)R, or C(=O)NHR;

G is C(=O)$NR^{10}R^{11}$ or C(=O)$OR^{12}$;

R is $C_1$-$C_2$ alkyl substituted with a phenyl group, said phenyl group being substituted with 0-3 halogen and 0-2 groups selected from $CH_3$, $CF_3$, $CH_3O$, CN, $CH_3S$, methylsulfonyl, dimethylamino, amino, phenyl or phenoxy; or

R is a phenyl or naphthyl ring, substituted with 0-3 halogen and 0-2 groups selected from $CH_3$, $CF_3$, $CH_3O$, CN, $CH_3S$, methylsulfonyl, dimethylamino, amino, phenyl or phenoxy;

$R^{10}$ and $R^{11}$ are independently H, $C_1$-$C_4$ alkyl, haloalkyl or benzyl substituted with 0-2 halogen, $CH_3$, $CF_3$, $CH_3O$ or CN; or $R^{10}$ and $R^{11}$, taken together with the nitrogen atom to which they are attached, can be piperidine, pyrrolidine or morpholine, each substituted with 0-2 methyl groups; and

X is Cl.

11. A compound of Claim 10 wherein:

A is C(=O)R;

G is C(=O)$NR^{10}R^{11}$;

R is $C_1$-$C_2$ alkyl substituted with a phenyl group, said phenyl group being substituted with 0-3 halogen and 0-2 groups selected from $CH_3$, $CF_3$, $CH_3O$, CN, $CH_3S$, methylsulfonyl, dimethylamino, amino, phenyl or phenoxy; or

R is a substituted phenyl or naphthyl ring, wherein the substituent is selected from: 0-3 halogen and 0-2 $CH_3$, $CF_3$, $CH_3O$, CN, $CH_3S$, methylsulfonyl, dimethylamino, amino, phenyl or phenoxy; and

X is Cl.

12. A compound of Claim 10 wherein:

A is C(=O)NHR;

G is C(=O)$NR^{10}R^{11}$;

R is $C_1$-$C_2$ alkyl substituted with a phenyl group, said phenyl group being substituted with 0-3 halogen and 0-2 groups selected from $CH_3$, $CF_3$, $CH_3O$, CN, $CH_3S$, methylsulfonyl, dimethylamino, amino, phenyl or phenoxy; or

R is a substituted phenyl or naphthyl ring, wherein the substituent is selected from: 0-3 halogen and 0-2 $CH_3$, $CF_3$, $CH_3O$, CN, $CH_3S$, methylsulfonyl, dimethylamino, amino, phenyl or phenoxy; and

X is Cl.

13. A compound selected from the following group:

N-[[2-naphthylcarbonyl]oxy]-2-(dimethylamino)-2-oxoethanimidoyl chloride;

N-[[[(3,5-dichlorophenyl)amino]carbonyl]oxy]-2-(dimethylamino)-2-oxoethanimidoyl chloride;

N-[[[(2,6-dichlorophenyl)amino]carbonyl]oxy]-2-(dimethylamino)-2-oxoethanimidoyl chloride;

N-[[[[3,5-Bis(trifluoromethyl)phenyl]amino]carbonyl]oxy]2-(dimethylamino)-2-oxo-ethanimidoyl chloride;

N-[[[3,4-(dichlorophenyl)amino]carbonyl]oxy]-2-(dimethylamino)-2-oxoethanimidoyl chloride;

N-[[[3,4-(dichlorophenyl)amino]carbonyl]oxy]-2-oxo-2-piperidino ethanimidoyl chloride;

N-[[[(3,5-dichlorophenyl)amino]carbonyl]oxy]-α-oxo-1-piperidineethanimidoyl chloride;

N-[[bis(2,2,2-trichloroethoxy)phosphinyl]oxy]-2-(dimethylamino)-2-oxoethanimidoyl chloride;

N-[[[[(2-chlorophenyl)methyl]amino]carbonyl]oxy]-2-(diethylamino)-2-oxoethanimidoyl chloride;

N-[[([1,1-biphenyl]-4-yl)carbonyl]oxy]-2-(dimethylamino)-2-oxoethanimidoyl chloride;

N-[[[(3,5-difluorophenyl)amino]carbonyl]oxy]-α-oxo-1-piperidineethanimidoyl chloride;

N-[[(2-naphthalenyl)carbonyl]oxy]-α-oxo-1-piperidineethanimidoyl chloride;

2-(dimethylamino)-2-oxo-N-[[[(2,4,5-trichlorophenyl)amino]carbonyl]oxy]ethanimidoyl chloride;
2-(dimethylamino)-2-oxo-N-[(1-oxooctadecyl)oxy]ethanimidoyl chloride; and
2-(dimethylamino)-2-oxo-N-[[(phenylmethoxy)carbonyl]oxy]ethanimidoyl chloride.

14. A compound useful for preparing the fungicides of Claims 7-13 selected from:
N-[(chlorocarbonyl)oxy]-2-dimethylamino-2-oxo-ethaneimidoyl chloride; and
N-[(chlorocarbonyl)oxy]-α-oxo-1-piperidineethanimidoyl chloride.

15. An agriculturally suitable composition of a compound of Claims 7-13 comprising a fungicidally active amount of said compound and at least one of the following: surfactant, solid diluent or liquid diluent.

European Patent Office

# EUROPEAN SEARCH REPORT

Application Number

EP 90 30 4388

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-2 134 332 (ROUSSEL-UCLAF) * Entire document * | 1-3 | C 07 C 271/60<br>C 07 C 259/02<br>C 07 C 317/28<br>C 07 C 327/40<br>C 07 D 295/15<br>C 07 F 9/09<br>A 01 N 37/52<br>A 01 N 41/10<br>A 01 N 47/10<br>A 01 N 57/02 |
| X | DE-A-2 621 102 (SCHERING) * Page 42, formula * | 7 | |
| X | EP-A-0 010 588 (CIBA-GEIGY) * Entire document * | 7,15 | |
| X | FR-A-2 327 984 (ROUSSEL-UCLAF) * Entire document * | 1,7,15 | |
| X | US-A-4 272 453 (D.L. BOOTH et al.) * Entire document * | 1,7,15 | |
| X | DE-A-1 806 120 (AGRIPAT) * Compound xII, example 4 * | 7 | |
| D,A | GB-A-2 028 797 (CIBA- GEIGY) * Compound 398 * & US equivalents listed on annex | 1,7 | TECHNICAL FIELDS SEARCHED (Int. Cl.5) |
| X | CHEMICAL ABSTRACTS, vol. 81, 1974, page 491, abstract no. 120126q, Columbus, Ohio, US; H. BRACHWITZ: "Hydroximic acid derivatives. VIII. Reduction of hydroximic acid derivatives", & Z. CHEM. 1974, 14(7), 268-9; & GENERIC DARC ONLINE GRAPHIC OF CHEM. ABS. REG. NO. 23477-23-8 | 7 | C 07 C 271/00<br>C 07 C 259/00<br>C 07 C 317/00<br>C 07 C 327/00<br>C 07 D 295/00<br>C 07 F 9/00<br>A 01 N 37/00<br>A 01 N 47/00 |

-/-

The present search report has been drawn up for all claims

| Place of search | Date of completion of the search | Examiner |
|---|---|---|
| THE HAGUE | 22-08-1990 | WELLS A.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 90 30 4388

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 71, 1969, page 283, abstract no. 101249j, Columbus, Ohio, US; Y.L. KRUGLYAK et al.: "Phosphorylated oximes. VI. Reaction of salts of nitro carboxylic acid esters with diethyl chloroposphite", & ZH. OBSHCH. KHIM. 1969, 39(6), 1263-4; & GENERIC DARC ONLINE GRAPHICS OF CHEM. ABS. REG. NOS. 25328-82-9,25309-11-9,25309-10-8,25309-09-5 --- | 7 | |
| X | CHEMICAL ABSTRACTS, vol. 95, 1981, page 732, abstract no. 169077c, Columbus, Ohio, US; K. HARADA et al.: "The synthetic reactions of aliphatic nitro compounds. Part XVII. Synthesis of five-membered heterocycles containing a nitrogen-oxygen bond via O-acylation of aliphatic nitro compounds", & CHEM. PHARM. BULL. 1980, 28(11), 3296-303; & GENERIC DARC ONLINE GRAPHICS OF CHEM. ABS. REG. NOS. 77597-06-9,69337-38-8 --- | 7 | TECHNICAL FIELDS SEARCHED (Int. Cl.5) |
| X | CHEMICAL ABSTRACTS, vol. 106, 1987, page 734, abstract no. 196306d, Columbus, Ohio, US; T. SHIMIZU et al.: "Convenient preparative method of nitrile oxides bt the dehydration of primary nitro compounds with ethyl chloroformate or benzenesulfonyl chloride in the presence of triethylamine", & BULL. CHEM. SOC. JPN. 1986, 59(9), 2827-31; & GENERIC DARC ONLINE GRAPHIC OF CHEM. ABS. REG. NO. 108199-01-5 ----- | | |

The present search report has been drawn up for all claims

| Place of search | Date of completion of the search | Examiner |
|---|---|---|
| THE HAGUE | 22-08-1990 | WELLS A.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document